(19) Europäisches Patentamt
European Patent Office
Office européen des brevets

(11) EP 2 446 052 B1

(12) EUROPEAN PATENT SPECIFICATION

(45) Date of publication and mention of the grant of the patent:
**08.08.2018 Bulletin 2018/32**

(21) Application number: **10722512.0**

(22) Date of filing: **04.06.2010**

(51) Int Cl.:
***C12Q 1/68*** *(2018.01)*

(86) International application number:
**PCT/US2010/037477**

(87) International publication number:
**WO 2010/151416 (29.12.2010 Gazette 2010/52)**

# (54) METHOD OF MEASURING ADAPTIVE IMMUNITY

VERFAHREN ZUR MESSUNG DER ADAPTIVEN IMMUNITÄT

PROCÉDÉ DE MESURE DE L'IMMUNITÉ ADAPTATIVE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **25.06.2009 US 220344 P**

(43) Date of publication of application:
**02.05.2012 Bulletin 2012/18**

(73) Proprietor: **Fred Hutchinson Cancer Research Center**
**Seattle, WA 98109 (US)**

(72) Inventors:
- **ROBINS, Harlan, S.**
  **Seattle**
  **WA 98119 (US)**
- **WARREN, Edus, H.**
  **Bainbridge Island**
  **WA 98110 (US)**
- **CARLSON, Christopher, Scott**
  **Kirkland**
  **WA 98033 (US)**

(74) Representative: **Boult Wade Tennant**
**Verulam Gardens**
**70 Gray's Inn Road**
**London WC1X 8BT (GB)**

(56) References cited:
**EP-A1- 2 062 982**

- **MARIANI SARA ET AL: "Comprehensive assessment of the TCRBV repertoire in small T-cell samples by means of an improved and convenient multiplex PCR method" EXPERIMENTAL HEMATOLOGY (NEW YORK), vol. 37, no. 6, 20 May 2009 (2009-05-20), - 20 May 2009 (2009-05-20) pages 728-738, XP002596859 ISSN: 0301-472X**
- **VAN DONGEN J ET AL: "Design and standardization of PCR primers and protocols for detection of clonal immunoglobuline and T-cell receptor gene recombinations is suspect lymphoproliferations: report of the BIOMED-2 concerted action BMH4-CT98-3936" LEUKEMIA, MACMILLAN PRESS LTD, US, vol. 17, no. 12, 1 December 2000 (2000-12-01), pages 2257-2317, XP008093070 ISSN: 0887-6924**
- **MASLANKA KRYSTYNA ET AL: "Molecular analysis of T cell repertoires: Spectratypes generated by multiplex polymerase chain reaction and evaluated by radioactivity or fluorescence" HUMAN IMMUNOLOGY, vol. 44, no. 1, 1995, pages 28-34, XP002596860 ISSN: 0198-8859**
- **KIIANITSA KONSTANTIN ET AL: "Development of tools for T cell repertoire analysis (TCRB spectratyping) for the canine model of hematopoietic cell transplantation" BLOOD, vol. 110, no. 11, Part 2, November 2007 (2007-11), page 293B, XP002596861 & 49TH ANNUAL MEETING OF THE AMERICAN-SOCIETY-OF-HEMATOLOGY; ATLANTA, GA, USA; DECEMBER 08 -11, 2007 ISSN: 0006-4971**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> FRED HUTCHINSON CANCER RESEARCH CENTER ROBINS, Harlan WARREN, Edus H., III CARLSON, Christopher

<120> METHOD OF MEASURING ADAPTIVE IMMUNITY

<130> HTCH-0106

<150> US 61/220,344
<151> 2009-06-25

<160> 484

<170> PatentIn version 3.5

<210> 1
<211> 32
<212> DNA
<213> Artificial Sequence

<220>
<223> Synthetic DNA: TRBV2

<220>
<221> misc_feature
<222> (1)..(1)
<223> n is a, c, g, or t; 5 end modified with universal forward primer

<400> 1
ntcaaatttc actctgaaga tccggtccac aa 32

<210> 2
<211> 32
<212> DNA
<213> Artificial Sequence

<220>
<223> Synthetic DNA: TRBV3-1

<220>
<221> misc_feature
<222> (1)..(1)
<223> n is a, c, g, or t; 5 end modified with universal forward primer

<400> 2
ngctcactta aatcttcaca tcaattccct gg 32

<210> 3
<211> 27
<212> DNA
<213> Artificial Sequence

<220>
<223> Synthetic DNA: TRBV4-1

<220>
<221> misc_feature
<222> (1)..(1)
<223> n is a, c, g, or t; 5 end modified with universal forward primer

<400> 3
ncttaaacct tcacctacac gccctgc 27

<210> 4
<211> 27
<212> DNA
<213> Artificial Sequence

<220>
<223> Synthetic DNA: TRBV(4-2, 4-3)

<220>
<221> misc_feature

<222> (1)..(1)
<223> n is a, c, g, or t; 5 end modified with universal forward primer

<400> 4
ncttattcct tcacctacac accctgc 27

<210> 5
<211> 27
<212> DNA
<213> Artificial Sequence

<220>
<223> Synthetic DNA: TRBV5-1

<220>
<221> misc_feature
<222> (1)..(1)
<223> n is a, c, g, or t; 5 end modified with universal forward primer

<400> 5
ngctctgaga tgaatgtgag caccttg 27

<210> 6
<211> 27
<212> DNA
<213> Artificial Sequence

<220>
<223> Synthetic DNA: TRBV5-3

<220>
<221> misc_feature
<222> (1)..(1)
<223> n is a, c, g, or t; 5 end modified with universal forward primer

<400> 6
ngctctgaga tgaatgtgag tgccttg 27

<210> 7
<211> 27
<212> DNA
<213> Artificial Sequence

<220>
<223> Synthetic DNA: TRBV(5-4, 5-5, 5-6, 5-7, 5-8)

<220>
<221> misc_feature
<222> (1)..(1)
<223> n is a, c, g, or t; 5 end modified with universal forward primer

<400> 7
ngctctgagc tgaatgtgaa cgccttg 27

<210> 8
<211> 23
<212> DNA
<213> Artificial Sequence

<220>
<223> Synthetic DNA: TRBV6-1

<220>
<221> misc_feature
<222> (1)..(1)
<223> n is a, c, g, or t; 5 end modified with universal forward primer

<400> 8
ntcgctcagg ctggagtcgg ctg 23

<210> 9
<211> 21
<212> DNA
<213> Artificial Sequence

<220>
<223> Synthetic DNA: TRBV(6-2, 6-3)

<220>
<221> misc_feature
<222> (1)..(1)
<223> n is a, c, g, or t; 5 end modified with universal forward primer

<400> 9
ngctggggtt ggagtcggct g 21

<210> 10
<211> 22
<212> DNA
<213> Artificial Sequence

<220>
<223> Synthetic DNA: TRBV6-4

<220>
<221> misc_feature
<222> (1)..(1)
<223> n is a, c, g, or t; 5 end modified with universal forward primer

<400> 10
nccctcacgt tggcgtctgc tg 22

<210> 11
<211> 21
<212> DNA
<213> Artificial Sequence

<220>
<223> Synthetic DNA: TRBV6-5

<220>
<221> misc_feature
<222> (1)..(1)
<223> n is a, c, g, or t; 5 end modified with universal forward primer

<400> 11
ngctcaggct gctgtcggct g 21

<210> 12
<211> 22
<212> DNA
<213> Artificial Sequence

<220>
<223> Synthetic DNA: TRBV6-6

<220>
<221> misc_feature
<222> (1)..(1)
<223> n is a, c, g, or t; 5 end modified with universal forward primer

<400> 12
ncgctcaggc tggagttggc tg 22

<210> 13
<211> 23
<212> DNA
<213> Artificial Sequence

<220>
<223> Synthetic DNA: TRBV6-7

<220>
<221> misc_feature
<222> (1)..(1)
<223> n is a, c, g, or t; 5 end modified with universal forward primer

<400> 13
ncccctcaag ctggagtcag ctg 23

<210> 14
<211> 22
<212> DNA
<213> Artificial Sequence

<220>
<223> Synthetic DNA: TRBV6-8

<220>
<221> misc_feature
<222> (1)..(1)
<223> n is a, c, g, or t; 5 end modified with universal forward primer

<400> 14
ncactcaggc tggtgtcggc tg 22

<210> 15
<211> 22
<212> DNA
<213> Artificial Sequence

<220>
<223> Synthetic DNA: TRBV6-9

<220>
<221> misc_feature

<222> (1)..(1)
<223> n is a, c, g, or t; 5 end modified with universal forward primer

<400> 15
ncgctcaggc tggagtcagc tg 22

<210> 16
<211> 25
<212> DNA
<213> Artificial Sequence

<220>
<223> Synthetic DNA: TRBV7-1

<220>
<221> misc_feature
<222> (1)..(1)
<223> n is a, c, g, or t; 5 end modified with universal forward primer

<400> 16
nccactctga agttccagcg cacac 25

<210> 17
<211> 24
<212> DNA
<213> Artificial Sequence

<220>
<223> Synthetic DNA: TRBV7-2

<220>
<221> misc_feature
<222> (1)..(1)
<223> n is a, c, g, or t; 5 end modified with universal forward primer

<400> 17
ncactctgac gatccagcgc acac 24

<210> 18
<211> 27
<212> DNA
<213> Artificial Sequence

<220>
<223> Synthetic DNA: TRBV7-3

<220>
<221> misc_feature
<222> (1)..(1)
<223> n is a, c, g, or t; 5 end modified with universal forward primer

<400> 18
nctctactct gaagatccag cgcacag 27

<210> 19
<211> 25
<212> DNA
<213> Artificial Sequence

<220>
<223> Synthetic DNA: TRBV7-4

<220>
<221> misc_feature
<222> (1)..(1)
<223> n is a, c, g, or t; 5 end modified with universal forward primer

<400> 19
nccactctga agatccagcg cacag 25

<210> 20
<211> 24
<212> DNA
<213> Artificial Sequence

<220>
<223> Synthetic DNA: TRBV7-6

<220>
<221> misc_feature
<222> (1)..(1)
<223> n is a, c, g, or t; 5 end modified with universal forward primer

<400> 20
ncactctgac gatccagcgc acag 24

<210> 21
<211> 25
<212> DNA
<213> Artificial Sequence

<220>
<223> Synthetic DNA: TRBV7-7

<220>
<221> misc_feature
<222> (1)..(1)
<223> n is a, c, g, or t; 5 end modified with universal forward primer

<400> 21
nccactctga cgattcagcg cacag 25

<210> 22
<211> 25
<212> DNA
<213> Artificial Sequence

<220>
<223> Synthetic DNA: TRBV7-8

<220>
<221> misc_feature
<222> (1)..(1)
<223> n is a, c, g, or t; 5 end modified with universal forward primer

<400> 22
nccactctga agatccagcg cacac 25

<210> 23
<211> 24
<212> DNA
<213> Artificial Sequence

<220>
<223> Synthetic DNA: TRBV7-9

<220>
<221> misc_feature
<222> (1)..(1)
<223> n is a, c, g, or t; 5 end modified with universal forward primer

<400> 23
ncaccttgga gatccagcgc acag 24

<210> 24
<211> 29
<212> DNA
<213> Artificial Sequence

<220>
<223> Synthetic DNA: TRBV9

<220>
<221> misc_feature
<222> (1)..(1)
<223> n is a, c, g, or t; 5 end modified with universal forward primer

<400> 24
ngcactctga actaaacctg agctctctg 29

<210> 25
<211> 23
<212> DNA
<213> Artificial Sequence

<220>
<223> Synthetic DNA: TRBV10-1

<220>
<221> misc_feature
<222> (1)..(1)
<223> n is a, c, g, or t; 5 end modified with universal forward primer

<400> 25
ncccctcact ctggagtctg ctg 23

<210> 26
<211> 24
<212> DNA
<213> Artificial Sequence

<220>
<223> Synthetic DNA: TRBV10-2

<220>
<221> misc_feature

<222> (1)..(1)
<223> n is a, c, g, or t; 5 end modified with universal forward primer

<400> 26
ncccccctcac tctggagtca gcta 24

<210> 27
<211> 24
<212> DNA
<213> Artificial Sequence

<220>
<223> Synthetic DNA: TRBV10-3

<220>
<221> misc_feature
<222> (1)..(1)
<223> n is a, c, g, or t; 5 end modified with universal forward primer

<400> 27
ncctcctcac tctggagtcc gcta 24

<210> 28
<211> 25
<212> DNA
<213> Artificial Sequence

<220>
<223> Synthetic DNA: TRBV(11-1, 11-3)

<220>
<221> misc_feature
<222> (1)..(1)
<223> n is a, c, g, or t; 5 end modified with universal forward primer

<400> 28
nccactctca agatccagcc tgcag 25

<210> 29
<211> 27
<212> DNA
<213> Artificial Sequence

<220>
<223> Synthetic DNA: TRBV11-2

<220>
<221> misc_feature
<222> (1)..(1)
<223> n is a, c, g, or t; 5 end modified with universal forward primer

<400> 29
nctccactct caagatccag cctgcaa 27

<210> 30
<211> 25
<212> DNA
<213> Artificial Sequence

<220>
<223> Synthetic DNA: TRBV(12-3, 12-4, 12-5)

<220>
<221> misc_feature
<222> (1)..(1)
<223> n is a, c, g, or t; 5 end modified with universal forward primer

<400> 30
nccactctga agatccagcc ctcag 25

<210> 31
<211> 29
<212> DNA
<213> Artificial Sequence

<220>
<223> Synthetic DNA: TRBV13

<220>
<221> misc_feature
<222> (1)..(1)
<223> n is a, c, g, or t; 5 end modified with universal forward primer

<400> 31
ncattctgaa ctgaacatga gctccttgg 29

<210> 32
<211> 25
<212> DNA
<213> Artificial Sequence

<220>
<223> Synthetic DNA: TRBV14

<220>
<221> misc_feature
<222> (1)..(1)
<223> n is a, c, g, or t; 5 end modified with universal forward primer

<400> 32
nctactctga aggtgcagcc tgcag 25

<210> 33
<211> 29
<212> DNA
<213> Artificial Sequence

<220>
<223> Synthetic DNA: TRBV15

<220>
<221> misc_feature
<222> (1)..(1)
<223> n is a, c, g, or t; 5 end modified with universal forward primer

<400> 33
ngataacttc caatccagga ggccgaaca 29

<210> 34
<211> 27
<212> DNA
<213> Artificial Sequence

<220>
<223> Synthetic DNA: TRBV16

<220>
<221> misc_feature
<222> (1)..(1)
<223> n is a, c, g, or t; 5 end modified with universal forward primer

<400> 34
nctgtagcct tgagatccag gctacga 27

<210> 35
<211> 25
<212> DNA
<213> Artificial Sequence

<220>
<223> Synthetic DNA: TRBV17

<220>
<221> misc_feature
<222> (1)..(1)
<223> n is a, c, g, or t; 5 end modified with universal forward primer

<400> 35
ncttccacgc tgaagatcca tcccg 25

<210> 36
<211> 25
<212> DNA
<213> Artificial Sequence

<220>
<223> Synthetic DNA: TRBV18

<220>
<221> misc_feature
<222> (1)..(1)
<223> n is a, c, g, or t; 5 end modified with universal forward primer

<400> 36
ngcatcctga ggatccagca ggtag 25

<210> 37
<211> 23
<212> DNA
<213> Artificial Sequence

<220>
<223> Synthetic DNA: TRBV19

<220>
<221> misc_feature

<222> (1)..(1)
<223> n is a, c, g, or t; 5 end modified with universal forward primer

<400> 37
ncctctcact gtgacatcgg ccc 23

<210> 38
<211> 27
<212> DNA
<213> Artificial Sequence

<220>
<223> Synthetic DNA: TRBV20-1

<220>
<221> misc_feature
<222> (1)..(1)
<223> n is a, c, g, or t; 5 end modified with universal forward primer

<400> 38
ncttgtccac tctgacagtg accagtg 27

<210> 39
<211> 24
<212> DNA
<213> Artificial Sequence

<220>
<223> Synthetic DNA: TRBV23-1

<220>
<221> misc_feature
<222> (1)..(1)
<223> n is a, c, g, or t; 5 end modified with universal forward primer

<400> 39
ncagcctggc aatcctgtcc tcag 24

<210> 40
<211> 25
<212> DNA
<213> Artificial Sequence

<220>
<223> Synthetic DNA: TRBV24-1

<220>
<221> misc_feature
<222> (1)..(1)
<223> n is a, c, g, or t; 5 end modified with universal forward primer

<400> 40
nctccctgtc cctagagtct gccat 25

<210> 41
<211> 22
<212> DNA
<213> Artificial Sequence

<220>
<223> Synthetic DNA: TRBV25-1

<220>
<221> misc_feature
<222> (1)..(1)
<223> n is a, c, g, or t; 5 end modified with universal forward primer

<400> 41
nccctgaccc tggagtctgc ca 22

<210> 42
<211> 22
<212> DNA
<213> Artificial Sequence

<220>
<223> Synthetic DNA: TRBV27

<220>
<221> misc_feature
<222> (1)..(1)
<223> n is a, c, g, or t; 5 end modified with universal forward primer

<400> 42
nccctgatcc tggagtcgcc ca 22

<210> 43
<211> 24
<212> DNA
<213> Artificial Sequence

<220>
<223> Synthetic DNA: TRBV28

<220>
<221> misc_feature
<222> (1)..(1)
<223> n is a, c, g, or t; 5 end modified with universal forward primer

<400> 43
nctccctgat tctggagtcc gcca 24

<210> 44
<211> 32
<212> DNA
<213> Artificial Sequence

<220>
<223> Synthetic DNA: TRBV29-1

<220>
<221> misc_feature
<222> (1)..(1)
<223> n is a, c, g, or t; 5 end modified with universal forward primer

<400> 44
nctaacattc tcaactctga ctgtgagcaa ca 32

<210> 45
<211> 29
<212> DNA
<213> Artificial Sequence

<220>
<223> Synthetic DNA: TRBV30

<220>
<221> misc_feature
<222> (1)..(1)
<223> n is a, c, g, or t; 5 end modified with universal forward primer

<400> 45
ncggcagttc atcctgagtt ctaagaagc 29

<210> 46
<211> 36
<212> DNA
<213> Artificial Sequence

<220>
<223> Synthetic DNA: TRBJ1-1

<220>
<221> misc_feature
<222> (1)..(1)
<223> n is a, c, g, or t; 5 end modified with universal reverse primer

<400> 46
nttacctaca actgtgagtc tggtgccttg tccaaa 36

<210> 47
<211> 34
<212> DNA
<213> Artificial Sequence

<220>
<223> Synthetic DNA: TRBJ1-2

<220>
<221> misc_feature
<222> (1)..(1)
<223> n is a, c, g, or t; 5 end modified with universal reverse primer

<400> 47
nacctacaac ggttaacctg gtccccgaac cgaa 34

<210> 48
<211> 34
<212> DNA
<213> Artificial Sequence

<220>
<223> Synthetic DNA: TRBJ1-3

<220>
<221> misc_feature

<222> (1)..(1)
<223> n is a, c, g, or t; 5 end modified with universal reverse primer

<400> 48
nacctacaac agtgagccaa cttccctctc caaa 34

<210> 49
<211> 32
<212> DNA
<213> Artificial Sequence

<220>
<223> Synthetic DNA: TRBJ1-4

<220>
<221> misc_feature
<222> (1)..(1)
<223> n is a, c, g, or t; 5 end modified with universal reverse primer

<400> 49
nccaagacag agagctgggt tccactgcca aa 32

<210> 50
<211> 32
<212> DNA
<213> Artificial Sequence

<220>
<223> Synthetic DNA: TRBJ1-6

<220>
<221> misc_feature
<222> (1)..(1)
<223> n is a, c, g, or t; 5 end modified with universal reverse primer

<400> 50
nctgtcacag tgagcctggt cccgttccca aa 32

<210> 51
<211> 26
<212> DNA
<213> Artificial Sequence

<220>
<223> Synthetic DNA: TRBJ2-1

<220>
<221> misc_feature
<222> (1)..(1)
<223> n is a, c, g, or t; 5 end modified with universal reverse primer

<400> 51
ncggtgagcc gtgtccctgg cccgaa 26

<210> 52
<211> 32
<212> DNA
<213> Artificial Sequence

<220>
<223> Synthetic DNA: TRBJ2-2

<220>
<221> misc_feature
<222> (1)..(1)
<223> n is a, c, g, or t; 5 end modified with universal reverse primer

<400> 52
nccagtacgg tcagcctaga gccttctcca aa 32

<210> 53
<211> 27
<212> DNA
<213> Artificial Sequence

<220>
<223> Synthetic DNA: TRBJ2-3

<220>
<221> misc_feature
<222> (1)..(1)
<223> n is a, c, g, or t; 5 end modified with universal reverse primer

<400> 53
nactgtcagc cgggtgcctg ggccaaa 27

<210> 54
<211> 23
<212> DNA
<213> Artificial Sequence

<220>
<223> Synthetic DNA: TRBJ2-4

<220>
<221> misc_feature
<222> (1)..(1)
<223> n is a, c, g, or t; 5 end modified with universal reverse primer

<400> 54
nagagccggg tcccggcgcc gaa 23

<210> 55
<211> 23
<212> DNA
<213> Artificial Sequence

<220>
<223> Synthetic DNA: TRBJ2-5

<220>
<221> misc_feature
<222> (1)..(1)
<223> n is a, c, g, or t; 5 end modified with universal reverse primer

<400> 55
nggagccgcg tgcctggccc gaa 23

<210> 56
<211> 24
<212> DNA
<213> Artificial Sequence

<220>
<223> Synthetic DNA: TRBJ2-6

<220>
<221> misc_feature
<222> (1)..(1)
<223> n is a, c, g, or t; 5 end modified with universal reverse primer

<400> 56
ngtcagcctg ctgccggccc cgaa 24

<210> 57
<211> 24
<212> DNA
<213> Artificial Sequence

<220>
<223> Synthetic DNA: TRBJ2-7

<220>
<221> misc_feature
<222> (1)..(1)
<223> n is a, c, g, or t; 5 end modified with universal reverse primer

<400> 57
ngtgagcctg gtgcccggcc cgaa 24

<210> 58
<211> 65
<212> DNA
<213> Artificial Sequence

<220>
<223> Synthetic DNA: TRBV2

<400> 58

**caagcagaag acggcatacg agctcttccg atcttcaaat ttcactctga agatccggtc 60**

**cacaa 65**

<210> 59
<211> 65
<212> DNA
<213> Artificial Sequence

<220>
<223> Synthetic DNA: TRBV3-1

<400> 59

caagcagaag acggcatacg agctcttccg atctgctcac ttaaatcttc acatcaattc 60

cctgg 65

<210> 60
<211> 60
<212> DNA
<213> Artificial Sequence

<220>
<223> Synthetic DNA: TRBV4-1

<400> 60
caagcagaag acggcatacg agctcttccg atctcttaaa ccttcaccta cacgccctgc 60

<210> 61
<211> 60
<212> DNA
<213> Artificial Sequence

<220>
<223> Synthetic DNA: TRBV(4-2, 4-3)

<400> 61
caagcagaag acggcatacg agctcttccg atctcttatt ccttcaccta cacaccctgc 60

<210> 62
<211> 60
<212> DNA
<213> Artificial Sequence

<220>
<223> Synthetic DNA: TRBV5-1

<400> 62
caagcagaag acggcatacg agctcttccg atctgctctg agatgaatgt gagcaccttg 60

<210> 63
<211> 60
<212> DNA
<213> Artificial Sequence

<220>
<223> Synthetic DNA: TRBV5-3

<400> 63
caagcagaag acggcatacg agctcttccg atctgctctg agatgaatgt gagtgccttg 60

<210> 64
<211> 60
<212> DNA
<213> Artificial Sequence

<220>
<223> Synthetic DNA: TRBV(5-4, 5-5, 5-6, 5-7, 5-8)

<400> 64

caagcagaag acggcatacg agctcttccg atctgctctg agctgaatgt gaacgccttg 60

<210> 65
<211> 56
<212> DNA
<213> Artificial Sequence

<220>
<223> Synthetic DNA: TRBV6-1

<400> 65
caagcagaag acggcatacg agctcttccg atcttcgctc aggctggagt cggctg 56

<210> 66
<211> 54
<212> DNA
<213> Artificial Sequence

<220>
<223> Synthetic DNA: TRBV(6-2, 6-3)

<400> 66
caagcagaag acggcatacg agctcttccg atctgctggg gttggagtcg gctg 54

<210> 67
<211> 55
<212> DNA
<213> Artificial Sequence

<220>
<223> Synthetic DNA: TRBV6-4

<400> 67
caagcagaag acggcatacg agctcttccg atctccctca cgttggcgtc tgctg 55

<210> 68
<211> 54
<212> DNA
<213> Artificial Sequence

<220>
<223> Synthetic DNA: TRBV6-5

<400> 68
caagcagaag acggcatacg agctcttccg atctgctcag gctgctgtcg gctg 54

<210> 69
<211> 55
<212> DNA
<213> Artificial Sequence

<220>
<223> Synthetic DNA: TRBV6-6

<400> 69
caagcagaag acggcatacg agctcttccg atctcgctca ggctggagtt ggctg 55

<210> 70

<211> 56
<212> DNA
<213> Artificial Sequence

<220>
<223> Synthetic DNA: TRBV6-7

<400> 70
caagcagaag acggcatacg agctcttccg atctcccctc aagctggagt cagctg 56

<210> 71
<211> 55
<212> DNA
<213> Artificial Sequence

<220>
<223> Synthetic DNA: TRBV6-8

<400> 71
caagcagaag acggcatacg agctcttccg atctcactca ggctggtgtc ggctg 55

<210> 72
<211> 55
<212> DNA
<213> Artificial Sequence

<220>
<223> Synthetic DNA: TRBV6-9

<400> 72
caagcagaag acggcatacg agctcttccg atctcgctca ggctggagtc agctg 55

<210> 73
<211> 58
<212> DNA
<213> Artificial Sequence

<220>
<223> Synthetic DNA: TRBV7-1

<400> 73
caagcagaag acggcatacg agctcttccg atctccactc tgaagttcca gcgcacac 58

<210> 74
<211> 57
<212> DNA
<213> Artificial Sequence

<220>
<223> Synthetic DNA: TRBV7-2

<400> 74
caagcagaag acggcatacg agctcttccg atctcactct gacgatccag cgcacac 57

<210> 75
<211> 60
<212> DNA
<213> Artificial Sequence

<220>
<223> Synthetic DNA: TRBV7-3

<400> 75
caagcagaag acggcatacg agctcttccg atctctctac tctgaagatc cagcgcacag 60

<210> 76
<211> 58
<212> DNA
<213> Artificial Sequence

<220>
<223> Synthetic DNA: TRBV7-4

<400> 76
caagcagaag acggcatacg agctcttccg atctccactc tgaagatcca gcgcacag 58

<210> 77
<211> 57
<212> DNA
<213> Artificial Sequence

<220>
<223> Synthetic DNA: TRBV7-6

<400> 77
caagcagaag acggcatacg agctcttccg atctcactct gacgatccag cgcacag 57

<210> 78
<211> 58
<212> DNA
<213> Artificial Sequence

<220>
<223> Synthetic DNA: TRBV7-7

<400> 78
caagcagaag acggcatacg agctcttccg atctccactc tgacgattca gcgcacag 58

<210> 79
<211> 58
<212> DNA
<213> Artificial Sequence

<220>
<223> Synthetic DNA: TRBV7-8

<400> 79
caagcagaag acggcatacg agctcttccg atctccactc tgaagatcca gcgcacac 58

<210> 80
<211> 57
<212> DNA
<213> Artificial Sequence

<220>
<223> Synthetic DNA: TRBV7-9

<400> 80
caagcagaag acggcatacg agctcttccg atctcacctt ggagatccag cgcacag 57

<210> 81
<211> 62
<212> DNA
<213> Artificial Sequence

<220>
<223> Synthetic DNA: TRBV9

<400> 81

caagcagaag acggcatacg agctcttccg atctgcactc tgaactaaac ctgagctctc 60

tg 62

<210> 82
<211> 56
<212> DNA
<213> Artificial Sequence

<220>
<223> Synthetic DNA: TRBV10-1

<400> 82
caagcagaag acggcatacg agctcttccg atctcccctc actctggagt ctgctg 56

<210> 83
<211> 57
<212> DNA
<213> Artificial Sequence

<220>
<223> Synthetic DNA: TRBV10-2

<400> 83
caagcagaag acggcatacg agctcttccg atctccccct cactctggag tcagcta 57

<210> 84
<211> 57
<212> DNA
<213> Artificial Sequence

<220>
<223> Synthetic DNA: TRBV10-3

<400> 84
caagcagaag acggcatacg agctcttccg atctcctcct cactctggag tccgcta 57

<210> 85
<211> 58
<212> DNA
<213> Artificial Sequence

<220>
<223> Synthetic DNA: TRBV(11-1, 11-3)

<400> 85
caagcagaag acggcatacg agctcttccg atctccactc tcaagatcca gcctgcag 58

<210> 86
<211> 60
<212> DNA
<213> Artificial Sequence

<220>
<223> Synthetic DNA: TRBV11-2

<400> 86
caagcagaag acggcatacg agctcttccg atctctccac tctcaagatc cagcctgcaa 60

<210> 87
<211> 58
<212> DNA
<213> Artificial Sequence

<220>
<223> Synthetic DNA: TRBV(12-3, 12-4, 12-5)

<400> 87
caagcagaag acggcatacg agctcttccg atctccactc tgaagatcca gccctcag 58

<210> 88
<211> 62
<212> DNA
<213> Artificial Sequence

<220>
<223> Synthetic DNA: TRBV13

<400> 88

caagcagaag acggcatacg agctcttccg atctcattct gaactgaaca tgagctcctt 60

gg 62

<210> 89
<211> 58
<212> DNA
<213> Artificial Sequence

<220>
<223> Synthetic DNA: TRBV14

<400> 89
caagcagaag acggcatacg agctcttccg atctctactc tgaaggtgca gcctgcag 58

<210> 90
<211> 62
<212> DNA
<213> Artificial Sequence

<220>
<223> Synthetic DNA: TRBV15

<400> 90

**caagcagaag acggcatacg agctcttccg atctgataac ttccaatcca ggaggccgaa 60**

**ca 62**

<210> 91
<211> 60
<212> DNA
<213> Artificial Sequence

<220>
<223> Synthetic DNA: TRBV16

<400> 91
caagcagaag acggcatacg agctcttccg atctctgtag ccttgagatc caggctacga 60

<210> 92
<211> 58
<212> DNA
<213> Artificial Sequence

<220>
<223> Synthetic DNA: TRBV17

<400> 92
caagcagaag acggcatacg agctcttccg atctcttcca cgctgaagat ccatcccg 58

<210> 93
<211> 58
<212> DNA
<213> Artificial Sequence

<220>
<223> Synthetic DNA: TRBV18

<400> 93
caagcagaag acggcatacg agctcttccg atctgcatcc tgaggatcca gcaggtag 58

<210> 94
<211> 56
<212> DNA
<213> Artificial Sequence

<220>
<223> Synthetic DNA: TRBV19

<400> 94
caagcagaag acggcatacg agctcttccg atctcctctc actgtgacat cggccc 56

<210> 95
<211> 60
<212> DNA
<213> Artificial Sequence

<220>
<223> Synthetic DNA: TRBV20-1

<400> 95
caagcagaag acggcatacg agctcttccg atctcttgtc cactctgaca gtgaccagtg 60

<210> 96
<211> 57
<212> DNA
<213> Artificial Sequence

<220>
<223> Synthetic DNA: TRBV23-1

<400> 96
caagcagaag acggcatacg agctcttccg atctcagcct ggcaatcctg tcctcag 57

<210> 97
<211> 58
<212> DNA
<213> Artificial Sequence

<220>
<223> Synthetic DNA: TRBV24-1

<400> 97
caagcagaag acggcatacg agctcttccg atctctccct gtccctagag tctgccat 58

<210> 98
<211> 55
<212> DNA
<213> Artificial Sequence

<220>
<223> Synthetic DNA: TRBV25-1

<400> 98
caagcagaag acggcatacg agctcttccg atctccctga ccctggagtc tgcca 55

<210> 99
<211> 55
<212> DNA
<213> Artificial Sequence

<220>
<223> Synthetic DNA: TRBV27

<400> 99
caagcagaag acggcatacg agctcttccg atctccctga tcctggagtc gccca 55

<210> 100
<211> 57
<212> DNA
<213> Artificial Sequence

<220>
<223> Synthetic DNA: TRBV28

<400> 100
caagcagaag acggcatacg agctcttccg atctctccct gattctggag tccgcca 57

<210> 101
<211> 65
<212> DNA
<213> Artificial Sequence

<220>
<223> Synthetic DNA: TRBV29-1

<400> 101

**caagcagaag acggcatacg agctcttccg atctctaaca ttctcaactc tgactgtgag 60**

**caaca 65**

<210> 102
<211> 62
<212> DNA
<213> Artificial Sequence

<220>
<223> Synthetic DNA: TRBV30

<400> 102

**caagcagaag acggcatacg agctcttccg atctcggcag ttcatcctga gttctaagaa 60**

**gc 62**

<210> 103
<211> 60
<212> DNA
<213> Artificial Sequence

<220>
<223> Synthetic DNA: TRBJ1-1

<400> 103
aatgatacgg cgaccaccga gatctttacc tacaactgtg agtctggtgc cttgtccaaa 60

<210> 104
<211> 58
<212> DNA
<213> Artificial Sequence

<220>
<223> Synthetic DNA: TRBJ1-3

<400> 104
aatgatacgg cgaccaccga gatctaccta caacagtgag ccaacttccc tctccaaa 58

<210> 105
<211> 56
<212> DNA
<213> Artificial Sequence

<220>
<223> Synthetic DNA: TRBJ1-4

<400> 105
aatgatacgg cgaccaccga gatctccaag acagagagct gggttccact gccaaa 56

<210> 106
<211> 56
<212> DNA
<213> Artificial Sequence

<220>
<223> Synthetic DNA: TRBJ1-6

<400> 106
aatgatacgg cgaccaccga gatctctgtc acagtgagcc tggtcccgtt cccaaa 56

<210> 107
<211> 50
<212> DNA
<213> Artificial Sequence

<220>
<223> Synthetic DNA: TRBJ2-1

<400> 107
aatgatacgg cgaccaccga gatctcggtg agccgtgtcc ctggcccgaa 50

<210> 108
<211> 56
<212> DNA
<213> Artificial Sequence

<220>
<223> Synthetic DNA: TRBJ2-2

<400> 108
aatgatacgg cgaccaccga gatctccagt acggtcagcc tagagccttc tccaaa 56

<210> 109
<211> 51
<212> DNA
<213> Artificial Sequence

<220>
<223> Synthetic DNA: TRBJ2-3

<400> 109
aatgatacgg cgaccaccga gatctactgt cagccgggtg cctgggccaa a 51

<210> 110
<211> 47
<212> DNA
<213> Artificial Sequence

<220>
<223> Synthetic DNA: TRBJ2-4

<400> 110
aatgatacgg cgaccaccga gatctagagc cgggtcccgg cgccgaa 47

<210> 111
<211> 47
<212> DNA
<213> Artificial Sequence

<220>
<223> Synthetic DNA: TRBJ2-5

<400> 111
aatgatacgg cgaccaccga gatctggagc cgcgtgcctg gcccgaa 47

<210> 112
<211> 48
<212> DNA
<213> Artificial Sequence

<220>
<223> Synthetic DNA: TRBJ2-6

<400> 112
aatgatacgg cgaccaccga gatctgtcag cctgctgccg gccccgaa 48

<210> 113
<211> 48
<212> DNA
<213> Artificial Sequence

<220>
<223> Synthetic DNA: TRBJ2-7

<400> 113
aatgatacgg cgaccaccga gatctgtgag cctggtgccc ggcccgaa 48

<210> 114
<211> 284
<212> DNA
<213> Artificial Sequence

<220>
<223> Synthetic DNA: TRBV1*01

<400> 114

gatactggaa ttacccagac accaaaatac ctggtcacag caatggggag taaaaggaca 60

atgaaacgtg agcatctggg acatgattct atgtattggt acagacagaa agctaagaaa 120

tccctggagt tcatgtttta ctacaactgt aaggaattca ttgaaaacaa gactgtgcca 180

aatcacttca cacctgaatg ccctgacagc tctcgcttat accttcatgt ggtcgcactg 240

cagcaagaag actcagctgc gtatctctgc accagcagcc aaga 284

<210> 115
<211> 290
<212> DNA
<213> Artificial Sequence

<220>
<223> Synthetic DNA: TRBV2*01

<400> 115

gaacctgaag tcacccagac tcccagccat caggtcacac agatgggaca ggaagtgatc 60

ttgcgctgtg tccccatctc taatcactta tacttctatt ggtacagaca aatcttgggg 120

cagaaagtcg agtttctggt ttccttttat aataatgaaa tctcagagaa gtctgaaata 180

ttcgatgatc aattctcagt tgaaaggcct gatggatcaa atttcactct gaagatccgg 240

tccacaaagc tggaggactc agccatgtac ttctgtgcca gcagtgaagc 290

<210> 116
<211> 288
<212> DNA
<213> Artificial Sequence

<220>
<223> Synthetic DNA: TRBV2*03

<400> 116

gaacctgaag tcacccagac tcccagccat caggtcacac agatgggaca ggaagtgatc 60

ttgcgctgtg tccccatctc taatcactta tacttctatt ggtacagaca aatcttgggg 120

cagaaagtcg agtttctggt ttccttttat aataatgaaa tctcagagaa gtctgaaata 180

ttcgatgatc aattctcagt tgagaggcct gatggatcaa atttcactct gaagatccgg 240

tccacaaagc tggaggactc agccatgtac ttctgtgcca gcagtgaa 288

<210> 117
<211> 287
<212> DNA
<213> Artificial Sequence

<220>
<223> Synthetic DNA: TRBV3-1*01

<400> 117

gacacagctg tttcccagac tccaaaatac ctggtcacac agatgggaaa cgacaagtcc 60

attaaatgtg aacaaaatct gggccatgat actatgtatt ggtataaaca ggactctaag 120

aaatttctga agataatgtt tagctacaat aataaggagc tcattataaa tgaaacagtt 180

ccaaatcgct tctcacctaa atctccagac aaagctcact taaatcttca catcaattcc 240

ctggagcttg gtgactctgc tgtgtatttc tgtgccagca gccaaga 287

<210> 118
<211> 279
<212> DNA

<213> Artificial Sequence

<220>
<223> Synthetic DNA: TRBV3-1*02

<400> 118

gacacagctg tttcccagac tccaaaatac ctggtcacac agatgggaaa cgacaagtcc 60

attaaatgtg aacaaaatct gggccatgat actatgtatt ggtataaaca ggactctaag 120

aaatttctga agataatgtt tagctacaat aacaaggaga tcattataaa tgaaacagtt 180

ccaaatcgat tctcacctaa atctccagac aaagctaaat taaatcttca catcaattcc 240

ctggagcttg gtgactctgc tgtgtatttc tgtgccagc 279

<210> 119
<211> 287
<212> DNA
<213> Artificial Sequence

<220>
<223> Synthetic DNA: TRBV3-2*01

<400> 119

gacacagccg tttcccagac tccaaaatac ctggtcacac agatgggaaa aaaggagtct 60

cttaaatgag aacaaaatct gggccataat gctatgtatt ggtataaaca ggactctaag 120

aaatttctga agacaatgtt tatctacagt aacaaggagc caattttaaa tgaaacagtt 180

ccaaatcgct tctcacctga ctctccagac aaagctcatt taaatcttca catcaattcc 240

ctggagcttg gtgactctgc tgtgtatttc tgtgccagca gccaaga 287

<210> 120
<211> 287
<212> DNA
<213> Artificial Sequence

<220>
<223> Synthetic DNA: TRBV3-2*02

<400> 120

gacacagccg tttcccagac tccaaaatac ctggtcacac agatgggaaa aaaggagtct 60

cttaaatgag aacaaaatct gggccataat gctatgtatt ggtataaaca ggactctaag 120

aaatttctga agacaatgtt tatctacagt aacaaggagc caattttaaa tgaaacagtt 180

ccaaatcgct tctcacctga ctctccagac aaagttcatt taaatcttca catcaattcc 240

ctggagcttg gtgactctgc tgtgtatttc tgtgccagca gccaaga 287

<210> 121
<211> 285
<212> DNA
<213> Artificial Sequence

<220>
<223> Synthetic DNA: TRBV3-2*03

<400> 121

```
gacacagccg tttcccagac tccaaaatac ctggtcacac agacgggaaa aaaggagtct      60
cttaaatgag aacaaaatct gggccataat gctatgtatt ggtataaaca ggactctaag     120
aaatttctga agacaatgtt tatctacagt aacaaggagc caattttaaa tgaaacagtt     180
ccaaatcgct tctcacctga ctctccagac aaagttcatt taaatcttca catcaattcc     240
ctggagcttg gtgactctgc tgtgtatttc tgtgccagca gccaa                     285
```

<210> 122
<211> 287
<212> DNA
<213> Artificial Sequence

<220>
<223> Synthetic DNA: TRBV4-1*01

<400> 122

```
gacactgaag ttacccagac accaaaacac ctggtcatgg gaatgacaaa taagaagtct      60
ttgaaatgtg aacaacatat ggggcacagg gctatgtatt ggtacaagca gaaagctaag     120
aagccaccgg agctcatgtt tgtctacagc tatgagaaac tctctataaa tgaaagtgtg     180
ccaagtcgct tctcacctga atgccccaac agctctctct taaaccttca cctacacgcc     240
ctgcagccag aagactcagc cctgtatctc tgcgccagca gccaaga                   287
```

<210> 123
<211> 258
<212> DNA
<213> Artificial Sequence

<220>
<223> Synthetic DNA: TRBV4-1*02

<400> 123

```
cacctggtca tgggaatgac aaataagaag tctttgaaat gtgaacaaca tatggggcac        60
agggcaatgt attggtacaa gcagaaagct aagaagccac cggagctcat gtttgtctac       120
agctatgaga aactctctat aaatgaaagt gtgccaagtc gcttctcacc tgaatgcccc       180
aacagctctc tcttaaacct tcacctacac gccctgcagc cagaagactc agccctgtat       240
ctctgcgcca gcagccaa                                                     258
```

<210> 124
<211> 287
<212> DNA
<213> Artificial Sequence

<220>
<223> Synthetic DNA: TRBV4-2*01

<400> 124

```
gaaacgggag ttacgcagac accaagacac ctggtcatgg gaatgacaaa taagaagtct        60
ttgaaatgtg aacaacatct ggggcataac gctatgtatt ggtacaagca aagtgctaag       120
aagccactgg agctcatgtt tgtctacaac tttaaagaac agactgaaaa caacagtgtg       180
ccaagtcgct tctcacctga atgccccaac agctctcact tattccttca cctacacacc       240
ctgcagccag aagactcggc cctgtatctc tgtgccagca gccaaga                     287
```

<210> 125
<211> 282
<212> DNA
<213> Artificial Sequence

<220>
<223> Synthetic DNA: TRBV4-2*02

<400> 125

```
gaaacgggag ttacgcagac accaagacac ctggtcatgg gaatgacaaa taagaagtct        60
ttgaaatgtg aacaacatct ggggcataac gctatgtatt ggtacaagca aagtgctaag       120
aagccactgg agctcatgtt tgtctacaac tttaaagaac agactgaaaa caacagtgtg       180
ccaagtcgct tctcacctga atgccccaac agctctcact tatgccttca cctacacacc       240
ctgcagccag aagactcggc cctgtatctc tgtgccagca cc                          282
```

<210> 126
<211> 287
<212> DNA
<213> Artificial Sequence

<220>
<223> Synthetic DNA: TRBV4-3*01

<400> 126

gaaacgggag ttacgcagac accaagacac ctggtcatgg gaatgacaaa taagaagtct 60
ttgaaatgtg aacaacatct gggtcataac gctatgtatt ggtacaagca aagtgctaag 120
aagccactgg agctcatgtt tgtctacagt cttgaagaac gggttgaaaa caacagtgtg 180
ccaagtcgct tctcacctga atgccccaac agctctcact tattccttca cctacacacc 240
ctgcagccag aagactcggc cctgtatctc tgcgccagca gccaaga 287

<210> 127
<211> 282
<212> DNA
<213> Artificial Sequence

<220>
<223> Synthetic DNA: TRBV4-3*02

<400> 127

gaaacgggag ttacgcagac accaagacac ctggtcatgg gaatgacaaa taagaagtct 60
ttgaaatgtg aacaacatct gggtcataac gctatgtatt ggtacaagca aagtgctaag 120
aagccactgg agctcatgtt tgtctacagt cttgaagaac gggttgaaaa caacagtgtg 180
ccaagtcgct tctcacctga atgccccaac agctctcact tatcccttca cctacacacc 240
ctgcagccag aagactcggc cctgtatctc tgcgccagca gc 282

<210> 128
<211> 282
<212> DNA
<213> Artificial Sequence

<220>
<223> Synthetic DNA: TRBV4-3*03

<400> 128

gaaacgggag ttacgcagac accaagacac ctggtcatgg gaatgacaaa taagaagtct 60
ttgaaatgtg aacaacatct gggtcataac gctatgtatt ggtacaagca aagtgctaag 120
aagccactgg agctcatgtt tgtctacagt cttgaagaac gtgttgaaaa caacagtgtg 180
ccaagtcgct tctcacctga atgccccaac agctctcact tattccttca cctacacacc 240
ctgcagccag aagactcggc cctgtatctc tgcgccagca gc 282

<210> 129
<211> 231
<212> DNA
<213> Artificial Sequence

<220>

<223> Synthetic DNA: TRBV4-3*04

<400> 129

```
aagaagtctt tgaaatgtga acaacatctg gggcataacg ctatgtattg gtacaagcaa      60
agtgctaaga agccactgga gctcatgttt gtctacagtc ttgaagaacg ggttgaaaac     120
aacagtgtgc caagtcgctt ctcacctgaa tgccccaaca gctctcactt attccttcac     180
ctacacaccc tgcagccaga agactcggcc ctgtatctct gcgccagcag c              231
```

<210> 130
<211> 286
<212> DNA
<213> Artificial Sequence

<220>
<223> Synthetic DNA: TRBV5-1*01

<400> 130

```
aaggctggag tcactcaaac tccaagatat ctgatcaaaa cgagaggaca gcaagtgaca      60
ctgagctgct cccctatctc tgggcatagg agtgtatcct ggtaccaaca gaccccagga     120
cagggccttc agttcctctt tgaatacttc agtgagacac agagaaacaa aggaaacttc     180
cctggtcgat tctcagggcg ccagttctct aactctcgct ctgagatgaa tgtgagcacc     240
ttggagctgg gggactcggc cctttatctt tgcgccagca gcttgg                    286
```

<210> 131
<211> 285
<212> DNA
<213> Artificial Sequence

<220>
<223> Synthetic DNA: TRBV5-1*02

<400> 131

```
agggctgggg tcactcaaac tccaagacat ctgatcaaaa cgagaggaca gcaagtgaca      60
ctgggctgct cccctatctc tgggcatagg agtgtatcct ggtaccaaca gaccctagga     120
cagggccttc agttcctctt tgaatacttc agtgagacac agagaaacaa aggaaacttc     180
cttggtcgat tctcagggcg ccagttctct aactctcgct ctgagatgaa tgtgagcacc     240
ttggagctgg gggactcggc cctttatctt tgcgccagcg cttgc                     285
```

<210> 132
<211> 286
<212> DNA
<213> Artificial Sequence

<220>
<223> Synthetic DNA: TRBV5-3*01

<400> 132

```
gaggctggag tcacccaaag tcccacacac ctgatcaaaa cgagaggaca gcaagtgact      60
ctgagatgct ctcctatctc tgggcacagc agtgtgtcct ggtaccaaca ggccccgggt     120
caggggcccc agtttatctt tgaatatgct aatgagttaa ggagatcaga aggaaacttc     180
cctaatcgat tctcagggcg ccagttccat gactgttgct ctgagatgaa tgtgagtgcc     240
ttggagctgg gggactcggc cctgtatctc tgtgccagaa gcttgg                    286
```

<210> 133
<211> 286
<212> DNA
<213> Artificial Sequence

<220>
<223> Synthetic DNA: TRBV5-3*02

<400> 133

```
gaggctggag tcacccaaag tcccacacac ctgatcaaaa cgagaggaca gcaagtgact      60
ctgagatgct ctcctatctc tgggcacagc agtgtgtcct ggtaccaaca ggccccgggt     120
caggggcccc agtttatctt tgaatatgct aatgagttaa ggagatcaga aggaaacttc     180
cctaatcgat tctcagggcg ccagttccat gactattgct ctgagatgaa tgtgagtgcc     240
ttggagctgg gggactcggc cctgtatctc tgtgccagaa gcttgg                    286
```

<210> 134
<211> 286
<212> DNA
<213> Artificial Sequence

<220>
<223> Synthetic DNA: TRBV5-4*01

<400> 134

```
gagactggag tcacccaaag tcccacacac ctgatcaaaa cgagaggaca gcaagtgact      60
ctgagatgct cttctcagtc tgggcacaac actgtgtcct ggtaccaaca ggccctgggt     120
caggggcccc agtttatctt tcagtattat agggaggaag agaatggcag aggaaacttc     180
cctcctagat tctcaggtct ccagttccct aattatagct ctgagctgaa tgtgaacgcc     240

ttggagctgg acgactcggc cctgtatctc tgtgccagca gcttgg                    286
```

<210> 135
<211> 282

<212> DNA
<213> Artificial Sequence

<220>
<223> Synthetic DNA: TRBV5-4*02

<400> 135

```
gagactggag tcacccaaag tcccacacac ctgatcaaaa cgagaggaca gcaagtgact    60
ctgagatgct cttctcagtc tgggcacaac actgtgtcct ggtaccaaca ggccctgggt   120
caggggcccc agtttatctt tcagtattat agggaggaag agaatggcag aggaaacttc   180
cctcctagat tctcaggtct ccagttccct aattataact ctgagctgaa tgtgaacgcc   240
ttggagctgg acgactcggc cctgtatctc tgtgccagca gc                      282
```

<210> 136
<211> 234
<212> DNA
<213> Artificial Sequence

<220>
<223> Synthetic DNA: TRBV5-4*03

<400> 136

```
cagcaagtga cactgagatg ctcttctcag tctgggcaca acactgtgtc ctggtaccaa    60
caggccctgg gtcaggggcc ccagtttatc tttcagtatt atagggagga agagaatggc   120
agaggaaact tccctcctag attctcaggt ctccagttcc ctaattatag ctctgagctg   180
aatgtgaacg ccttggagct ggacgactcg gccctgtatc tctgtgccag cagc         234
```

<210> 137
<211> 192
<212> DNA
<213> Artificial Sequence

<220>
<223> Synthetic DNA: TRBV5-4*04

<400> 137

```
actgtgtcct ggtaccaaca ggccctgggt caggggcccc agtttatctt tcagtattat    60
agggaggaag agaatggcag aggaaactcc cctcctagat tctcaggtct ccagttccct   120
aattatagct ctgagctgaa tgtgaacgcc ttggagctgg acgactcggc cctgtatctc   180
tgtgccagca gc                                                       192
```

<210> 138
<211> 286
<212> DNA
<213> Artificial Sequence

<220>
<223> Synthetic DNA: TRBV5-5*01

<400> 138

```
gacgctggag tcacccaaag tcccacacac ctgatcaaaa cgagaggaca gcaagtgact        60
ctgagatgct ctcctatctc tgggcacaag agtgtgtcct ggtaccaaca ggtcctgggt       120
caggggcccc agtttatctt tcagtattat gagaaagaag agagaggaag aggaaacttc       180
cctgatcgat tctcagctcg ccagttccct aactatagct ctgagctgaa tgtgaacgcc       240
ttgttgctgg gggactcggc cctgtatctc tgtgccagca gcttgg                      286
```

<210> 139
<211> 282
<212> DNA
<213> Artificial Sequence

<220>
<223> Synthetic DNA: TRBV5-5*02

<400> 139

```
gacgctggag tcacccaaag tcccacacac ctgatcaaaa cgagaggaca gcacgtgact        60
ctgagatgct ctcctatctc tgggcacaag agtgtgtcct ggtaccaaca ggtcctgggt       120
caggggcccc agtttatctt tcagtattat gagaaagaag agagaggaag aggaaacttc       180
cctgatcgat tctcagctcg ccagttccct aactatagct ctgagctgaa tgtgaacgcc       240
ttgttgctgg gggactcggc cctgtatctc tgtgccagca gc                          282
```

<210> 140
<211> 282
<212> DNA
<213> Artificial Sequence

<220>
<223> Synthetic DNA: TRBV5-5*03

<400> 140

```
gacgctggag tcacccaaag tcccacacac ctgatcaaaa cgagaggaca gcaagtgact        60
ctgagatgct ctcctatctc tgagcacaag agtgtgtcct ggtaccaaca ggtcctgggt       120
caggggcccc agtttatctt tcagtattat gagaaagaag agagaggaag aggaaacttc       180
cctgatcgat tctcagctcg ccagttccct aactatagct ctgagctgaa tgtgaacgcc       240
ttgttgctgg gggactcggc cctgtatctc tgtgccagca gc                          282
```

<210> 141
<211> 286
<212> DNA

<213> Artificial Sequence

<220>
<223> Synthetic DNA: TRBV5-6*01

<400> 141

```
gacgctggag tcacccaaag tcccacacac ctgatcaaaa cgagaggaca gcaagtgact        60
ctgagatgct ctcctaagtc tgggcatgac actgtgtcct ggtaccaaca ggccctgggt       120
caggggcccc agtttatctt tcagtattat gaggaggaag agagacagag aggcaacttc       180
cctgatcgat tctcaggtca ccagttccct aactatagct ctgagctgaa tgtgaacgcc       240
ttgttgctgg gggactcggc cctctatctc tgtgccagca gcttgg                      286
```

<210> 142
<211> 286
<212> DNA
<213> Artificial Sequence

<220>
<223> Synthetic DNA: TRBV5-7*01

<400> 142

```
gacgctggag tcacccaaag tcccacacac ctgatcaaaa cgagaggaca gcacgtgact        60
ctgagatgct ctcctatctc tgggcacacc agtgtgtcct cgtaccaaca ggccctgggt       120
caggggcccc agtttatctt tcagtattat gagaaagaag agagaggaag aggaaacttc       180
cctgatcaat tctcaggtca ccagttccct aactatagct ctgagctgaa tgtgaacgcc       240
ttgttgctag gggactcggc cctctatctc tgtgccagca gcttgg                      286
```

<210> 143
<211> 286
<212> DNA
<213> Artificial Sequence

<220>
<223> Synthetic DNA: TRBV5-8*01

<400> 143

```
gaggctggag tcacacaaag tcccacacac ctgatcaaaa cgagaggaca gcaagcgact        60
ctgagatgct ctcctatctc tgggcacacc agtgtgtact ggtaccaaca ggccctgggt       120
ctgggcctcc agttcctcct ttggtatgac gagggtgaag agagaaacag aggaaacttc       180
cctcctagat tttcaggtcg ccagttccct aattatagct ctgagctgaa tgtgaacgcc       240
ttggagctgg aggactcggc cctgtatctc tgtgccagca gcttgg                      286
```

<210> 144
<211> 238
<212> DNA
<213> Artificial Sequence

<220>
<223> Synthetic DNA: TRBV5-8*02

<400> 144

aggacagcaa gcgactctga gatgctctcc tatctctggg cacaccagtg tgtactggta 60

ccaacaggcc ctgggtctgg gcctccagct cctcctttgg tatgacgagg gtgaagagag 120

aaacagagga aacttccctc ctagattttc aggtcgccag ttccctaatt atagctctga 180

gctgaatgtg aacgccttgg agctggagga ctcggccctg tatctctgtg ccagcagc 238

<210> 145
<211> 287
<212> DNA
<213> Artificial Sequence

<220>
<223> Synthetic DNA: TRBV6-1*01

<400> 145

aatgctggtg tcactcagac cccaaaattc caggtcctga agacaggaca gagcatgaca 60

ctgcagtgtg cccaggatat gaaccataac tccatgtact ggtatcgaca agacccaggc 120

atgggactga ggctgattta ttactcagct tctgagggta ccactgacaa aggagaagtc 180

cccaatggct acaatgtctc cagattaaac aaacgggagt tctcgctcag gctggagtcg 240

gctgctccct cccagacatc tgtgtacttc tgtgccagca gtgaagc 287

<210> 146
<211> 287
<212> DNA
<213> Artificial Sequence

<220>
<223> Synthetic DNA: TRBV6-2*01

<400> 146

aatgctggtg tcactcagac cccaaaattc cgggtcctga agacaggaca gagcatgaca 60

ctgctgtgtg cccaggatat gaaccatgaa tacatgtact ggtatcgaca agacccaggc 120

atggggctga ggctgattca ttactcagtt ggtgagggta caactgccaa aggagaggtc 180

cctgatggct acaatgtctc cagattaaaa aaacagaatt tcctgctggg gttggagtcg 240

gctgctccct cccaaacatc tgtgtacttc tgtgccagca gttactc 287

<210> 147
<211> 287
<212> DNA
<213> Artificial Sequence

<220>
<223> Synthetic DNA: TRBV6-3*01

<400> 147

aatgctggtg tcactcagac cccaaaattc cgggtcctga agacaggaca gagcatgaca 60

ctgctgtgtg cccaggatat gaaccatgaa tacatgtact ggtatcgaca agacccaggc 120

atggggctga ggctgattca ttactcagtt ggtgagggta caactgccaa aggagaggtc 180

cctgatggct acaatgtctc cagattaaaa aaacagaatt tcctgctggg gttggagtcg 240

gctgctccct cccaaacatc tgtgtacttc tgtgccagca gttactc 287

<210> 148
<211> 287
<212> DNA
<213> Artificial Sequence

<220>
<223> Synthetic DNA: TRBV6-4*01

<400> 148

attgctggga tcacccaggc accaacatct cagatcctgg cagcaggacg gcgcatgaca 60

ctgagatgta cccaggatat gagacataat gccatgtact ggtatagaca agatctagga 120

ctggggctaa ggctcatcca ttattcaaat actgcaggta ccactggcaa aggagaagtc 180

cctgatggtt atagtgtctc cagagcaaac acagatgatt tcccccctcac gttggcgtct 240

gctgtaccct ctcagacatc tgtgtacttc tgtgccagca gtgactc 287

<210> 149
<211> 287
<212> DNA
<213> Artificial Sequence

<220>
<223> Synthetic DNA: TRBV6-4*02

<400> 149

actgctggga tcacccaggc accaacatct cagatcctgg cagcaggacg gagcatgaca 60
ctgagatgta cccaggatat gagacataat gccatgtact ggtatagaca agatctagga 120
ctggggctaa ggctcatcca ttattcaaat actgcaggta ccactggcaa aggagaagtc 180
cctgatggtt atagtgtctc cagagcaaac acagatgatt tccccctcac gttggcgtct 240
gctgtaccct ctcagacatc tgtgtacttc tgtgccagca gtgactc 287

<210> 150
<211> 287
<212> DNA
<213> Artificial Sequence

<220>
<223> Synthetic DNA: TRBV6-5*01

<400> 150

aatgctggtg tcactcagac cccaaaattc caggtcctga agacaggaca gagcatgaca 60
ctgcagtgtg cccaggatat gaaccatgaa tacatgtcct ggtatcgaca agacccaggc 120
atggggctga ggctgattca ttactcagtt ggtgctggta tcactgacca aggagaagtc 180
cccaatggct acaatgtctc cagatcaacc acagaggatt tcccgctcag gctgctgtcg 240
gctgctccct cccagacatc tgtgtacttc tgtgccagca gttactc 287

<210> 151
<211> 287
<212> DNA
<213> Artificial Sequence

<220>
<223> Synthetic DNA: TRBV6-6*01

<400> 151

aatgctggtg tcactcagac cccaaaattc cgcatcctga agataggaca gagcatgaca 60
ctgcagtgta cccaggatat gaaccataac tacatgtact ggtatcgaca agacccaggc 120
atggggctga agctgattta ttattcagtt ggtgctggta tcactgataa aggagaagtc 180
ccgaatggct acaacgtctc cagatcaacc acagaggatt tcccgctcag gctggagttg 240
gctgctccct cccagacatc tgtgtacttc tgtgccagca gttactc 287

<210> 152
<211> 282
<212> DNA
<213> Artificial Sequence

<220>
<223> Synthetic DNA: TRBV6-6*02

<400> 152

```
aatgctggtg tcactcagac cccaaaattc cgcatcctga agataggaca gagcatgaca      60
ctgcagtgtg cccaggatat gaaccataac tacatgtact ggtatcgaca agacccaggc     120
atggggctga agctgattta ttattcagtt ggtgctggta tcactgacaa aggagaagtc     180
ccgaatggct acaacgtctc cagatcaacc acagaggatt tcccgctcag gctggagttg     240
gctgctccct cccagacatc tgtgtacttc tgtgccagca gt                        282
```

<210> 153
<211> 282
<212> DNA
<213> Artificial Sequence

<220>
<223> Synthetic DNA: TRBV6-6*03

<400> 153

```
aatgctggtg tcactcagac cccaaaattc cgcatcctga agataggaca gagcatgaca      60
ctgcagtgtg cccaggatat gaaccataac tacatgtact ggtatcgaca agacccaggc     120
atggggctga agctgattta ttattcagtt ggtgctggta tcactgataa aggagaagtc     180
ccgaatggct acaacgtctc cagatcaacc acagaggatt tcccgctcag gctggagttg     240
gctgctccct cccagacatc tgtgtacttc tgtgccagca gt                        282
```

<210> 154
<211> 285
<212> DNA
<213> Artificial Sequence

<220>
<223> Synthetic DNA: TRBV6-6*04

<400> 154

```
aatgctggtg tcactcagac cccaaaattc cgcatcctga agataggaca gagcatgaca      60
ctgcagtgta cccaggatat gaaccatgaa tacatgtact ggtatcgaca agacccaggc     120
atggggctga agctgattta ttattcagtt ggtgctggta tcactgataa aggagaagtc     180
ccgaatggct acaatgtctc cagatcaacc acagaggatt tcccgctcag gctggagttg     240
gctgctccct cccagacatc tgtgtacttc tgtgccagca gtcga                     285
```

<210> 155
<211> 282
<212> DNA
<213> Artificial Sequence

<220>
<223> Synthetic DNA: TRBV6-6*05

<400> 155

```
aatgctggtg tcactcagac cccaaaattc cgcatcctga agataggaca gagcatgaca      60
ctgcagtgtg cccaggatat gaaccataac tacatgtact ggtatcgaca agacccaggc     120
atggggctga agctgattta ttattcagtt ggtgctggta tcactgacaa aggagaagtc     180
ccgaatggct acaacgtctc cagatcaacc acagaggatt tcccgctcag gctggagttg     240
gctgctgcct cccagacatc tgtgtacttc tgtgccagca gc                        282
```

<210> 156
<211> 287
<212> DNA
<213> Artificial Sequence

<220>
<223> Synthetic DNA: TRBV6-7*01

<400> 156

```
aatgctggtg tcactcagac cccaaaattc cacgtcctga agacaggaca gagcatgact      60
ctgctgtgtg cccaggatat gaaccatgaa tacatgtatc ggtatcgaca agacccaggc     120
aaggggctga ggctgattta ctactcagtt gctgctgctc tcactgacaa aggagaagtt     180
cccaatggct acaatgtctc cagatcaaac acagaggatt tccccctcaa gctggagtca     240
gctgctccct ctcagacttc tgtttacttc tgtgccagca gttactc                   287
```

<210> 157
<211> 284
<212> DNA
<213> Artificial Sequence

<220>
<223> Synthetic DNA: TRBV6-8*01

<400> 157

```
aatgctggtg tcactcagac cccaaaattc cacatcctga agacaggaca gagcatgaca      60
ctgcagtgtg cccaggatat gaaccatgga tacatgtcct ggtatcgaca agacccaggc     120
atggggctga gactgattta ctactcagct gctgctggta ctactgacaa agaagtcccc     180
aatggctaca atgtctctag attaaacaca gaggatttcc cactcaggct ggtgtcggct     240
gctccctccc agacatctgt gtacttgtgt gccagcagtt actc                      284
```

<210> 158
<211> 287
<212> DNA

<213> Artificial Sequence

<220>
<223> Synthetic DNA: TRBV6-9*01

<400> 158

aatgctggtg tcactcagac cccaaaattc cacatcctga agacaggaca gagcatgaca 60

ctgcagtgtg cccaggatat gaaccatgga tacttgtcct ggtatcgaca agacccaggc 120

atggggctga ggcgcattca ttactcagtt gctgctggta tcactgacaa aggagaagtc 180

cccgatggct acaatgtatc cagatcaaac acagaggatt tcccgctcag gctggagtca 240

gctgctccct cccagacatc tgtatacttc tgtgccagca gttattc 287

<210> 159
<211> 290
<212> DNA
<213> Artificial Sequence

<220>
<223> Synthetic DNA: TRBV7-1*01

<400> 159

ggtgctggag tctcccagtc cctgagacac aaggtagcaa agaagggaaa ggatgtagct 60

ctcagatatg atccaatttc aggtcataat gccctttatt ggtaccgaca gagcctgggg 120

cagggcctgg agtttccaat ttacttccaa ggcaaggatg cagcagacaa atcggggctt 180

ccccgtgatc ggttctctgc acagaggtct gagggatcca tctccactct gaagttccag 240

cgcacacagc agggggactt ggctgtgtat ctctgtgcca gcagctcagc 290

<210> 160
<211> 290
<212> DNA
<213> Artificial Sequence

<220>
<223> Synthetic DNA: TRBV7-2*01

<400> 160

ggagctggag tctcccagtc ccccagtaac aaggtcacag agaagggaaa ggatgtagag 60

ctcaggtgtg atccaatttc aggtcatact gccctttact ggtaccgaca gagcctgggg 120

cagggcctgg agtttttaat ttacttccaa ggcaacagtg caccagacaa atcagggctg 180

cccagtgatc gcttctctgc agagaggact gggggatccg tctccactct gacgatccag 240

cgcacacagc aggaggactc ggccgtgtat ctctgtgcca gcagcttagc 290

<210> 161

<211> 290
<212> DNA
<213> Artificial Sequence

<220>
<223> Synthetic DNA: TRBV7-2*02

<400> 161

```
ggagctggag tctcccagtc ccccagtaac aaggtcacag agaagggaaa ggatgtagag        60
ctcaggtgtg atccaatttc aggtcatact gccctttact ggtaccgaca gaggctgggg       120
cagggcctgg agtttttaat ttacttccaa ggcaacagtg caccagacaa atcagggctg       180
cccagtgatc gcttctctgc agagaggact ggggaatccg tctccactct gacgatccag       240
cgcacacagc aggaggactc ggccgtgtat ctctgtgcca gcagcttagc                  290
```

<210> 162
<211> 290
<212> DNA
<213> Artificial Sequence

<220>
<223> Synthetic DNA: TRBV7-2*03

<400> 162

```
ggagctggag tctcccagtc ccccagtaac aaggtcacag agaagggaaa ggatgtagag        60
ctcaggtgtg atccaatttc aggtcatact gccctttact ggtaccgaca gaggctgggg       120
cagggcctgg agtttttaat ttacttccaa ggcaacagtg caccagacaa atcagggctg       180
cccagtgatc gcttctctgc agagaggact ggggaatccg tctccactct gacgatccag       240
cgcacacagc aggaggactc ggccgtgtat ctctgtacca gcagcttagc                  290
```

<210> 163
<211> 288
<212> DNA
<213> Artificial Sequence

<220>
<223> Synthetic DNA: TRBV7-2*04

<400> 163

```
ggagctggag tttcccagtc ccccagtaac aaggtcacag agaagggaaa ggatgtagag        60
ctcaggtgtg atccaatttc aggtcatact gccctttact ggtaccgaca gagcctgggg       120
cagggcctgg agtttttaat ttacttccaa ggcaacagtg caccagacaa atcagggctg       180
cccagtgatc gcttctctgc agagaggact gggggatccg tctccactct gacgatccag       240
cgcacacagc aggaggactc ggccgtgtat ctctgtgcca gcagctta                    288
```

<210> 164
<211> 290
<212> DNA
<213> Artificial Sequence

<220>
<223> Synthetic DNA: TRBV7-3*01

<400> 164

ggtgctggag tctcccagac ccccagtaac aaggtcacag agaagggaaa atatgtagag 60

ctcaggtgtg atccaatttc aggtcatact gccctttact ggtaccgaca aagcctgggg 120

cagggcccag agtttctaat ttacttccaa ggcacgggtg cggcagatga ctcagggctg 180

cccaacgatc ggttctttgc agtcaggcct gagggatccg tctctactct gaagatccag 240

cgcacagagc gggggactc agccgtgtat ctctgtgcca gcagcttaac 290

<210> 165
<211> 290
<212> DNA
<213> Artificial Sequence

<220>
<223> Synthetic DNA: TRBV7-3*02

<400> 165

ggtgctggag tctcccagac ccccagtaac aaggtcacag agaagggaaa agatgtagag 60

ctcaggtgtg atccaatttc aggtcatact gccctttact ggtaccgaca aagcctgggg 120

cagggcccag agtttctaat ttacttccaa ggcacgggtg cggcagatga ctcagggctg 180

cccaaagatc ggttctttgc agtcaggcct gagggatccg tctctactct gaagatccag 240

cgcacagagc aggggactc agccgtgtat ctccgtgcca gcagcttaac 290

<210> 166
<211> 288
<212> DNA
<213> Artificial Sequence

<220>
<223> Synthetic DNA: TRBV7-3*03

<400> 166

```
ggtgctggag tctcccagac ccccagtaac aaggtcacag agaagggaaa agatgtagag        60
ctcaggtgtg atccaatttc aggtcatact gccctttact ggtaccgaca aagcctgggg       120
cagggcccag agtttctaat ttacttccaa ggcacgggtg cggcagatga ctcagggctg       180
cccaaagatc ggttctttgc agtcaggcct gagggatccg tctctactct gaagatccag       240
cgcacagagc aggggactc agccgcgtat ctccgtgcca gcagctta                     288
```

<210> 167
<211> 285
<212> DNA
<213> Artificial Sequence

<220>
<223> Synthetic DNA: TRBV7-3*04

<400> 167

```
ggtgctggag tctcccagac ccccagtaac aaggtcacag agaagggaaa atatgtagag        60
ctcaggtgtg atccaatttc aggtcatact gccctttact ggtaccgaca aagcctgggg       120
cagggcccag agtttctaat ttacttccaa ggcacgggtg cggcagatga ctcagggctg       180
cccaacgatc ggttctttgc agtcaggcct gagggatccg tctctactct gaagatccag       240
cgcacagagc gggggactc tgccgtgtat ctctgtgcca gcagc                        285
```

<210> 168
<211> 231
<212> DNA
<213> Artificial Sequence

<220>
<223> Synthetic DNA: TRBV7-3*05

<400> 168

```
tgggagctca ggtgtgatcc aatttcaggt catactgccc tttactggta ccgacaaagc        60
ctggggcagg gcccagagct tctaatttac ttccaaggca cgggtgcggc agatgactca       120
gggctgccca acgatcggtt ctttgcagtc aggcctgagg gatccgtctc tactctgaag       180
atccagcgca cagagcgggg ggactcagcc gtgtatctct gtgccagcag c                231
```

<210> 169
<211> 290
<212> DNA
<213> Artificial Sequence

<220>
<223> Synthetic DNA: TRBV7-4*01

<400> 169

ggtgctggag tctcccagtc cccaaggtac aaagtcgcaa agaggggacg ggatgtagct 60

ctcaggtgtg attcaatttc gggtcatgta accctttatt ggtaccgaca gaccctgggg 120

cagggctcag aggttctgac ttactcccag agtgatgctc aacgagacaa atcagggcgg 180

cccagtggtc ggttctctgc agagaggcct gagagatccg tctccactct gaagatccag 240

cgcacagagc agggggactc agctgtgtat ctctgtgcca gcagcttagc 290

<210> 170
<211> 288
<212> DNA
<213> Artificial Sequence

<220>
<223> Synthetic DNA: TRBV7-5*01

<400> 170

ggtgctggag tctcccagtc cccaaggtac gaagtcacac agaggggaca ggatgtagct 60

cccaggtgtg atccaatttc gggtcaggta accctttatt ggtaccgaca gaccctgggg 120

cagggccaag agtttctgac ttccttccag gatgaaactc aacaagataa atcagggctg 180

ctcagtgatc aattctccac agagaggtct gaggatcttt ctccacctga agatccagcg 240

cacagagcaa gggcgactcg gctgtgtatc tctgtgccag aagcttag 288

<210> 171
<211> 289
<212> DNA
<213> Artificial Sequence

<220>
<223> Synthetic DNA: TRBV7-5*02

<400> 171

ggtgctggag tctcccagtc cccaaggtac gaagtcacac agaggggaca ggatgtagct 60

cccaggtgtg atccaatttc gggtcaggta accctttatt ggtaccgaca gaccctgggg 120

cagggccaag agtttctgac ttccttccag gatgaaactc aacaagataa atcagggctg 180

ctcagtgatc aattctccac agagaggtct gaggatcttt ctccacctga agatccagcg 240

cacagagcaa gggcgactcg gctgtgtatc tctgtgtcag aagcttagc 289

<210> 172
<211> 290
<212> DNA
<213> Artificial Sequence

<220>
<223> Synthetic DNA: TRBV7-6*01

<400> 172

```
ggtgctggag tctcccagtc tcccaggtac aaagtcacaa agaggggaca ggatgtagct      60
ctcaggtgtg atccaatttc gggtcatgta tccctttatt ggtaccgaca ggccctgggg     120
cagggcccag agtttctgac ttacttcaat tatgaagccc aacaagacaa atcagggctg     180
cccaatgatc ggttctctgc agagaggcct gagggatcca tctccactct gacgatccag     240
cgcacagagc agcgggactc ggccatgtat cgctgtgcca gcagcttagc                290
```

<210> 173
<211> 285
<212> DNA
<213> Artificial Sequence

<220>
<223> Synthetic DNA: TRBV7-6*02

<400> 173

```
ggtgctggag tctcccagtc tcccaggtac aaagtcacaa agaggggaca ggatgtagct      60
ctcaggtgtg atccaatctc gggtcatgta tccctttatt ggtaccgaca ggccctgggg     120
cagggcccag agtttctgac ttacttcaat tatgaagccc aacaagacaa atcagggctg     180
cccaatgatc ggttctctgc agagaggcct gagggatcca tctccactct gacgatccag     240
cgcacagagc agcgggactc ggccatgtat cgctgtgcca gcagc                     285
```

<210> 174
<211> 290
<212> DNA
<213> Artificial Sequence

<220>
<223> Synthetic DNA: TRBV7-7*01

<400> 174

```
ggtgctggag tctcccagtc tcccaggtac aaagtcacaa agaggggaca ggatgtaact      60
ctcaggtgtg atccaatttc gagtcatgca accctttatt ggtatcaaca ggccctgggg     120
cagggcccag agtttctgac ttacttcaat tatgaagctc aaccagacaa atcagggctg     180
cccagtgatc ggttctctgc agagaggcct gagggatcca tctccactct gacgattcag     240
cgcacagagc agcgggactc agccatgtat cgctgtgcca gcagcttagc                290
```

<210> 175
<211> 285
<212> DNA
<213> Artificial Sequence

<220>

<223> Synthetic DNA: TRBV7-7*02

<400> 175

ggtgctggag tctcccagtc tcccaggtac aaagtcacaa agaggggaca ggatgtaact 60

ctcaggtgtg atccaatttc gagtcatgta accctttatt ggtatcaaca ggccctgggg 120

cagggcccag agtttctgac ttacttcaat tatgaagctc aaccagacaa atcagggctg 180

cccagtgatc ggttctctgc agagaggcct gagggatcca tctccactct gacgattcag 240

cgcacagagc agcgggactc agccatgtat cgctgtgcca gcagc 285

<210> 176
<211> 290
<212> DNA
<213> Artificial Sequence

<220>
<223> Synthetic DNA: TRBV7-8*01

<400> 176

ggtgctggag tctcccagtc ccctaggtac aaagtcgcaa agagaggaca ggatgtagct 60

ctcaggtgtg atccaatttc gggtcatgta tccctttttt ggtaccaaca ggccctgggg 120

caggggccag agtttctgac ttatttccag aatgaagctc aactagacaa atcggggctg 180

cccagtgatc gcttctttgc agaaaggcct gagggatccg tctccactct gaagatccag 240

cgcacacagc aggaggactc cgccgtgtat ctctgtgcca gcagcttagc 290

<210> 177
<211> 290
<212> DNA
<213> Artificial Sequence

<220>
<223> Synthetic DNA: TRBV7-8*02

<400> 177

ggtgctggag tctcccagtc ccctaggtac aaagtcgcaa agagaggaca ggatgtagct 60

ctcaggtgtg atccaatttc gggtcatgta tccctttttt ggtaccaaca ggccctgggg 120

caggggccag agtttctgac ttatttccag aatgaagctc aactagacaa atcggggctg 180

cccagtgatc gcttctttgc agaaaggcct gagggatccg tctccactct gaagatccag 240

cgcacacaga aggaggactc cgccgtgtat ctctgtgcca gcagcttagc 290

<210> 178
<211> 288
<212> DNA

<213> Artificial Sequence

<220>
<223> Synthetic DNA: TRBV7-8*03

<400> 178

```
ggtgctggag tctcccagtc ccctaggtac aaagtcgcaa agagaggaca ggatgtagct      60
ctcaggtgtg atccaatttc gggtcatgta tccctttttt ggtaccaaca ggccctcggg     120
caggggccag agtttctgac ttatttccag aatgaagctc aactagacaa atcggggctg     180
cccagtgatc gcttctttgc agaaaggcct gagggatccg tctccactct gaagatccag     240
cgcacacagc aggaggactc cgccgtgtat ctctgtgcca gcagccga                 288
```

<210> 179
<211> 288
<212> DNA
<213> Artificial Sequence

<220>
<223> Synthetic DNA: TRBV7-9*05

<400> 179

```
gatactggag tctcccagaa ccccagacac aagatcacaa agaggggaca gaatgtaact      60
ttcaggtgtg atccaatttc tgaacacaac cgcctttatt ggtaccgaca gaccctgggg     120
cagggcccag agtttctgac ttacttccag aatgaagctc aactagaaaa atcaaggctg     180
ctcagtgatc ggttctctgc agagaggcct aagggatctc tctccacctt ggagatccag     240
cgcacagagc aggggggactc ggccatgtat ctctgtgcca gcaccaaa                288
```

<210> 180
<211> 288
<212> DNA
<213> Artificial Sequence

<220>
<223> Synthetic DNA: TRBV7-9*06

<400> 180

```
gatactggag tctcccagaa ccccagacac aagatcacaa agaggggaca gaatgtaact      60
ttcaggtgtg atccaatttc tgaacacaac cgcctttatt ggtaccgaca gaccctgggg     120
cagggcccag agtttctgac ttacttccag aatgaagctc aactagaaaa atcaaggctg     180
ctcagtgatc ggttctctgc agagaggcct aagggatctc tttccacctt ggagatccag     240
cgcacagagc aggggggactc ggccatgtat ctctgtgcca gcacgttg                288
```

<210> 181
<211> 285
<212> DNA
<213> Artificial Sequence

<220>
<223> Synthetic DNA: TRBV7-9*03

<400> 181

gatactggag tctcccagga ccccagacac aagatcacaa agaggggaca gaatgtaact 60

ttcaggtgtg atccaatttc tgaacacaac cgcctttatt ggtaccgaca gaccctgggg 120

cagggcccag agtttctgac ttacttccag aatgaagctc aactagaaaa atcaaggctg 180

ctcagtgatc ggttctctgc agagaggcct aagggatctt tctccacctt ggagatccag 240

cgcacagagc aggggggactc ggccatgtat ctctgtgcca gcagc 285

<210> 182
<211> 290
<212> DNA
<213> Artificial Sequence

<220>
<223> Synthetic DNA: TRBV7-9*01

<400> 182

gatactggag tctcccagaa ccccagacac aagatcacaa agaggggaca gaatgtaact 60

ttcaggtgtg atccaatttc tgaacacaac cgcctttatt ggtaccgaca gaccctgggg 120

cagggcccag agtttctgac ttacttccag aatgaagctc aactagaaaa atcaaggctg 180

ctcagtgatc ggttctctgc agagaggcct aagggatctt tctccacctt ggagatccag 240

cgcacagagc aggggggactc ggccatgtat ctctgtgcca gcagcttagc 290

<210> 183
<211> 288
<212> DNA
<213> Artificial Sequence

<220>
<223> Synthetic DNA: TRBV7-9*02

<400> 183

gatactggag tctcccagaa ccccagacac aacatcacaa agaggggaca gaatgtaact 60

ttcaggtgtg atccaatttc tgaacacaac cgcctttatt ggtaccgaca gaccctgggg 120

cagggcccag agtttctgac ttacttccag aatgaagctc aactagaaaa atcaaggctg 180

ctcagtgatc ggttctctgc agagaggcct aagggatctt tctccacctt ggagatccag 240

cgcacagagc aggggactc ggccatgtat ctctgtgcca gcagctta 288

<210> 184
<211> 207
<212> DNA
<213> Artificial Sequence

<220>
<223> Synthetic DNA: TRBV7-9*07

<400> 184

cacaaccgcc tttattggta ccgacagacc ctggggcagg gcccagagtt tctgacttac 60

ttccagaatg aagctcaact agaaaaatca aggctgctca gtgatcggtt ctctgcagag 120

aggcctaagg gatctttctc caccttggag atccagcgca cagaggaggg ggactcggcc 180

atgtatctct gtgccagcag cagcagt 207

<210> 185
<211> 288
<212> DNA
<213> Artificial Sequence

<220>
<223> Synthetic DNA: TRBV7-9*04

<400> 185

atatctggag tctcccacaa ccccagacac aagatcacaa agaggggaca gaatgtaact 60

ttcaggtgtg atccaatttc tgaacacaac cgcctttatt ggtaccgaca gaaccctggg 120

cagggcccag agtttctgac ttacttccag aatgaagctc aactggaaaa atcagggctg 180

ctcagtgatc ggatctctgc agagaggcct aagggatctt tctccacctt ggagatccag 240

cgcacagagc aggggactc ggccatgtat ctctgtgcca gcagctct 288

<210> 186
<211> 279
<212> DNA
<213> Artificial Sequence

<220>
<223> Synthetic DNA: TRBV8-1*01

<400> 186

gaggcaggga tcagccagat accaagatat cacagacaca cagggaaaaa gatcatcctg 60

aaatatgctc agattaggaa ccattattca gtgttctgtt atcaataaga ccaagaatag 120

gggctgaggc tgatccatta ttcaggtagt attggcagca tgaccaaagg cggtgccaag 180

gaagggtaca atgtctctgg aaacaagctc aagcattttc cctcaaccct ggagtctact 240

agcaccagcc agacctctgt acctctgtgg cagtgcatc 279

<210> 187
<211> 271
<212> DNA
<213> Artificial Sequence

<220>
<223> Synthetic DNA: TRBV8-2*01

<400> 187

gatgctggga tcacccagat gccaagatat cacattgtac agaagaaaga gatgatcctg 60

gaatgtgctc aggttaggaa cagtgttctg atatcgacag gacccaagac gggggctgaa 120

gcttatccac tattcaggca gtggtcacag caggaccaaa gttgatgtca cagaggggta 180

ctgtgtttct tgaaacaagc ttgagcattt ccccaatcct ggcatccacc agcaccagcc 240

agacctatct gtaccactgt ggcagcacat c 271

<210> 188
<211> 286
<212> DNA
<213> Artificial Sequence

<220>
<223> Synthetic DNA: TRBV9*01

<400> 188

gattctggag tcacacaaac cccaaagcac ctgatcacag caactggaca gcgagtgacg 60

ctgagatgct cccctaggtc tggagacctc tctgtgtact ggtaccaaca gagcctggac 120

cagggcctcc agttcctcat tcagtattat aatggagaag agagagcaaa aggaaacatt 180

cttgaacgat tctccgcaca acagttccct gacttgcact ctgaactaaa cctgagctct 240

ctggagctgg gggactcagc tttgtatttc tgtgccagca gcgtag 286

<210> 189
<211> 282
<212> DNA
<213> Artificial Sequence

<220>
<223> Synthetic DNA: TRBV9*03

<400> 189

gattctggag tcacacaaac cccaaagcac ctgatcacag caactggaca gcgagtgacg 60
ctgagatgct cccctaggtc tggagacctc tctgtgtact ggtaccaaca gagcctggac 120
cagggcctcc agttcctcat tcaatattat aatggagaag agagagcaaa aggaaacatt 180
cttgaacgat tctccgcaca acagttccct gacttgcact ctgaactaaa cctgagctct 240
ctggagctgg gggactcagc tttgtatttc tgtgccagca gc 282

<210> 190
<211> 286
<212> DNA
<213> Artificial Sequence

<220>
<223> Synthetic DNA: TRBV9*02

<400> 190

gattctggag tcacacaaac cccaaagcac ctgatcacag caactggaca gcgagtgacg 60
ctgagatgct cccctaggtc tggagacctc tctgtgtact ggtaccaaca gagcctggac 120
cagggcctcc agttcctcat tcactattat aatggagaag agagagcaaa aggaaacatt 180
cttgaacgat tctccgcaca acagttccct gacttgcact ctgaactaaa cctgagctct 240
ctggagctgg gggactcagc tttgtatttc tgtgccagca gcgtag 286

<210> 191
<211> 287
<212> DNA
<213> Artificial Sequence

<220>
<223> Synthetic DNA: TRBV10-1*01

<400> 191

gatgctgaaa tcacccagag cccaagacac aagatcacag agacaggaag gcaggtgacc 60
ttggcgtgtc accagacttg gaaccacaac aatatgttct ggtatcgaca agacctggga 120
catgggctga ggctgatcca ttactcatat ggtgttcaag acactaacaa aggagaagtc 180
tcagatggct acagtgtctc tagatcaaac acagaggacc tcccccctcac tctggagtct 240
gctgcctcct cccagacatc tgtatatttc tgcgccagca gtgagtc 287

<210> 192
<211> 282
<212> DNA
<213> Artificial Sequence

<220>

<223> Synthetic DNA: TRBV10-1*02

<400> 192

```
gatgctgaaa tcacccagag cccaagacac aagatcacag agacaggaag gcaggtgacc       60
ttggcgtgtc accagacttg gaaccacaac aatatgttct ggtatcgaca agacctggga      120
catgggctga ggctgatcca ttactcatat ggtgttcacg acactaacaa aggagaagtc      180
tcagatggct acagtgtctc tagatcaaac acagaggacc tcccccctcac tctggagtct     240
gctgcctcct cccagacatc tgtatatttc tgcgccagca gt                         282
```

<210> 193
<211> 287
<212> DNA
<213> Artificial Sequence

<220>
<223> Synthetic DNA: TRBV10-2*01

<400> 193

```
gatgctggaa tcacccagag cccaagatac aagatcacag agacaggaag gcaggtgacc       60
ttgatgtgtc accagacttg gagccacagc tatatgttct ggtatcgaca agacctggga      120
catgggctga ggctgatcta ttactcagca gctgctgata ttacagataa aggagaagtc      180
cccgatggct atgttgtctc cagatccaag acagagaatt tcccccctcac tctggagtca     240
gctacccgct cccagacatc tgtgtatttc tgcgccagca gtgagtc                    287
```

<210> 194
<211> 217
<212> DNA
<213> Artificial Sequence

<220>
<223> Synthetic DNA: TRBV10-2*02

<400> 194

```
aaggcaggtg accttgatgt gtcaccagac ttggagccac agctatatgt tctggtatcg       60
acaagacctg ggacatgggc tgaggctgat ctattactca gcagctgctg atattacaga      120
taaaggagaa gtccccgatg gctacgttgt ctccagatcc aagacagaga atttcccccct     180
cactctggag tcagctaccc gctcccagac atctgtg                               217
```

<210> 195
<211> 273
<212> DNA
<213> Artificial Sequence

<220>
<223> Synthetic DNA: TRBV10-3*03

<400> 195

```
gatgctggaa tcacccagag cccaagacac aaggtcacag agacaggaac accagtgact        60
ctgagatgtc accagactga gaaccaccgc tacatgtact ggtatcgaca agacccgggg       120
catgggctga ggctaatcca ttactcatat ggtgttaaag atactgacaa aggagaagtc       180
tcagatggct atagtgtctc tagatcaaag acagaggatt tcctcctcac tctggagtcc       240
gctaccagct cccagacatc tgtgtacttc tgt                                    273
```

<210> 196
<211> 273
<212> DNA
<213> Artificial Sequence

<220>
<223> Synthetic DNA: TRBV10-3*04

<400> 196

```
gatgctggaa tcacccagag cccaagacac aaggtcacag agacaggaac accagtgact        60
ctgagatgtc accagactga gaaccaccgc tacatgtact ggtatcgaca agacccgggg       120
catgggctga ggctgatcca ttactcatat ggtgttaaag atactgacaa aggagaagtc       180
tcagatggct atagtgtctc tagatcaaag acagaggatt tcctcctcac tctggagtcc       240
gctaccagct cccagacatc tgtgtacttc tgt                                    273
```

<210> 197
<211> 287
<212> DNA
<213> Artificial Sequence

<220>
<223> Synthetic DNA: TRBV10-3*01

<400> 197

```
gatgctggaa tcacccagag cccaagacac aaggtcacag agacaggaac accagtgact        60
ctgagatgtc accagactga gaaccaccgc tatatgtact ggtatcgaca agacccgggg       120
catgggctga ggctgatcca ttactcatat ggtgttaaag atactgacaa aggagaagtc       180
tcagatggct atagtgtctc tagatcaaag acagaggatt tcctcctcac tctggagtcc       240
gctaccagct cccagacatc tgtgtacttc tgtgccatca gtgagtc                     287
```

<210> 198
<211> 287
<212> DNA

<213> Artificial Sequence

<220>
<223> Synthetic DNA: TRBV10-3*02

<400> 198

```
gatgctggaa tcacccagag cccaagacac aaggtcacag agacaggaac accagtgact        60
ctgagatgtc atcagactga gaaccaccgc tatatgtact ggtatcgaca agacccgggg       120
catgggctga ggctgatcca ttactcatat ggtgttaaag atactgacaa aggagaagtc       180
tcagatggct atagtgtctc tagatcaaag acagaggatt tcctcctcac tctggagtcc       240
gctaccagct cccagacatc tgtgtacttc tgtgccatca gtgagtc                     287
```

<210> 199
<211> 290
<212> DNA
<213> Artificial Sequence

<220>
<223> Synthetic DNA: TRBV11-1*01

<400> 199

```
gaagctgaag ttgcccagtc ccccagatat aagattacag agaaaagcca ggctgtggct        60
ttttggtgtg atcctatttc tggccatgct accctttact ggtaccggca gatcctggga       120
cagggcccgg agcttctggt tcaatttcag gatgagagtg tagtagatga ttcacagttg       180
cctaaggatc gattttctgc agagaggctc aaaggagtag actccactct caagatccag       240
cctgcagagc ttggggactc ggccatgtat ctctgtgcca gcagcttagc                  290
```

<210> 200
<211> 290
<212> DNA
<213> Artificial Sequence

<220>
<223> Synthetic DNA: TRBV11-3*01

<400> 200

```
gaagctggag tggttcagtc tcccagatat aagattatag agaaaaaaca gcctgtggct        60
ttttggtgca atcctatttc tggccacaat accctttact ggtacctgca gaacttggga       120
cagggcccgg agcttctgat tcgatatgag aatgaggaag cagtagacga ttcacagttg       180
cctaaggatc gattttctgc agagaggctc aaaggagtag actccactct caagatccag       240
cctgcagagc ttggggactc ggccgtgtat ctctgtgcca gcagcttaga                  290
```

<210> 201

<211> 285
<212> DNA
<213> Artificial Sequence

<220>
<223> Synthetic DNA: TRBV11-3*02

<400> 201

```
gaagctggag tggttcagtc tcccagatat aagattatag agaaaaagca gcctgtggct      60
ttttggtgca atcctatttc tggccacaat accctttact ggtaccggca gaacttggga     120
cagggcccgg agcttctgat tcgatatgag aatgaggaag cagtagacga ttcacagttg     180
cctaaggatc gattttctgc agagaggctc aaaggagtag actccactct caagatccag     240
cctgcagagc ttggggactc ggccgtgtat ctctgtgcca gcagc                     285
```

<210> 202
<211> 269
<212> DNA
<213> Artificial Sequence

<220>
<223> Synthetic DNA: TRBV11-3*03

<400> 202

```
ggtctcccag atataagatt atagagaaga aacagcctgt ggctttttgg tgcaatccaa      60
tttctggcca caataccctt tactggtacc tgcagaactt gggacagggc ccggagcttc     120
tgattcgata tgagaatgag gaagcagtag acgattcaca gttgcctaag gatcgatttt     180
ctgcagagag gctcaaagga gtagactcca ctctcaagat ccagccagca gagcttgggg     240
actcggccat gtatctctgt gccagcagc                                       269
```

<210> 203
<211> 290
<212> DNA
<213> Artificial Sequence

<220>
<223> Synthetic DNA: TRBV11-2*01

<400> 203

```
gaagctggag ttgcccagtc tcccagatat aagattatag agaaaaggca gagtgtggct      60
ttttggtgca atcctatatc tggccatgct accctttact ggtaccagca gatcctggga     120
cagggcccaa agcttctgat tcagtttcag aataacggtg tagtggatga ttcacagttg     180
```

cctaaggatc gattttctgc agagaggctc aaaggagtag actccactct caagatccag 240

cctgcaaagc ttgaggactc ggccgtgtat ctctgtgcca gcagcttaga 290

<210> 204
<211> 285
<212> DNA
<213> Artificial Sequence

<220>
<223> Synthetic DNA: TRBV11-2*03

<400> 204

gaagctggag ttgcccagtc tcccagatat aagattatag agaaaaggca gagtgtggct 60

ttttggtgca atcctatatc tggccatgct accctttact ggtaccagca gatcctggga 120

cagggcccaa agcttctgat tcagtttcag aataacggtg tagtggatga ttcacagttg 180

cctaaggatc gattttctgc agagaggctc aaaggagtag actccactct caagatccaa 240

cctgcaaagc ttgaggactc ggccgtgtat ctctgtgcca gcagc 285

<210> 205
<211> 285
<212> DNA
<213> Artificial Sequence

<220>
<223> Synthetic DNA: TRBV11-2*02

<400> 205

gaagctggag ttgcccagtc tcccagatat aagattatag agaaaaggca gagtgtggct 60

ttttggtgca atcctatatc tggccatgct accctttact ggtaccagca gatcctggga 120

cagggcccaa agcttctgat tcagtttcag aataacggtg tagtggatga ttcacagttg 180

cctaaggatc gattttctgc agagaggctc aaaggagtag actccactct caagatccag 240

cctgcaaagc ttgagaactc ggccgtgtat ctctgtgcca gcagt 285

<210> 206
<211> 290
<212> DNA
<213> Artificial Sequence

<220>
<223> Synthetic DNA: TRBV12-1*01

<400> 206

gatgctggtg ttatccagtc acccaggcac aaagtgacag agatgggaca atcagtaact 60

ctgagatgcg aaccaatttc aggccacaat gatcttctct ggtacagaca gacctttgtg 120

cagggactgg aattgctgaa ttacttctgc agctggaccc tcgtagatga ctcaggagtg 180

tccaaggatt gattctcagc acagatgcct gatgtatcat tctccactct gaggatccag 240

cccatggaac ccagggactt gggcctatat ttctgtgcca gcagctttgc 290

<210> 207
<211> 290
<212> DNA
<213> Artificial Sequence

<220>
<223> Synthetic DNA: TRBV12-2*01

<400> 207

gatgctggca ttatccagtc acccaagcat gaggtgacag aaatgggaca aacagtgact 60

ctgagatgtg agccaatttt tggccacaat ttccttttct ggtacagaga taccttcgtg 120

cagggactgg aattgctgag ttacttccgg agctgatcta ttatagataa tgcaggtatg 180

cccacagagc gattctcagc tgagaggcct gatggatcat tctctactct gaagatccag 240

cctgcagagc aggggggactc ggccgtgtat gtctgtgcaa gtcgcttagc 290

<210> 208
<211> 290
<212> DNA
<213> Artificial Sequence

<220>
<223> Synthetic DNA: TRBV12-4*01

<400> 208

gatgctggag ttatccagtc accccggcac gaggtgacag agatgggaca agaagtgact 60

ctgagatgta aaccaatttc aggacacgac taccttttct ggtacagaca gaccatgatg 120

cggggactgg agttgctcat ttactttaac aacaacgttc cgatagatga ttcagggatg 180

cccgaggatc gattctcagc taagatgcct aatgcatcat tctccactct gaagatccag 240

ccctcagaac ccagggactc agctgtgtac ttctgtgcca gcagtttagc 290

<210> 209
<211> 288
<212> DNA
<213> Artificial Sequence

<220>
<223> Synthetic DNA: TRBV12-4*02

<400> 209

gatgctggag ttatccagtc accccggcac gaggtgacag agatgggaca agaagtgact 60

ctgagatgta aaccaatttc aggacatgac taccttttct ggtacagaca gaccatgatg 120

cggggactgg agttgctcat ttactttaac aacaacgttc cgatagatga ttcagggatg 180

cccgaggatc gattctcagc taagatgcct aatgcatcat tctccactct gaggatccag 240

ccctcagaac ccagggactc agctgtgtac ttctgtgcca gcagttta 288

<210> 210
<211> 290
<212> DNA
<213> Artificial Sequence

<220>
<223> Synthetic DNA: TRBV12-3*01

<400> 210

gatgctggag ttatccagtc accccgccat gaggtgacag agatgggaca agaagtgact 60

ctgagatgta aaccaatttc aggccacaac tcccttttct ggtacagaca gaccatgatg 120

cggggactgg agttgctcat ttactttaac aacaacgttc cgatagatga ttcagggatg 180

cccgaggatc gattctcagc taagatgcct aatgcatcat tctccactct gaagatccag 240

ccctcagaac ccagggactc agctgtgtac ttctgtgcca gcagtttagc 290

<210> 211
<211> 290
<212> DNA
<213> Artificial Sequence

<220>
<223> Synthetic DNA: TRBV12-5*01

<400> 211

gatgctagag tcacccagac accaaggcac aaggtgacag agatgggaca agaagtaaca 60

atgagatgtc agccaatttt aggccacaat actgttttct ggtacagaca gaccatgatg 120

caaggactgg agttgctggc ttacttccgc aaccgggctc ctctagatga ttcggggatg 180

ccgaaggatc gattctcagc agagatgcct gatgcaactt tagccactct gaagatccag 240

ccctcagaac ccagggactc agctgtgtat ttttgtgcta gtggtttggt 290

<210> 212
<211> 287
<212> DNA
<213> Artificial Sequence

<220>

<223> Synthetic DNA: TRBV13*01

<400> 212

```
gctgctggag tcatccagtc cccaagacat ctgatcaaag aaaagaggga aacagccact        60
ctgaaatgct atcctatccc tagacacgac actgtctact ggtaccagca gggtccaggt       120
caggaccccc agttcctcat ttcgttttat gaaaagatgc agagcgataa aggaagcatc       180
cctgatcgat tctcagctca acagttcagt gactatcatt ctgaactgaa catgagctcc       240
ttggagctgg gggactcagc cctgtacttc tgtgccagca gcttagg                     287
```

<210> 213
<211> 282
<212> DNA
<213> Artificial Sequence

<220>
<223> Synthetic DNA: TRBV13*02

<400> 213

```
gctgctggag tcatccagtc cccaagacat ctgatcagag aaaagaggga aacagccact        60
ctgaaatgct atcctatccc tagacacgac actgtctact ggtaccagca gggcccaggt       120
caggaccccc agttcttcat ttcgttttat gaaaagatgc agagcgataa aggaagcatc       180
cctgatcgat tctcagctca acagttcagt gactatcatt ctgaactgaa catgagctcc       240
ttggagctgg gggactcagc cctgtacttc tgtgccagca gc                          282
```

<210> 214
<211> 290
<212> DNA
<213> Artificial Sequence

<220>
<223> Synthetic DNA: TRBV14*01

<400> 214

```
gaagctggag ttactcagtt ccccagccac agcgtaatag agaagggcca gactgtgact        60
ctgagatgtg acccaatttc tggacatgat aatctttatt ggtatcgacg tgttatggga       120
aaagaaataa aatttctgtt acattttgtg aaagagtcta aacaggatga gtccggtatg       180
cccaacaatc gattcttagc tgaaaggact ggagggacgt attctactct gaaggtgcag       240
cctgcagaac tggaggattc tggagtttat ttctgtgcca gcagccaaga                  290
```

<210> 215
<211> 285
<212> DNA
<213> Artificial Sequence

<220>
<223> Synthetic DNA: TRBV14*02

<400> 215

```
gaagctggag ttactcagtt ccccagccac agcgtaatag agaagggcca gactgtgact      60
ctgagatgtg acccaatttc tggacatgat aatctttatt ggtatcgacg tgttatggga     120
aaagaaataa aatttctgtt acattttgtg aaagagtcta aacaggatga atccggtatg     180
cccaacaatc gattcttagc tgaaaggact ggagggacgt attctactct gaaggtgcag     240
cctgcagaac tggaggattc tggagtttat ttctgtgcca gcagc                     285
```

<210> 216
<211> 287
<212> DNA
<213> Artificial Sequence

<220>
<223> Synthetic DNA: TRBV15*01

<400> 216

```
gatgccatgg tcatccagaa cccaagatac caggttaccc agtttggaaa gccagtgacc      60
ctgagttgtt ctcagacttt gaaccataac gtcatgtact ggtaccagca gaagtcaagt     120
caggccccaa agctgctgtt ccactactat gacaaagatt ttaacaatga agcagacacc     180
cctgataact tccaatccag gaggccgaac acttctttct gctttcttga catccgctca     240
ccaggcctgg gggacacagc catgtacctg tgtgccacca gcagaga                   287
```

<210> 217
<211> 282
<212> DNA
<213> Artificial Sequence

<220>
<223> Synthetic DNA: TRBV15*03

<400> 217

```
gatgccatgg tcatccagaa cccaagatac cgggttaccc agtttggaaa gccagtgacc      60
ctgagttgtt ctcagacttt gaaccataac gtcatgtact ggtaccagca gaagtcaagt     120
caggccccaa agctgctgtt ccactactat aacaaagatt ttaacaatga agcagacacc     180
cctgataact tccaatccag gaggccgaac acttctttct gctttctaga catccgctca     240
ccaggcctgg gggacgcagc catgtaccag tgtgccacca gc                        282
```

<210> 218
<211> 282

<212> DNA
<213> Artificial Sequence

<220>
<223> Synthetic DNA: TRBV15*02

<400> 218

gatgccatgg tcatccagaa cccaagatac caggttaccc agtttggaaa gccagtgacc 60

ctgagttgtt ctcagacttt gaaccataac gtcatgtact ggtaccagca gaagtcaagt 120

caggccccaa agctgctgtt ccactactat gacaaagatt ttaacaatga agcagacacc 180

cctgataact tccaatccag gaggccgaac acttctttct gctttcttga catccgctca 240

ccaggcctgg gggacgcagc catgtacctg tgtgccacca gc 282

<210> 219
<211> 290
<212> DNA
<213> Artificial Sequence

<220>
<223> Synthetic DNA: TRBV16*01

<400> 219

ggtgaagaag tcgcccagac tccaaaacat cttgtcagag ggaaggaca gaaagcaaaa 60

ttatattgtg ccccaataaa aggacacagt tatgtttttt ggtaccaaca ggtcctgaaa 120

aacgagttca agttcttgat ttccttccag aatgaaaatg tctttgatga aacaggtatg 180

cccaaggaaa gattttcagc taagtgcctc ccaaattcac cctgtagcct tgagatccag 240

gctacgaagc ttgaggattc agcagtgtat ttttgtgcca gcagccaatc 290

<210> 220
<211> 290
<212> DNA
<213> Artificial Sequence

<220>
<223> Synthetic DNA: TRBV16*02

<400> 220

ggtgaagaag tcgcccagac tccaaaacat cttgtcagag ggaaggaca gaaagcaaaa 60

ttatattgtg ccccaataaa aggacacagt taggtttttt ggtaccaaca ggtcctgaaa 120

aacgagttca agttcttgat ttccttccag aatgaaaatg tctttgatga aacaggtatg 180

cccaaggaaa gattttcagc taagtgcctc ccaaattcac cctgtagcct tgagatccag 240

gctacgaagc ttgaggattc agcagtgtat ttttgtgcca gcagccaatc 290

<210> 221
<211> 285
<212> DNA
<213> Artificial Sequence

<220>
<223> Synthetic DNA: TRBV16*03

<400> 221

ggtgaagaag tcgcccagac tccaaaacat cttgtcagag gggaaggaca gaaagcaaaa 60

ttatattgtg ccccaataaa aggacacagt tatgtttttt ggtaccaaca ggtcctgaaa 120

aacgagttca agttcttggt ttccttccag aatgaaaatg tctttgatga aacaggtatg 180

cccaaggaaa gattttcagc taagtgcctc ccaaattcac cctgtagcct tgagatccag 240

gctacgaagc ttgaggattc agcagtgtat ttttgtgcca gcagc 285

<210> 222
<211> 287
<212> DNA
<213> Artificial Sequence

<220>
<223> Synthetic DNA: TRBV17*01

<400> 222

gagcctggag tcagccagac ccccagacac aaggtcacca acatgggaca ggaggtgatt 60

ctgaggtgcg atccatcttc tggtcacatg tttgttcact ggtaccgaca gaatctgagg 120

caagaaatga agttgctgat ttccttccag taccaaaaca ttgcagttga ttcagggatg 180

cccaaggaac gattcacagc tgaaagacct aacggaacgt cttccacgct gaagatccat 240

cccgcagagc cgagggactc agccgtgtat ctctacagta gcggtgg 287

<210> 223
<211> 290
<212> DNA
<213> Artificial Sequence

<220>
<223> Synthetic DNA: TRBV18*01

<400> 223

aatgccggcg tcatgcagaa cccaagacac ctggtcagga ggaggggaca ggaggcaaga 60

ctgagatgca gcccaatgaa aggacacagt catgtttact ggtatcggca gctcccagag 120

gaaggtctga aattcatggt ttatctccag aaagaaaata tcatagatga gtcaggaatg 180

ccaaaggaac gattttctgc tgaatttccc aaagagggcc ccagcatcct gaggatccag 240

caggtagtgc gaggagattc ggcagcttat ttctgtgcca gctcaccacc 290

<210> 224
<211> 287
<212> DNA
<213> Artificial Sequence

<220>
<223> Synthetic DNA: TRBV19*01

<400> 224

gatggtggaa tcactcagtc cccaaagtac ctgttcagaa aggaaggaca gaatgtgacc 60

ctgagttgtg aacagaattt gaaccacgat gccatgtact ggtaccgaca ggacccaggg 120

caagggctga gattgatcta ctactcacag atagtaaatg actttcagaa aggagatata 180

gctgaagggt acagcgtctc tcgggagaag aaggaatcct ttcctctcac tgtgacatcg 240

gcccaaaaga acccgacagc tttctatctc tgtgccagta gtataga 287

<210> 225
<211> 287
<212> DNA
<213> Artificial Sequence

<220>
<223> Synthetic DNA: TRBV19*02

<400> 225

gatggtggaa tcactcagtc cccaaagtac ctgttcagaa aggaaggaca gaatgtgacc 60

ctgagttgtg aacagaattt gaaccacgat gccatgtact ggtaccgaca ggtcccaggg 120

caagggctga gattgatcta ctactcacac atagtaaatg actttcagaa aggagatata 180

gctgaagggt acagcgtctc tcgggagaag aaggaatcct ttcctctcac tgtgacatcg 240

gcccaaaaga acccgacagc tttctatctc tgtgccagta gtataga 287

<210> 226
<211> 282
<212> DNA
<213> Artificial Sequence

<220>
<223> Synthetic DNA: TRBV19*03

<400> 226

```
gatggtggaa tcactcagtc cccaaagtac ctgttcagaa aggaaggaca gaatgtgacc        60
ctgagttgtg aacagaattt gaaccacgat gccatgtact ggtaccgaca ggacccaggg       120
caagggctga gattgatcta ctactcacac atagtaaatg actttcagaa aggagatata       180
gctgaagggt acagcgtctc tcgggagaag aaggaatcct ttcctctcac tgtgacatcg       240
gcccaaaaga acccgacagc tttctatctc tgtgccagta gc                          282
```

<210> 227
<211> 291
<212> DNA
<213> Artificial Sequence

<220>
<223> Synthetic DNA: TRBV20-1*05

<400> 227

```
ggtgctgtcg tctctcaaca tccgagcagg gttatctgta agagtggaac ctctgtgaag        60
atcgagtgcc gttccctgga ctttcaggcc acaactatgt tttggtatcg tcagttcccg       120
aaaaagagtc tcatgctgat ggcaacttcc aatgagggct ccaaggccac atacgagcaa       180
ggcgtcgaga aggacaagtt tctcatcaac catgcaagcc tgaccttgtc cactctgaca       240
gtgaccagtg cccatcctga agacagcagc ttctacatct gcagtgctag a                291
```

<210> 228
<211> 291
<212> DNA
<213> Artificial Sequence

<220>
<223> Synthetic DNA: TRBV20-1*07

<400> 228

```
ggtgctgtcg tctctcaaca tccgagcagg gttatctgta agagtggaac ctctgtgaag        60
atcgagtgcc gttccctgga ctttcaggcc acaactatgt tttggtatcg tcagttcccg       120
aaaaagagtc tcatgcagat cgcaacttcc aatgagggct ccaaggccac atacgagcaa       180
ggcgtcgaga aggacaagtt tctcatcaac catgcaagcc tgaccttgtc cactctgaca       240
gtgaccagtg cccatcctga agacagcagc ttctacatct gcagtgctag a                291
```

<210> 229
<211> 291
<212> DNA
<213> Artificial Sequence

<220>

<223> Synthetic DNA: TRBV20-1*04

<400> 229

```
ggtgctgtcg tctctcaaca tccgagcagg gttatctgta agagtggaac ctctgtgaag       60
atcgagtgcc gttccttgga ctttcaggcc acaactatgt tttggtatcg tcagttcccg      120
aaaaagagtc tcatgctgat ggcaacttcc aatgagggct ccaaggccac atacgagcaa      180
ggcgtcgaga aggacaagtt tctcatcaac catgcaagcc tgaccttgtc cactctgaca      240
gtgaccagtg cccatcctga agacagcagc ttctacatct gcagtgctag t               291
```

<210> 230
<211> 288
<212> DNA
<213> Artificial Sequence

<220>
<223> Synthetic DNA: TRBV20-1*06

<400> 230

```
ggtgctgtcg tctctcaaca tccgagtagg gttatctgta agagtggaac ctctgtgaag       60
atcgagtgcc gttccctgga ctttcaggcc acaactatgt tttggtatcg tcagttcccg      120
aaaaagagtc tcatgctgat ggcaacttcc aatgagggct ccaaggccac atacgagcaa      180
ggcgtcgaga aggacaagtt tctcatcaac catgcaagcc tgaccttgtc cactctgaca      240
gtgaccagtg cccatcctga agacagcagc ttctacatct gcagtgct                   288
```

<210> 231
<211> 288
<212> DNA
<213> Artificial Sequence

<220>
<223> Synthetic DNA: TRBV20-1*02

<400> 231

```
ggtgctgtcg tctctcaaca tccgagcagg gttatctgta agagtggaac ctctgtgaag       60
atcgagtgcc gttccctgga ctttcaggcc acaactatgt tttggtatcg tcagttcccg      120
aaacagagtc tcatgctgat ggcaacttcc aatgagggct ccaaggccac atacgagcaa      180
ggcgtcgaga aggacaagtt tctcatcaac catgcaagcc tgaccttgtc cactctgaca      240
gtgaccagtg cccatcctga agacagcagc ttctacatct gcagtgct                   288
```

<210> 232
<211> 293
<212> DNA

<213> Artificial Sequence

<220>
<223> Synthetic DNA: TRBV20-1*01

<400> 232

```
ggtgctgtcg tctctcaaca tccgagctgg gttatctgta agagtggaac ctctgtgaag      60
atcgagtgcc gttccctgga ctttcaggcc acaactatgt tttggtatcg tcagttcccg     120
aaacagagtc tcatgctgat ggcaacttcc aatgagggct ccaaggccac atacgagcaa     180
ggcgtcgaga aggacaagtt tctcatcaac catgcaagcc tgaccttgtc cactctgaca     240
gtgaccagtg cccatcctga agacagcagc ttctacatct gcagtgctag aga            293
```

<210> 233
<211> 288
<212> DNA
<213> Artificial Sequence

<220>
<223> Synthetic DNA: TRBV20-1*03

<400> 233

```
ggtgctgtcg tctctcaaca tccgagctgg gttatctgta agagtggaac ctctgtgaag      60
atcgagtgcc gttccctgga ctttcaggcc acaactatgt tttggtatcg tcagttcccg     120
aaacagagtc tcatgctgat ggcaacttcc aatgagggct gcaaggccac atacgagcaa     180
ggcgtcgaga aggacaagtt tctcatcaac catgcaagcc tgaccttgtc cactctgaca     240
gtgaccagtg cccatcctga agacagcagc ttctacatct gcagtgct                  288
```

<210> 234
<211> 290
<212> DNA
<213> Artificial Sequence

<220>
<223> Synthetic DNA: TRBV21-1*01

<400> 234

```
gacaccaagg tcacccagag acctagactt ctggtcaaag caagtgaaca gaaagcaaag      60
atggattgtg ttcctataaa agcacatagt tatgtttact ggtatcgtaa gaagctggaa     120
gaagagctca agtttttggt ttactttcag aatgaagaac ttattcagaa agcagaaata     180
atcaatgagc gatttttagc ccaatgctcc aaaaactcat cctgtacctt ggagatccag     240
tccacggagt caggggacac agcactgtat ttctgtgcca gcagcaaagc                290
```

<210> 235

<211> 288
<212> DNA
<213> Artificial Sequence

<220>
<223> Synthetic DNA: TRBV22-1*01

<400> 235

```
gatgctgaca tctatcagat gccattccag ctcactgggg ctggatggga tgtgactctg      60
gagtggaaac ggaatttgag acacaatgac atgtactgct actggtactg gcaggaccca     120
aagcaaaatc tgagactgat ctattactca agggttgaaa aggatattca gagaggagat     180
ctaactgaag gctacgtgtc tgccaagagg agaaggggct atttcttctc agggtgaagt     240
tggcccacac cagccaaaca gctttgtact tctgtcctgg gagcgcac                  288
```

<210> 236
<211> 290
<212> DNA
<213> Artificial Sequence

<220>
<223> Synthetic DNA: TRBV23-1*01

<400> 236

```
catgccaaag tcacacagac tccaggacat ttggtcaaag gaaaaggaca gaaaacaaag      60
atggattgta cccccgaaaa aggacatact tttgtttatt ggtatcaaca gaatcagaat     120
aaagagttta tgcttttgat ttcctttcag aatgaacaag ttcttcaaga aacggagatg     180
cacaagaagc gattctcatc tcaatgcccc aagaacgcac cctgcagcct ggcaatcctg     240
tcctcagaac cgggagacac ggcactgtat ctctgcgcca gcagtcaatc                290
```

<210> 237
<211> 288
<212> DNA
<213> Artificial Sequence

<220>
<223> Synthetic DNA: TRBV24-1*01

<400> 237

```
gatgctgatg ttacccagac cccaaggaat aggatcacaa agacaggaaa gaggattatg      60
ctggaatgtt ctcagactaa gggtcatgat agaatgtact ggtatcgaca agacccagga     120
ctgggcctac ggttgatcta ttactccttt gatgtcaaag atataaacaa aggagagatc     180
tctgatggat acagtgtctc tcgacaggca caggctaaat tctccctgtc cctagagtct     240
gccatcccca accagacagc tctttacttc tgtgccacca gtgatttg                  288
```

<210> 238
<211> 287
<212> DNA
<213> Artificial Sequence

<220>
<223> Synthetic DNA: TRBV25-1*01

<400> 238

gaagctgaca tctaccagac cccaagatac cttgttatag ggacaggaaa gaagatcact 60

ctggaatgtt ctcaaaccat gggccatgac aaaatgtact ggtatcaaca agatccagga 120

atggaactac acctcatcca ctattcctat ggagttaatt ccacagagaa gggagatctt 180

tcctctgagt caacagtctc cagaataagg acggagcatt ttcccctgac cctggagtct 240

gccaggccct cacatacctc tcagtacctc tgtgccagca gtgaata 287

<210> 239
<211> 287
<212> DNA
<213> Artificial Sequence

<220>
<223> Synthetic DNA: TRBV26*01

<400> 239

gatgctgtag ttacacaatt cccaagacac agaatcattg ggacaggaaa ggaattcatt 60

ctacagtgtt cccagaatat gaatcatgtt acaatgtact ggtatcgaca ggacccagga 120

cttggactga agctggtcta ttattcacct ggcactggga gcactgaaaa aggagatatc 180

tctgaggggt atcatgtttc ttgaaatact atagcatctt ttcccctgac cctgaagtct 240

gccagcacca accagacatc tgtgtatctc tatgccagca gttcatc 287

<210> 240
<211> 287
<212> DNA
<213> Artificial Sequence

<220>
<223> Synthetic DNA: TRBV27*01

<400> 240

gaagcccaag tgacccagaa cccaagatac ctcatcacag tgactggaaa gaagttaaca 60

gtgacttgtt ctcagaatat gaaccatgag tatatgtcct ggtatcgaca agacccaggg 120

ctgggcttaa ggcagatcta ctattcaatg aatgttgagg tgactgataa gggagatgtt 180

cctgaagggt acaaagtctc tcgaaaagag aagaggaatt tcccctgat cctggagtcg 240

cccagcccca accagacctc tctgtacttc tgtgccagca gtttatc 287

<210> 241
<211> 287
<212> DNA
<213> Artificial Sequence

<220>
<223> Synthetic DNA: TRBV28*01

<400> 241

gatgtgaaag taacccagag ctcgagatat ctagtcaaaa ggacgggaga gaaagttttt 60

ctggaatgtg tccaggatat ggaccatgaa aatatgttct ggtatcgaca agacccaggt 120

ctggggctac ggctgatcta tttctcatat gatgttaaaa tgaaagaaaa aggagatatt 180

cctgaggggt acagtgtctc tagagagaag aaggagcgct tctccctgat tctggagtcc 240

gccagcacca accagacatc tatgtacctc tgtgccagca gtttatg 287

<210> 242
<211> 290
<212> DNA
<213> Artificial Sequence

<220>
<223> Synthetic DNA: TRBV29-1*01

<400> 242

agtgctgtca tctctcaaaa gccaagcagg gatatctgtc aacgtggaac ctccctgacg 60

atccagtgtc aagtcgatag ccaagtcacc atgatgttct ggtaccgtca gcaacctgga 120

cagagcctga cactgatcgc aactgcaaat cagggctctg aggccacata tgagagtgga 180

tttgtcattg acaagtttcc catcagccgc ccaaacctaa cattctcaac tctgactgtg 240

agcaacatga gccctgaaga cagcagcata tatctctgca gcgttgaaga 290

<210> 243
<211> 288
<212> DNA
<213> Artificial Sequence

<220>
<223> Synthetic DNA: TRBV29-1*02

<400> 243

agtgctgtca tctctcaaaa gccaagcagg gatatctgtc aacgtggaac ctccctgacg 60

atccagtgtc aagtcgatag ccaagtcacc atgatgttct ggtaccgtca gcaacctgga 120

cagagcctga cactgatcgc aactgcaaat cagggctctg aggccacata tgagagtgga 180

tttgtcattg acaagtttcc catcagccgc ccaaacctaa cattctcaag tctgactgtg 240

agcaacatga gccctgaaga cagcagcata tatctctgca gcgttgaa 288

<210> 244
<211> 231
<212> DNA
<213> Artificial Sequence

<220>
<223> Synthetic DNA: TRBV29-1*03

<400> 244

acgatccagt gtcaagtcga tagccaagtc accatgatat tctggtaccg tcagcaacct 60

ggacagagcc tgacactgat cgcaactgca aatcagggct ctgaggccac atatgagagt 120

ggatttgtca ttgacaagtt tcccatcagc cgcccaaacc taacattctc aactctgact 180

gtgagcaaca tgagccctga agacagcagc atatatctct gcagcgcggg c 231

<210> 245
<211> 284
<212> DNA
<213> Artificial Sequence

<220>
<223> Synthetic DNA: TRBV30*02

<400> 245

tctcagacta ttcatcaatg gccagcgacc ctggtgcagc ctgtgggcag cccgctctct 60

ctggagtgca ctgtggaggg aacatcaaac cccaacctat actggtaccg acaggctgca 120

ggcaggggcc tccagctgct cttctactcc gttggtattg gccagatcag ctctgaggtg 180

ccccagaatc tctcagcctc cagaccccag gaccggcagt tcatcctgag ttctaagaag 240

ctcctcctca gtgactctgg cttctatctc tgtgcctgga gtgt 284

<210> 246
<211> 282
<212> DNA
<213> Artificial Sequence

<220>
<223> Synthetic DNA: TRBV30*05

<400> 246

```
tctcagacta ttcatcaatg gccagcgacc ctggtgcagc ctgtgggcag cccgctctcc      60
ctggagtgca ctgtggaggg aacatcaaac cccaacctat actggtaccg acaggctgca     120
ggacggggcc tccagctgct cttctactcc gttggtattg gccagatcag ctctgaggtg     180
ccccagaatc tctcagcctc cagaccccag gaccggcagt tcatcctgag ttctaagaag     240
ctccttctca gtgactctgg cttctatctc tgtgcctggg ga                        282
```

<210> 247
<211> 284
<212> DNA
<213> Artificial Sequence

<220>
<223> Synthetic DNA: TRBV30*01

<400> 247

```
tctcagacta ttcatcaatg gccagcgacc ctggtgcagc ctgtgggcag cccgctctct      60
ctggagtgca ctgtggaggg aacatcaaac cccaacctat actggtaccg acaggctgca     120
ggcaggggcc tccagctgct cttctactcc gttggtattg gccagatcag ctctgaggtg     180
ccccagaatc tctcagcctc cagaccccag gaccggcagt tcatcctgag ttctaagaag     240
ctccttctca gtgactctgg cttctatctc tgtgcctgga gtgt                      284
```

<210> 248
<211> 276
<212> DNA
<213> Artificial Sequence

<220>
<223> Synthetic DNA: TRBV30*04

<400> 248

```
actattcatc aatggccagc gaccctggtg cagcctgtgg gcagcccgct ctctctggag      60
tgcactgtgg agggaacatc aaaccccaac ctatactggt accgacaggc tgcaggcagg     120
ggcctccagc tgctcttcta ctccattggt attgaccaga tcagctctga ggtgccccag     180
aatctctcag cctccagacc ccaggaccgg cagttcattc tgagttctaa gaagctcctc     240
ctcagtgact ctggcttcta tctctgtgcc tggagt                               276
```

<210> 249
<211> 448
<212> DNA
<213> Artificial Sequence

<220>

<223> Synthetic DNA: TCRBJ1S1

<400> 249

```
ttgaaaaagg aacctaggac cctgtggatg gactctgtca ttctccatgg tcctaaaaag        60
caaaagtcaa agtgttcttc tgtgtaatac ccataaagca caggaggaga tttcttagct       120
cactgtcctc catcctagcc agggccctct cccctctcta tgccttcaat gtgattttca       180
ccttgacccc tgtcactgtg tgaacactga agctttcttt ggacaaggca ccagactcac       240
agttgtaggt aagacatttt tcaggttctt ttgcagatcc gtcacaggga aaagtgggtc       300
cacagtgtcc cttttagagt ggctatattc ttatgtgcta actatggcta caccttcggt       360
tcggggacca ggttaaccgt tgtaggtaag gctgggggtc tctaggaggg gtgcgatgag       420
ggaggactct gtcctgggaa atgtcaaa                                          448
```

<210> 250
<211> 448
<212> DNA
<213> Artificial Sequence

<220>
<223> Synthetic DNA: TCRBJ1S2

<400> 250

```
gccagggccc tctcccctct ctatgccttc aatgtgattt tcaccttgac ccctgtcact        60
gtgtgaacac tgaagctttc tttggacaag gcaccagact cacagttgta ggtaagacat       120
ttttcaggtt cttttgcaga tccgtcacag ggaaaagtgg gtccacagtg tcccttttag       180
agtggctata ttcttatgtg ctaactatgg ctacaccttc ggttcgggga ccaggttaac       240
cgttgtaggt aaggctgggg gtctctagga ggggtgcgat gagggaggac tctgtcctgg       300
gaaatgtcaa agagaacaga gatcccagct cccggagcca gactgaggga gacgtcatgt       360
catgtcccgg gattgagttc aggggaggct ccctgtgagg gcgaatccac ccaggcttcc       420
cagaggctct gagcagtcac agctgagc                                          448
```

<210> 251
<211> 450
<212> DNA
<213> Artificial Sequence

<220>
<223> Synthetic DNA: TCRBJ1S3

<400> 251

gattttatag gaggccactc tgtgtctctt tttgtcacct gcctgagtct tgggcaagct 60

ctggaaggga acacagagta ctggaagcag agctgctgtc cctgtgaggg aagagttccc 120

atgaactccc aacctctgcc tgaatcccag ctgtgctcag cagagactgg ggggttttga 180

agtggccctg ggaggctgtg ctctggaaac accatatatt ttggagaggg aagttggctc 240

actgttgtag gtgagtaagt caaggctgga cagctgggaa cttgcaaaaa ggggctggaa 300

tccagacgga gcctttgtct ctagtgctta ggtgaaagtg tatttttgtc aggaaggcct 360

atgaggcaga tgaggagggg atagcctccc tctcctctcg actattttgt agactgcctg 420

tgccaagtta ggttccccta ctgagagatg 450

<210> 252
<211> 451
<212> DNA
<213> Artificial Sequence

<220>
<223> Synthetic DNA: TCRBJ1S4

<400> 252

cagaagaggg aacttggggg atcacacggg gcctaattgg tctgctgacc accgcatttt 60

gggttgtacc attgtctacc cctctaccca ccagggttaa aattctacta aggaacagga 120

gaggacctgg caggtggact tggggaggca ggagtggaag gcagcaggtc gcggttttcc 180

ttccagtctt taatgttgtg caactaatga aaaactgttt tttggcagtg gaacccagct 240

ctctgtcttg ggtatgtaaa agacttcttt cgggatagtg tatcataagg tcggagttcc 300

aggaggaccc cttgcgggag ggcagaaact gagaacacag ccaagaaaag ctcataaaat 360

gtgggtcagt ggagtgtgtg gtggggcccc aagagttctg tgtgtaagca gcttctggaa 420

ggaagggccc acaccagctc ctctggggtt t 451

<210> 253
<211> 450
<212> DNA
<213> Artificial Sequence

<220>
<223> Synthetic DNA: TCRBJ1S5

<400> 253

gatagtgtat cataaggtcg gagttccagg aggacccctt gcgggagggc agaaactgag 60

aacacagcca agaaaagctc ataaaatgtg ggtcagtgga gtgtgtggtg gggccccaag 120

agttctgtgt gtaagcagct tctggaagga agggcccaca ccagctcctc tggggtttgc 180

cacactcatg atgcactgtg tagcaatcag ccccagcatt ttggtgatgg gactcgactc 240

tccatcctag gtaagttgca gaatcagggt ggtatggcca ttgtcccttg aaggcagagt 300

tctctgcttc tcctcccggt gctggtgagg cagattgagt aaaatctctt accccatggg 360

gtaagagctg tgcctgtgcc tgcgttccct ttggtgtgtc ttggttgact cctctatttc 420

tcttctctaa gtcttcagtc cataatctgc 450

<210> 254
<211> 453
<212> DNA
<213> Artificial Sequence

<220>
<223> Synthetic DNA: TCRBJ1S6

<400> 254

atggctctgc ctctcctaag cctcttcctc ttgcgcctta tgctgcacag tatgcttagg 60

cctttttcct aacagaatcc ctttggtcca gagccatgaa tccaggcaga gaaaggcagc 120

catcctgctg tcagggagct aagacttgcc ctctgactgg agatcgccgg gtgggtttta 180

tctaagcctc tgcagctgtg ctcctataat tcacccctcc actttgggaa cgggaccagg 240

ctcactgtga caggtatggg ggctccactc ttgactcggg ggtgcctggg tttgactgca 300

atgatcagtt gctgggaagg gaattgagtg taagaacgga ggtcagggtc accccttctt 360

acctggagca ctgtgccctc tcctcccctc cctggagctc ttccagcttg ttgctctgct 420

gtgttgcctg cagttcctca gctgtagagc tcc 453

<210> 255
<211> 449
<212> DNA
<213> Artificial Sequence

<220>
<223> Synthetic DNA: TCRBJ2S1

<400> 255

aatccactgt gttgtccccc agccaagtgg attctcctct gcaaattggt ggtggcctca 60

tgcaagatcc agttaccgtg tccagctaac tcgagacagg aaaagatagg ctcaggaaag 120

agaggaaggg tgtgccctct gtctgtgcta agggaggtgg ggaaggagaa ggaattctgg 180

gcagcccctt cccactgtgc tcctacaatg agcagttctt cgggccaggg acacggctca 240

ccgtgctagg taagaagggg gctccaggtg ggagagaggg tgagcagccc agcctgcacg 300

accccagaac cctgttctta ggggagtgga cactgggcaa tccagggccc tcctcgaggg 360

aagcggggtt tgcgccaggg tccccagggc tgtgcgaaca ccggggagct gtttttttgga 420

gaaggctcta ggctgaccgt actgggtaa 449

<210> 256
<211> 451
<212> DNA
<213> Artificial Sequence

<220>
<223> Synthetic DNA: TCRBJ2S2

<400> 256

ctgtgctcct acaatgagca gttcttcggg ccagggacac ggctcaccgt gctaggtaag 60

aagggggctc caggtgggag agagggtgag cagcccagcc tgcacgaccc cagaaccctg 120

ttcttagggg agtggacact gggcaatcca gggccctcct cgagggaagc ggggtttgcg 180

ccagggtccc cagggctgtg cgaacaccgg ggagctgttt tttggagaag gctctaggct 240

gaccgtactg ggtaaggagg cggttggggc tccggagagc tccgagaggg cgggatgggc 300

agaggtaagc agctgcccca ctctgagagg ggctgtgctg agaggcgctg ctgggcgtct 360

gggcggagga ctcctggttc tgggtgctgg gagagcgatg gggctctcag cggtgggaag 420

gacccgagct gagtctggga cagcagagcg g 451

<210> 257
<211> 449
<212> DNA
<213> Artificial Sequence

<220>
<223> Synthetic DNA: TCRBJ2S3

<400> 257

gggcgggatg ggcagaggta agcagctgcc ccactctgag aggggctgtg ctgagaggcg 60
ctgctgggcg tctgggcgga ggactcctgg ttctgggtgc tgggagagcg atggggctct 120
cagcggtggg aaggacccga gctgagtctg ggacagcaga gcgggcagca ccggtttttg 180
tcctgggcct ccaggctgtg agcacagata cgcagtattt tggcccaggc acccggctga 240
cagtgctcgg taagcggggg ctcccgctga agccccggaa ctggggaggg ggcgccccgg 300
gacgccgggg gcgtcgcagg gccagtttct gtgccgcgtc tcggggctgt gagccaaaaa 360
cattcagtac ttcggcgccg ggacccggct ctcagtgctg ggtaagctgg ggccgccggg 420
ggaccgggga cgagactgcg ctcgggttt 449

<210> 258
<211> 450
<212> DNA
<213> Artificial Sequence

<220>
<223> Synthetic DNA: TCRBJ2S4

<400> 258

gacagcagag cgggcagcac cggtttttgt cctgggcctc caggctgtga gcacagatac 60
gcagtatttt ggcccaggca cccggctgac agtgctcggt aagcgggggc tcccgctgaa 120
gccccggaac tggggagggg gcgccccggg acgccggggg cgtcgcaggg ccagtttctg 180
tgccgcgtct cggggctgtg agccaaaaac attcagtact tcggcgccgg gacccggctc 240
tcagtgctgg gtaagctggg gccgccgggg gaccggggac gagactgcgc tcgggttttt 300
gtgcggggct cgggggccgt gaccaagaga cccagtactt cgggccaggc acgcggctcc 360
tggtgctcgg tgagcgcggg ctgctggggc gcgggcgcgg gcggcttggg tctggttttt 420
gcggggagtc cccgggctgt gctctggggc 450

<210> 259
<211> 448
<212> DNA
<213> Artificial Sequence

<220>
<223> Synthetic DNA: TCRBJ2S5

<400> 259

```
ccccggaact ggggaggggg cgccccggga cgccgggggc gtcgcagggc cagtttctgt        60
gccgcgtctc ggggctgtga gccaaaaaca ttcagtactt cggcgccggg acccggctct       120
cagtgctggg taagctgggg ccgccggggg accggggacg agactgcgct cgggtttttg       180
tgcggggctc gggggccgtg accaagagac ccagtacttc gggccaggca cgcggctcct       240
ggtgctcggt gagcgcgggc tgctggggcg cgggcgcggg cggcttgggt ctggtttttg       300
cggggagtcc ccgggctgtg ctctggggcc aacgtcctga ctttcggggc cggcagcagg       360
ctgaccgtgc tgggtgagtt ttcgcgggac cacccgggcg gcgggattca ggtggaaggc       420
ggcggctgct tcgcggcacc cggtccgg                                          448
```

<210> 260
<211> 453
<212> DNA
<213> Artificial Sequence

<220>
<223> Synthetic DNA: TCRBJ2S6

<400> 260

```
cagtgctggg taagctgggg ccgccggggg accggggacg agactgcgct cgggtttttg        60
tgcggggctc gggggccgtg accaagagac ccagtacttc gggccaggca cgcggctcct       120
ggtgctcggt gagcgcgggc tgctggggcg cgggcgcggg cggcttgggt ctggtttttg       180
cggggagtcc ccgggctgtg ctctggggcc aacgtcctga ctttcggggc cggcagcagg       240
ctgaccgtgc tgggtgagtt ttcgcgggac cacccgggcg gcgggattca ggtggaaggc       300
ggcggctgct tcgcggcacc cggtccggcc ctgtgctggg agacctgggc tgggtcccca       360
gggtgggcag gagctcgggg agccttagag gtttgcatgc gggggtgcac ctccgtgctc       420
ctacgagcag tacttcgggc cgggcaccag gct                                    453
```

<210> 261
<211> 447
<212> DNA
<213> Artificial Sequence

<220>
<223> Synthetic DNA: TCRBJ2S7

<400> 261

tgactttcgg ggccggcagc aggctgaccg tgctgggtga gttttcgcgg gaccacccgg 60

gcggcgggat tcaggtggaa ggcggcggct gcttcgcggc acccggtccg gccctgtgct 120

gggagacctg ggctgggtcc ccagggtggg caggagctcg gggagcctta gaggtttgca 180

tgcgggggtg cacctccgtg ctcctacgag cagtacttcg ggccgggcac caggctcacg 240

gtcacaggtg agattcgggc gtctccccac cttccagccc ctcggtcccc ggagtcggag 300

ggtggaccgg agctggagga gctgggtgtc cggggtcagc tctgcaaggt cacctccccg 360

ctcctgggga aagactgggg aagagggagg gggtggggag gtgctcagag tccggaaagc 420

tgagcagagg gcgaggccac ttttaat 447

<210> 262
<211> 296
<212> DNA
<213> Artificial Sequence

<220>
<223> Synthetic DNA: IGHV1-46*02

<400> 262

caggtgcagc tggtgcagtc tggggctgag gtgaagaagc ctggggcctc agtgaaggtt 60

tcctgcaagg catctggata caccttcaac agctactata tgcactgggt gcgacaggcc 120

cctggacaag ggcttgagtg gatgggaata atcaacccta gtggtggtag cacaagctac 180

gcacagaagt tccagggcag agtcaccatg accagggaca cgtccacgag cacagtctac 240

atggagctga gcagcctgag atctgaggac acggccgtgt attactgtgc gagaga 296

<210> 263
<211> 260
<212> DNA
<213> Artificial Sequence

<220>
<223> Synthetic DNA: IGHV1/OR15-5*01

<400> 263

agaagcctgg ggcctcagtg aaggtctcct gcaaggcttc tggatacacc ttcaccagct 60

actgtatgca ctgggtgcac caggtccatg cacaagggct tgagtggatg ggattggtgt 120

gccctagtga tggcagcaca agctatgcac agaagttcca ggccagagtc accataacca 180

gggacacatc catgagcaca gcctacatgg agctaagcag tctgagatct gaggacacgg 240

ccatgtatta ctgtgtgaga 260

<210> 264
<211> 294

<212> DNA
<213> Artificial Sequence

<220>
<223> Synthetic DNA: IGHV1/OR15-5*03

<400> 264

```
caggtacagc tggtgcagtc tggggctgag gtgaagaagc ctggggcctc agtgaaggtc      60
tcctgcaagg cttctggata caccttcacc aactactgta tgcactgggt gcgccaggtc     120
catgcacaag ggcttgagtg gatgggattg gtgtgcccta gtgatggcag cacaagctat     180
gcacaaaagt tccaggccag agtcaccata accagggaca catccatgag cacagcctac     240
atggagctaa gcagtctgag atctgaggac acggccatgt attactgtgt gaga           294
```

<210> 265
<211> 296
<212> DNA
<213> Artificial Sequence

<220>
<223> Synthetic DNA: IGHV1/OR15-9*01

<400> 265

```
caggtacagc tgatgcagtc tggggctgag gtgaagaagc ctggggcctc agtgaggatc      60
tcctgcaagg cttctggata caccttcacc agctactgta tgcactgggt gtgccaggcc     120
catgcacaag ggcttgagtg gatgggattg gtgtgcccta gtgatggcag cacaagctat     180
gcacagaagt tccagggcag agtcaccata accagggaca catccatggg cacagcctac     240
atggagctaa gcagcctgag atctgaggac acggccatgt attactgtgt gagaga         296
```

<210> 266
<211> 260
<212> DNA
<213> Artificial Sequence

<220>
<223> Synthetic DNA: IGHV1-c*01

<400> 266

```
ggaagtctgg ggcctcagtg aaagtctcct gtagtttttc tgggtttacc atcaccagct      60
acggtataca ttgggtgcaa cagtcccctg gacaagggct tgagtggatg ggatggatca     120
accctggcaa tggtagccca agctatgcca agaagtttca gggcagattc accatgacca     180
gggacatgtc cacaaccaca gcctacacag acctgagcag cctgacatct gaggacatgg     240
ctgtgtatta ctatgcaaga                                                 260
```

<210> 267
<211> 296
<212> DNA
<213> Artificial Sequence

<220>
<223> Synthetic DNA: IGHV1-NL1*01

<220>
<221> misc_feature
<222> (136)..(136)
<223> n is a, c, g, or t

<220>
<221> misc_feature
<222> (253)..(253)
<223> n is a, c, g, or t

<400> 267

```
caggttcagc tgttgcagcc tggggtccag gtgaagaagc ctgggtcctc agtgaaggtc      60
tcctgctagg cttccagata caccttcacc aaatacttta cacggtgggt gtgacaaagc     120
cctggacaag ggcatnagtg gatgggatga atcaaccctt acaacgataa cacacactac     180
gcacagacgt tctggggcag agtcaccatt accagtgaca ggtccatgag cacagcctac     240
atggagctga gcngcctgag atccgaagac atggtcgtgt attactgtgt gagaga         296
```

<210> 268
<211> 296
<212> DNA
<213> Artificial Sequence

<220>
<223> Synthetic DNA: IGHV1-58*01

<400> 268

```
caaatgcagc tggtgcagtc tgggcctgag gtgaagaagc ctgggacctc agtgaaggtc      60
tcctgcaagg cttctggatt cacctttact agctctgctg tgcagtgggt gcgacaggct     120
cgtggacaac gccttgagtg gataggatgg atcgtcgttg gcagtggtaa cacaaactac     180
gcacagaagt tccaggaaag agtcaccatt accagggaca tgtccacaag cacagcctac     240
atggagctga gcagcctgag atccgaggac acggccgtgt attactgtgc ggcaga         296
```

<210> 269
<211> 296
<212> DNA
<213> Artificial Sequence

<220>
<223> Synthetic DNA: IGHV1-58*02

<400> 269

```
caaatgcagc tggtgcagtc tgggcctgag gtgaagaagc ctgggacctc agtgaaggtc      60
tcctgcaagg cttctggatt cacctttact agctctgcta tgcagtgggt gcgacaggct     120
cgtggacaac gccttgagtg gataggatgg atcgtcgttg gcagtggtaa cacaaactac     180
gcacagaagt tccaggaaag agtcaccatt accagggaca tgtccacaag cacagcctac     240
atggagctga gcagcctgag atccgaggac acggccgtgt attactgtgc ggcaga         296
```

<210> 270
<211> 275
<212> DNA
<213> Artificial Sequence

<220>
<223> Synthetic DNA: IGHV1-69*03

<400> 270

```
caggtgcagc tggtgcagtc tggggctgag gtgaagaagc ctgggtcctc ggtgaaggtc      60
tcctgcaagg cttctggagg caccttcagc agctatgcta tcagctgggt gcgacaggcc     120
cctggacaag ggcttgagtg gatgggaggg atcatcccta tctttggtac agcaaactac     180
gcacagaagt tccagggcag agtcacgatt accgcggacg aatccacgag cacagcctac     240
atggagctga gcagcctgag atctgatgac acggc                                275
```

<210> 271
<211> 233
<212> DNA
<213> Artificial Sequence

<220>
<223> Synthetic DNA: IGHV1-69*07

<400> 271

```
agaagcctgg gtcctcggtg aaggtctcct gcaaggcttc tggaggcacc ttcagcagct      60
atgctatcag ctgggtgcga caggcccctg gacaagggct tgagtggatg ggaaggatca     120
tccctatctt tggtacagca aactacgcac agaagttcca gggcagagtc acgattaccg     180
cggacgaatc cacgagcaca gcctacatgg agctgagcag cctgagatct gag            233
```

<210> 272
<211> 296
<212> DNA
<213> Artificial Sequence

<220>
<223> Synthetic DNA: IGHV1-69*12

<400> 272

```
caggtccagc tggtgcagtc tggggctgag gtgaagaagc ctgggtcctc ggtgaaggtc      60
tcctgcaagg cttctggagg caccttcagc agctatgcta tcagctgggt gcgacaggcc     120
cctggacaag ggcttgagtg gatgggaggg atcatcccta tctttggtac agcaaactac     180
gcacagaagt tccagggcag agtcacgatt accgcggacg aatccacgag cacagcctac     240
atggagctga gcagcctgag atctgaggac acggccgtgt attactgtgc gagaga         296
```

<210> 273
<211> 294
<212> DNA
<213> Artificial Sequence

<220>
<223> Synthetic DNA: IGHV1-69*05

<400> 273

```
caggtccagc tggtgcagtc tggggctgag gtgaagaagc ctgggtcctc ggtgaaggtc      60
tcctgcaagg cttctggagg caccttcagc agctatgcta tcagctgggt gcgacaggcc     120
cctggacaag ggcttgagtg gatgggaggg atcatcccta tctttggtac agcaaactac     180
gcacagaagt tccagggcag agtcacgatt accacggacg aatccacgag cacagcctac     240
atggagctga gcagcctgag atctgaggac acggccgtgt attactgtgc gaga           294
```

<210> 274
<211> 296
<212> DNA
<213> Artificial Sequence

<220>
<223> Synthetic DNA: IGHV1-69*13

<400> 274

```
caggtccagc tggtgcagtc tggggctgag gtgaagaagc ctgggtcctc agtgaaggtc      60
tcctgcaagg cttctggagg caccttcagc agctatgcta tcagctgggt gcgacaggcc     120
cctggacaag ggcttgagtg gatgggaggg atcatcccta tctttggtac agcaaactac     180
gcacagaagt tccagggcag agtcacgatt accgcggacg aatccacgag cacagcctac     240
atggagctga gcagcctgag atctgaggac acggccgtgt attactgtgc gagaga         296
```

<210> 275
<211> 296
<212> DNA
<213> Artificial Sequence

<220>

<223> Synthetic DNA: IGHV1-69*01

<400> 275

```
caggtgcagc tggtgcagtc tggggctgag gtgaagaagc ctgggtcctc ggtgaaggtc        60
tcctgcaagg cttctggagg caccttcagc agctatgcta tcagctgggt gcgacaggcc       120
cctggacaag ggcttgagtg gatgggaggg atcatcccta tctttggtac agcaaactac       180
gcacagaagt tccagggcag agtcacgatt accgcggacg aatccacgag cacagcctac       240
atggagctga gcagcctgag atctgaggac acggccgtgt attactgtgc gagaga           296
```

<210> 276
<211> 296
<212> DNA
<213> Artificial Sequence

<220>
<223> Synthetic DNA: IGHV1-69*06

<400> 276

```
caggtgcagc tggtgcagtc tggggctgag gtgaagaagc ctgggtcctc ggtgaaggtc        60
tcctgcaagg cttctggagg caccttcagc agctatgcta tcagctgggt gcgacaggcc       120
cctggacaag ggcttgagtg gatgggaggg atcatcccta tctttggtac agcaaactac       180
gcacagaagt tccagggcag agtcacgatt accgcggaca aatccacgag cacagcctac       240
atggagctga gcagcctgag atctgaggac acggccgtgt attactgtgc gagaga           296
```

<210> 277
<211> 294
<212> DNA
<213> Artificial Sequence

<220>
<223> Synthetic DNA: IGHV1-69*02

<400> 277

```
caggtccagc tggtgcaatc tggggctgag gtgaagaagc ctgggtcctc ggtgaaggtc        60
tcctgcaagg cttctggagg caccttcagc agctatacta tcagctgggt gcgacaggcc       120
cctggacaag ggcttgagtg gatgggaagg atcatcccta tccttggtat agcaaactac       180
gcacagaagt tccagggcag agtcacgatt accgcggaca aatccacgag cacagcctac       240
atggagctga gcagcctgag atctgaggac acggccgtgt attactgtgc gaga             294
```

<210> 278
<211> 296
<212> DNA
<213> Artificial Sequence

<220>
<223> Synthetic DNA: IGHV1-69*08

<400> 278

```
caggtccagc tggtgcaatc tggggctgag gtgaagaagc ctgggtcctc ggtgaaggtc     60
tcctgcaagg cttctggagg caccttcagc agctatacta tcagctgggt gcgacaggcc    120
cctggacaag ggcttgagtg gatgggaagg atcatcccta tccttggtac agcaaactac    180
gcacagaagt tccagggcag agtcacgatt accgcggaca aatccacgag cacagcctac    240
atggagctga gcagcctgag atctgaggac acggccgtgt attactgtgc gagaga        296
```

<210> 279
<211> 296
<212> DNA
<213> Artificial Sequence

<220>
<223> Synthetic DNA: IGHV1-69*04

<400> 279

```
caggtccagc tggtgcagtc tggggctgag gtgaagaagc ctgggtcctc ggtgaaggtc     60
tcctgcaagg cttctggagg caccttcagc agctatgcta tcagctgggt gcgacaggcc    120
cctggacaag ggcttgagtg gatgggaagg atcatcccta tccttggtat agcaaactac    180
gcacagaagt tccagggcag agtcacgatt accgcggaca aatccacgag cacagcctac    240
atggagctga gcagcctgag atctgaggac acggccgtgt attactgtgc gagaga        296
```

<210> 280
<211> 296
<212> DNA
<213> Artificial Sequence

<220>
<223> Synthetic DNA: IGHV1-69*11

<400> 280

```
caggtccagc tggtgcagtc tggggctgag gtgaagaagc ctgggtcctc ggtgaaggtc     60
tcctgcaagg cttctggagg caccttcagc agctatgcta tcagctgggt gcgacaggcc    120
cctggacaag ggcttgagtg gatgggaagg atcatcccta tccttggtac agcaaactac    180
gcacagaagt tccagggcag agtcacgatt accgcggacg aatccacgag cacagcctac    240
atggagctga gcagcctgag atctgaggac acggccgtgt attactgtgc gagaga        296
```

<210> 281
<211> 296

<212> DNA
<213> Artificial Sequence

<220>
<223> Synthetic DNA: IGHV1-69*09

<400> 281

```
caggtgcagc tggtgcagtc tggggctgag gtgaagaagc ctgggtcctc ggtgaaggtc       60
tcctgcaagg cttctggagg caccttcagc agctatgcta tcagctgggt gcgacaggcc      120
cctggacaag ggcttgagtg gatgggaagg atcatcccta tccttggtat agcaaactac      180
gcacagaagt tccagggcag agtcacgatt accgcggaca aatccacgag cacagcctac      240
atggagctga gcagcctgag atctgaggac acggccgtgt attactgtgc gagaga          296
```

<210> 282
<211> 296
<212> DNA
<213> Artificial Sequence

<220>
<223> Synthetic DNA: IGHV1-69*10

<400> 282

```
caggtccagc tggtgcagtc tggggctgag gtgaagaagc ctgggtcctc agtgaaggtc       60
tcctgcaagg cttctggagg caccttcagc agctatgcta tcagctgggt gcgacaggcc      120
cctggacaag ggcttgagtg gatgggaggg atcatcccta tccttggtat agcaaactac      180
gcacagaagt tccagggcag agtcacgatt accgcggaca aatccacgag cacagcctac      240
atggagctga gcagcctgag atctgaggac acggccgtgt attactgtgc gagaga          296
```

<210> 283
<211> 294
<212> DNA
<213> Artificial Sequence

<220>
<223> Synthetic DNA: IGHV1-f*01

<400> 283

```
gaggtccagc tggtacagtc tggggctgag gtgaagaagc ctggggctac agtgaaaatc       60
tcctgcaagg tttctggata caccttcacc gactactaca tgcactgggt gcaacaggcc      120
cctggaaaag ggcttgagtg gatgggactt gttgatcctg aagatggtga aacaatatac      180
gcagagaagt tccagggcag agtcaccata accgcggaca cgtctacaga cacagcctac      240
atggagctga gcagcctgag atctgaggac acggccgtgt attactgtgc aaca            294
```

<210> 284
<211> 233
<212> DNA
<213> Artificial Sequence

<220>
<223> Synthetic DNA: IGHV1-f*02

<400> 284

agaagcctgg ggctacagtg aaaatctcct gcaaggtttc tggatacacc ttcaccgact 60

actacatgca ctgggtgcaa caggcccctg gaaaagggct tgagtggatg ggacttgttg 120

atcctgaaga tggtgaaaca atatatgcag agaagttcca gggcagagtc accataaccg 180

cggacacgtc tacagacaca gcctacatgg agctgagcag cctgagatct gag 233

<210> 285
<211> 296
<212> DNA
<213> Artificial Sequence

<220>
<223> Synthetic DNA: IGHV1-24*01

<400> 285

caggtccagc tggtacagtc tggggctgag gtgaagaagc ctggggcctc agtgaaggtc 60

tcctgcaagg tttccggata caccctcact gaattatcca tgcactgggt gcgacaggct 120

cctggaaaag ggcttgagtg gatgggaggt tttgatcctg aagatggtga aacaatctac 180

gcacagaagt tccagggcag agtcaccatg accgaggaca catctacaga cacagcctac 240

atggagctga gcagcctgag atctgaggac acggccgtgt attactgtgc aacaga 296

<210> 286
<211> 294
<212> DNA
<213> Artificial Sequence

<220>
<223> Synthetic DNA: IGHV7-4-1*01

<400> 286

caggtgcagc tggtgcaatc tgggtctgag ttgaagaagc ctggggcctc agtgaaggtt 60

tcctgcaagg cttctggata caccttcact agctatgcta tgaattgggt gcgacaggcc 120

cctggacaag ggcttgagtg gatgggatgg atcaacacca acactgggaa cccaacgtat 180

gcccagggct tcacaggacg gtttgtcttc tccttggaca cctctgtcag cacggcatat 240

ctgcagatct gcagcctaaa ggctgaggac actgccgtgt attactgtgc gaga 294

<210> 287
<211> 274
<212> DNA
<213> Artificial Sequence

<220>
<223> Synthetic DNA: IGHV7-4-1*03

<400> 287

```
caggtgcagc tggtgcaatc tgggtctgag ttgaagaagc ctggggcctc agtgaaggtt       60
tcctgcaagg cttctggata caccttcact agctatgcta tgaattgggt gcgacaggcc      120
cctggacaag ggcttgagtg gatgggatgg atcaacacca acactgggaa cccaacgtat      180
gcccagggct tcacaggacg gtttgtcttc tccttggaca cctctgtcag cacggcatat      240
ctgcagatca gcacgctaaa ggctgaggac actg                                  274
```

<210> 288
<211> 296
<212> DNA
<213> Artificial Sequence

<220>
<223> Synthetic DNA: IGHV7-4-1*02

<400> 288

```
caggtgcagc tggtgcaatc tgggtctgag ttgaagaagc ctggggcctc agtgaaggtt       60
tcctgcaagg cttctggata caccttcact agctatgcta tgaattgggt gcgacaggcc      120
cctggacaag ggcttgagtg gatgggatgg atcaacacca acactgggaa cccaacgtat      180
gcccagggct tcacaggacg gtttgtcttc tccttggaca cctctgtcag cacggcatat      240
ctgcagatca gcagcctaaa ggctgaggac actgccgtgt attactgtgc gagaga          296
```

<210> 289
<211> 296
<212> DNA
<213> Artificial Sequence

<220>
<223> Synthetic DNA: IGHV7-81*01

<400> 289

caggtgcagc tggtgcagtc tggccatgag gtgaagcagc ctggggcctc agtgaaggtc 60

tcctgcaagg cttctggtta cagtttcacc acctatggta tgaattgggt gccacaggcc 120

cctggacaag ggcttgagtg gatgggatgg ttcaacacct acactgggaa cccaacatat 180

gcccagggct tcacaggacg gtttgtcttc tccatggaca cctctgccag cacagcatac 240

ctgcagatca gcagcctaaa ggctgaggac atggccatgt attactgtgc gagata 296

<210> 290
<211> 289
<212> DNA
<213> Artificial Sequence

<220>
<223> Synthetic DNA: IGHV7-40*03

<400> 290

ctgcagctgg tgcagtctgg gcctgaggtg aagaagcctg gggcctcagt gaaggtctcc 60

tataagtctt ctggttacac cttcaccatc tatggtatga attgggtatg atagacccct 120

ggacagggct ttgagtggat gtgatggatc atcacctaca ctgggaaccc aacgtatacc 180

cacggcttca caggatggtt tgtcttctcc atggacacgt ctgtcagcac ggcgtgtctt 240

cagatcagca gcctaaaggc tgaggacacg gccgagtatt actgtgcga 289

<210> 291
<211> 296
<212> DNA
<213> Artificial Sequence

<220>
<223> Synthetic DNA: IGHV5-51*01

<400> 291

gaggtgcagc tggtgcagtc tggagcagag gtgaaaaagc ccggggagtc tctgaagatc 60

tcctgtaagg gttctggata cagctttacc agctactgga tcggctgggt gcgccagatg 120

cccgggaaag gcctggagtg gatggggatc atctatcctg gtgactctga taccagatac 180

agcccgtcct tccaaggcca ggtcaccatc tcagccgaca agtccatcag caccgcctac 240

ctgcagtgga gcagcctgaa ggcctcggac accgccatgt attactgtgc gagaca 296

<210> 292
<211> 245
<212> DNA
<213> Artificial Sequence

<220>
<223> Synthetic DNA: IGHV5-51*05

<400> 292

```
aaaagcccgg ggagtctctg aagatctcct gtaagggttc tggatacagc tttaccagct      60
actggatcgg ctgggtgcgc cagatgccca ggaaaggcct ggagtggatg gggatcatct     120
atcctggtga ctctgatacc agatacagcc cgtccttcca aggccaggtc accatctcag     180
ccgacaagtc catcagcacc gcctacctgc agtggagcag cctgaaggcc tcggacaccg     240
ccatg                                                                 245
```

<210> 293
<211> 296
<212> DNA
<213> Artificial Sequence

<220>
<223> Synthetic DNA: IGHV5-51*02

<400> 293

```
gaggtgcagc tggtgcagtc tggagcagag gtgaaaaagc ccggggagtc tctgaagatc      60
tcctgtaagg gttctggata cagctttacc agctactgga ccggctgggt gcgccagatg     120
cccgggaaag gcttggagtg gatggggatc atctatcctg gtgactctga taccagatac     180
agcccgtcct tccaaggcca ggtcaccatc tcagccgaca agtccatcag caccgcctac     240
ctgcagtgga gcagcctgaa ggcctcggac accgccatgt attactgtgc gagaca         296
```

<210> 294
<211> 294
<212> DNA
<213> Artificial Sequence

<220>
<223> Synthetic DNA: IGHV5-51*03

<400> 294

```
gaggtgcagc tggtgcagtc tggagcagag gtgaaaaagc cgggggagtc tctgaagatc      60
tcctgtaagg gttctggata cagctttacc agctactgga tcggctgggt gcgccagatg     120
cccgggaaag gcctggagtg gatggggatc atctatcctg gtgactctga taccagatac     180
agcccgtcct tccaaggcca ggtcaccatc tcagccgaca agtccatcag caccgcctac     240
ctgcagtgga gcagcctgaa ggcctcggac accgccatgt attactgtgc gaga           294
```

<210> 295
<211> 294
<212> DNA
<213> Artificial Sequence

<220>
<223> Synthetic DNA: IGHV5-51*04

<400> 295

```
gaggtgcagc tggtgcagtc tggagcagag gtgaaaaagc cgggggagtc tctgaagatc      60
tcctgtaagg gttctggata cagctttacc agctactgga tcggctgggt gcgccagatg     120
cccgggaaag gcctggagtg gatggggatc atctatcctg gtgactctga taccagatac     180
agcccgtcct tccaaggcca ggtcaccatc tcagccgaca agcccatcag caccgcctac     240
ctgcagtgga gcagcctgaa ggcctcggac accgccatgt attactgtgc gaga           294
```

<210> 296
<211> 294
<212> DNA
<213> Artificial Sequence

<220>
<223> Synthetic DNA: IGHV5-a*01

<400> 296

```
gaagtgcagc tggtgcagtc tggagcagag gtgaaaaagc ccggggagtc tctgaggatc      60
tcctgtaagg gttctggata cagctttacc agctactgga tcagctgggt gcgccagatg     120
cccgggaaag gcctggagtg gatggggagg attgatccta gtgactctta taccaactac     180
agcccgtcct tccaaggcca cgtcaccatc tcagctgaca agtccatcag cactgcctac     240
ctgcagtgga gcagcctgaa ggcctcggac accgccatgt attactgtgc gaga           294
```

<210> 297
<211> 294
<212> DNA
<213> Artificial Sequence

<220>
<223> Synthetic DNA: IGHV5-a*03

<400> 297

```
gaagtgcagc tggtgcagtc cggagcagag gtgaaaaagc ccggggagtc tctgaggatc      60
tcctgtaagg gttctggata cagctttacc agctactgga tcagctgggt gcgccagatg     120
cccgggaaag gcctggagtg gatggggagg attgatccta gtgactctta taccaactac     180
agcccgtcct tccaaggcca cgtcaccatc tcagctgaca agtccatcag cactgcctac     240
ctgcagtgga gcagcctgaa ggcctcggac accgccatgt attactgtgc gaga           294
```

<210> 298
<211> 294
<212> DNA

<213> Artificial Sequence

<220>
<223> Synthetic DNA: IGHV5-a*04

<400> 298

```
gaagtgcagc tggtgcagtc tggagcagag gtgaaaaagc ccggggagtc tctgaggatc      60
tcctgtaagg gttctggata cagctttacc agctactgga tcagctgggt gcgccagatg     120
cccgggaaag gcctggagtg gatggggagg attgatccta gtgactctta taccaactac     180
agcccgtcct tccaaggcca ggtcaccatc tcagctgaca agtccatcag cactgcctac     240
ctgcagtgga gcagcctgaa ggcctcggac accgccatgt attactgtgc gaga           294
```

<210> 299
<211> 295
<212> DNA
<213> Artificial Sequence

<220>
<223> Synthetic DNA: IGHV5-a*02

<400> 299

```
gaagtgcagc tggtgcagtc tggagcagag gtgaaaaagc ccggggagtc tctgaggatc      60
tcctgtaagg gttctggata cagctttacc agctactgga tcagctgggt gcgccagatg     120
cccgggaaag gcttggagtg gatggggagg attgatccta gtgactctta taccaactac     180
agcccgtcct tccaaggcca cgtcaccatc tcagctgaca agtccatcag cactgcctac     240
ctgcagtgga gcagcctgaa ggctcggaca ccgccatgta ttactgtgcg agaca          295
```

<210> 300
<211> 294
<212> DNA
<213> Artificial Sequence

<220>
<223> Synthetic DNA: IGHV5-78*01

<400> 300

```
gaggtgcagc tgttgcagtc tgcagcagag gtgaaaagac ccggggagtc tctgaggatc      60
tcctgtaaga cttctggata cagctttacc agctactgga tccactgggt gcgccagatg     120
cccgggaaag aactggagtg gatggggagc atctatcctg ggaactctga taccagatac     180
agcccatcct tccaaggcca cgtcaccatc tcagccgaca gctccagcag caccgcctac     240
ctgcagtgga gcagcctgaa ggcctcggac gccgccatgt attattgtgt gaga           294
```

<210> 301

<211> 296
<212> DNA
<213> Artificial Sequence

<220>
<223> Synthetic DNA: IGHV3-11*01

<400> 301

caggtgcagc tggtggagtc tgggggaggc ttggtcaagc ctggagggtc cctgagactc 60

tcctgtgcag cctctggatt caccttcagt gactactaca tgagctggat ccgccaggct 120

ccagggaagg ggctggagtg ggtttcatac attagtagta gtggtagtac catatactac 180

gcagactctg tgaagggccg attcaccatc tccagggaca acgccaagaa ctcactgtat 240

ctgcaaatga acagcctgag agccgaggac acggccgtgt attactgtgc gagaga 296

<210> 302
<211> 294
<212> DNA
<213> Artificial Sequence

<220>
<223> Synthetic DNA: IGHV3-11*03

<400> 302

caggtgcagc tgttggagtc tgggggaggc ttggtcaagc ctggagggtc cctgagactc 60

tcctgtgcag cctctggatt caccttcagt gactactaca tgagctggat ccgccaggct 120

ccagggaagg ggctggagtg ggtttcatac attagtagta gtagtagtta cacaaactac 180

gcagactctg tgaagggccg attcaccatc tccagagaca acgccaagaa ctcactgtat 240

ctgcaaatga acagcctgag agccgaggac acggccgtgt attactgtgc gaga 294

<210> 303
<211> 296
<212> DNA
<213> Artificial Sequence

<220>
<223> Synthetic DNA: IGHV3-21*01

<400> 303

gaggtgcagc tggtggagtc tgggggaggc ctggtcaagc ctgggggtc cctgagactc 60

tcctgtgcag cctctggatt caccttcagt agctatagca tgaactgggt ccgccaggct 120

ccagggaagg ggctggagtg ggtctcatcc attagtagta gtagtagtta catatactac 180

gcagactcag tgaagggccg attcaccatc tccagagaca acgccaagaa ctcactgtat 240

ctgcaaatga acagcctgag agccgaggac acggctgtgt attactgtgc gagaga 296

<210> 304
<211> 296
<212> DNA
<213> Artificial Sequence

<220>
<223> Synthetic DNA: IGHV3-21*02

<400> 304

gaggtgcaac tggtggagtc tgggggaggc ctggtcaagc ctggggggtc cctgagactc 60

tcctgtgcag cctctggatt caccttcagt agctatagca tgaactgggt ccgccaggct 120

ccagggaagg ggctggagtg ggtctcatcc attagtagta gtagtagtta catatactac 180

gcagactcag tgaagggccg attcaccatc tccagagaca acgccaagaa ctcactgtat 240

ctgcaaatga acagcctgag agccgaggac acggctgtgt attactgtgc gagaga 296

<210> 305
<211> 296
<212> DNA
<213> Artificial Sequence

<220>
<223> Synthetic DNA: IGHV3-48*01

<400> 305

gaggtgcagc tggtggagtc tgggggaggc ttggtacagc ctggggggtc cctgagactc 60

tcctgtgcag cctctggatt caccttcagt agctatagca tgaactgggt ccgccaggct 120

ccagggaagg ggctggagtg ggtttcatac attagtagta gtagtagtac catatactac 180

gcagactctg tgaagggccg attcaccatc tccagagaca atgccaagaa ctcactgtat 240

ctgcaaatga acagcctgag agccgaggac acggctgtgt attactgtgc gagaga 296

<210> 306
<211> 296
<212> DNA
<213> Artificial Sequence

<220>
<223> Synthetic DNA: IGHV3-48*02

<400> 306

gaggtgcagc tggtggagtc tgggggaggc ttggtacagc ctggggggtc cctgagactc 60

tcctgtgcag cctctggatt caccttcagt agctatagca tgaactgggt ccgccaggct 120

ccagggaagg ggctggagtg ggtttcatac attagtagta gtagtagtac catatactac 180

gcagactctg tgaagggccg attcaccatc tccagagaca atgccaagaa ctcactgtat 240

ctgcaaatga acagcctgag agacgaggac acggctgtgt attactgtgc gagaga 296

<210> 307
<211> 293
<212> DNA
<213> Artificial Sequence

<220>
<223> Synthetic DNA: IGHV3-h*01

<400> 307

gaggtgcagc tggtggagtc tgggggaggc ttggtaaagc ctggggggtc cctgagactc 60

tcctgtgcag cctctggatt caccttcagt gactactaca tgaactgggt ccgccaggct 120

ccagggaagg ggctggagtg ggtctcatcc attagtagta gtagtaccat atactacgca 180

gactctgtga agggccgatt caccatctcc agagacaacg ccaagaactc actgtatctg 240

caaatgaaca gcctgagagc cgaggacacg gctgtgtatt actgtgcgag aga 293

<210> 308
<211> 293
<212> DNA
<213> Artificial Sequence

<220>
<223> Synthetic DNA: IGHV3-h*02

<400> 308

gaggtgcagc tggtggagtc tgggggaggc ttggtaaagc ctggggggtc cctgagactc 60

tcctgtgcag cctctggatt caccttcagt gactactaca tgaactgggt ccgccaggct 120

ccagggaagg ggctggagtg ggtctcatcc attagtagta gtagtaccat atactacgca 180

gactctgtga agggccgatt caccatctcc agagacaacg ccaagaactc actgtatctg 240

caaatgaaca gcctgagagc cgaggacacg gctgtttatt actgtgcgag aga 293

<210> 309
<211> 296
<212> DNA
<213> Artificial Sequence

<220>
<223> Synthetic DNA: IGHV3-48*03

<400> 309

gaggtgcagc tggtggagtc tgggggaggc ttggtacagc ctggagggtc cctgagactc 60
tcctgtgcag cctctggatt caccttcagt agttatgaaa tgaactgggt ccgccaggct 120
ccagggaagg ggctggagtg ggtttcatac attagtagta gtggtagtac catatactac 180
gcagactctg tgaagggccg attcaccatc tccagagaca acgccaagaa ctcactgtat 240
ctgcaaatga acagcctgag agccgaggac acggctgttt attactgtgc gagaga 296

<210> 310
<211> 292
<212> DNA
<213> Artificial Sequence

<220>
<223> Synthetic DNA: IGHV3/OR16-8*01

<400> 310

gaggtgcagc tggtggagtc tgggggaggc ttggtacagc ctggggggtc cctgagactg 60
tcctgtccag cctctggatt caccttcagt aaccactaca tgagctgggt ccgccaggct 120
ccagggaagg gactggagtg ggtttcatac attagtggtg atagtggtta cacaaactac 180
gcagactctg tgaagggccg attcaccatc tccagggaca acgccaataa ctcaccgtat 240
ctgcaaatga acagcctgag agctgaggac acggctgtgt attactgtgt ga 292

<210> 311
<211> 292
<212> DNA
<213> Artificial Sequence

<220>
<223> Synthetic DNA: IGHV3/OR16-9*01

<400> 311

gaggtgcagc tggtggagtc tggaggaggc ttggtacagc ctggggggtc cctgagactc 60
tcctgtgcag cctctggatt caccttcagt aaccactaca cgagctgggt ccgccaggct 120
ccagggaagg gactggagtg ggtttcatac agtagtggta atagtggtta cacaaactac 180
gcagactctg tgaaaggccg attcaccatc tccagggaca acgccaagaa ctcactgtat 240
ctgcaaatga acagcctgag agccgaggac acggctgtgt attactgtgt ga 292

<210> 312
<211> 293
<212> DNA
<213> Artificial Sequence

<220>

<223> Synthetic DNA: IGHV3-13*01

<400> 312

```
gaggtgcagc tggtggagtc tgggggaggc ttggtacagc ctgggggtc cctgagactc        60
tcctgtgcag cctctggatt caccttcagt agctacgaca tgcactgggt ccgccaagct       120
acaggaaaag gtctggagtg ggtctcagct attggtactg ctggtgacac atactatcca       180
ggctccgtga agggccgatt caccatctcc agagaaaatg ccaagaactc cttgtatctt       240
caaatgaaca gcctgagagc cggggacacg gctgtgtatt actgtgcaag aga              293
```

<210> 313
<211> 291
<212> DNA
<213> Artificial Sequence

<220>
<223> Synthetic DNA: IGHV3-13*03

<400> 313

```
gaggtgcagc tggtggagtc tgggggaggc ttggtacagc ctgggggtc cctgagactc        60
tcctgtgcag cctgtggatt caccttcagt agctacgaca tgcactgggt ccgccaagct       120
acaggaaaag gtctggagtg ggtctcagct attggtactg ctggtgacac atactatcca       180
ggctccgtga agggccaatt caccatctcc agagaaaatg ccaagaactc cttgtatctt       240
caaatgaaca gcctgagagc cggggacacg gctgtgtatt actgtgcaag a                291
```

<210> 314
<211> 293
<212> DNA
<213> Artificial Sequence

<220>
<223> Synthetic DNA: IGHV3-13*02

<400> 314

```
gaggtgcatc tggtggagtc tgggggaggc ttggtacagc ctgggggggc cctgagactc       60
tcctgtgcag cctctggatt caccttcagt aactacgaca tgcactgggt ccgccaagct       120
acaggaaaag gtctggagtg ggtctcagcc aatggtactg ctggtgacac atactatcca       180
ggctccgtga aggggcgatt caccatctcc agagaaaatg ccaagaactc cttgtatctt       240
caaatgaaca gcctgagagc cggggacacg gctgtgtatt actgtgcaag aga              293
```

<210> 315
<211> 123
<212> DNA
<213> Artificial Sequence

<220>
<223> Synthetic DNA: IGHV3-47*02

<400> 315

atactatgca gactccgtga tggccgatt caccatctcc agagacaacg ccaagaagtc 60

cttgtatctt caaatgaaca gcctgatagc tgaggacatg gctgtgtatt attgtgcaag 120

aga 123

<210> 316
<211> 282
<212> DNA
<213> Artificial Sequence

<220>
<223> Synthetic DNA: IGHV3-47*03

<400> 316

gaggatcagc tggtggagtc tgggggaggc ttggtacagc ctgggggtc cctgagaccc 60

tcctgtgcag cctctggatt cgccttcagt agctatgttc tgcactgggt tcgccgggct 120

ccagggaagg gtccggagtg ggtatcagct attggtactg gtggtgatac atactatgca 180

gactccgtga tgggccgatt caccatctcc agagacaacg ccaagaagtc cttgtatctc 240

aaatgaacag cctgatagct gaggacatgg ctgtgtatta tg 282

<210> 317
<211> 291
<212> DNA
<213> Artificial Sequence

<220>
<223> Synthetic DNA: IGHV3-47*01

<400> 317

gaggatcagc tggtggagtc tgggggaggc ttggtacagc ctgggggtc cctgcgaccc 60

tcctgtgcag cctctggatt cgccttcagt agctatgctc tgcactgggt tcgccgggct 120

ccagggaagg gtctggagtg ggtatcagct attggtactg gtggtgatac atactatgca 180

gactccgtga tgggccgatt caccatctcc agagacaacg ccaagaagtc cttgtatctt 240

catatgaaca gcctgatagc tgaggacatg gctgtgtatt attgtgcaag a 291

<210> 318
<211> 291
<212> DNA
<213> Artificial Sequence

<220>

<223> Synthetic DNA: IGHV3/OR16-10*01

<400> 318

```
gaggttcagc tggtgcagtc tgggggaggc ttggtacatc ctgggggtc cctgagactc        60
tcctgtgcag gctctggatt caccttcagt agctatgcta tgcactgggt tcgccaggct      120
ccaggaaaag gtctggagtg ggtatcagct attggtactg gtggtggcac atactatgca      180
gactccgtga agggccgatt caccatctcc agagacaatg ccaagaactc cttgtatctt      240
caaatgaaca gcctgagagc cgaggacatg gctgtgtatt actgtgcaag a               291
```

<210> 319
<211> 291
<212> DNA
<213> Artificial Sequence

<220>
<223> Synthetic DNA: IGHV3/OR16-10*02

<400> 319

```
gaggttcagc tggtgcagtc tgggggaggc ttggtacagc ctgggggtc cctgagactc        60
tcctgtgcag gctctggatt caccttcagt agctatgcta tgcactgggt tcgccaggct      120
ccaggaaaag gtctggagtg ggtatcagct attggtactg gtggtggcac atactatgca      180
gactccgtga agggccgatt caccatctcc agagacaatg ccaagaactc cttgtatctt      240
caaatgaaca gcctgagagc cgaggacatg gctgtgtatt actgtgcaag a               291
```

<210> 320
<211> 296
<212> DNA
<213> Artificial Sequence

<220>
<223> Synthetic DNA: IGHV3-62*01

<400> 320

```
gaggtgcagc tggtggagtc tggggaaggc ttggtccagc ctgggggtc cctgagactc        60
tcctgtgcag cctctggatt caccttcagt agctctgcta tgcactgggt ccgccaggct      120
ccaagaaagg gtttgtagtg ggtctcagtt attagtacaa gtggtgatac cgtactctac      180
acagactctg tgaagggccg attcaccatc tccagagaca atgcccagaa ttcactgtct      240
ctgcaaatga acagcctgag agccgagggc acagttgtgt actactgtgt gaaaga          296
```

<210> 321
<211> 296
<212> DNA
<213> Artificial Sequence

<220>
<223> Synthetic DNA: IGHV3-64*01

<400> 321

```
gaggtgcagc tggtggagtc tgggggaggc ttggtccagc ctggggggtc cctgagactc    60
tcctgtgcag cctctggatt caccttcagt agctatgcta tgcactgggt ccgccaggct   120
ccagggaagg gactggaata tgtttcagct attagtagta atgggggtag cacatattat   180
gcaaactctg tgaagggcag attcaccatc tccagagaca attccaagaa cacgctgtat   240
cttcaaatgg gcagcctgag agctgaggac atggctgtgt attactgtgc gagaga       296
```

<210> 322
<211> 296
<212> DNA
<213> Artificial Sequence

<220>
<223> Synthetic DNA: IGHV3-64*02

<400> 322

```
gaggtgcagc tggtggagtc tggggaaggc ttggtccagc ctggggggtc cctgagactc    60
tcctgtgcag cctctggatt caccttcagt agctatgcta tgcactgggt ccgccaggct   120
ccagggaagg gactggaata tgtttcagct attagtagta atgggggtag cacatattat   180
gcagactctg tgaagggcag attcaccatc tccagagaca attccaagaa cacgctgtat   240
cttcaaatgg gcagcctgag agctgaggac atggctgtgt attactgtgc gagaga       296
```

<210> 323
<211> 296
<212> DNA
<213> Artificial Sequence

<220>
<223> Synthetic DNA: IGHV3-64*03

<400> 323

```
gaggtgcagc tggtggagtc tgggggaggc ttggtccagc ctggggggtc cctgagactc    60
tcctgttcag cctctggatt caccttcagt agctatgcta tgcactgggt ccgccaggct   120
ccagggaagg gactggaata tgtttcagct attagtagta atgggggtag cacatactac   180
gcagactcag tgaagggcag attcaccatc tccagagaca attccaagaa cacgctgtat   240
gtccaaatga gcagtctgag agctgaggac acggctgtgt attactgtgt gaaaga       296
```

<210> 324
<211> 296
<212> DNA

<213> Artificial Sequence

<220>
<223> Synthetic DNA: IGHV3-64*05

<400> 324

gaggtgcagc tggtggagtc tgggggaggc ttggtccagc ctggggggtc cctgagactc 60
tcctgttcag cctctggatt caccttcagt agctatgcta tgcactgggt ccgccaggct 120
ccagggaagg gactggaata tgtttcagct attagtagta atgggggtag cacatactac 180
gcagactcag tgaagggcag attcaccatc tccagagaca attccaagaa cacgctgtat 240
gttcaaatga gcagtctgag agctgaggac acggctgtgt attactgtgt gaaaga 296

<210> 325
<211> 296
<212> DNA
<213> Artificial Sequence

<220>
<223> Synthetic DNA: IGHV3-64*04

<400> 325

caggtgcagc tggtggagtc tgggggaggc ttggtccagc ctggggggtc cctgagactc 60
tcctgttcag cctctggatt caccttcagt agctatgcta tgcactgggt ccgccaggct 120
ccagggaagg gactggaata tgtttcagct attagtagta atgggggtag cacatactac 180
gcagactcag tgaagggcag attcaccatc tccagagaca attccaagaa cacgctgtat 240
ctgcaaatga acagcctgag agctgaggac acggctgtgt attactgtgc gagaga 296

<210> 326
<211> 296
<212> DNA
<213> Artificial Sequence

<220>
<223> Synthetic DNA: IGHV3-16*01

<400> 326

gaggtacaac tggtggagtc tgggggaggc ttggtacagc ctggggggtc cctgagactc 60
tcctgtgcag cctctggatt caccttcagt aacagtgaca tgaactgggc ccgcaaggct 120
ccaggaaagg ggctggagtg ggtatcgggt gttagttgga atggcagtag gacgcactat 180
gtggactccg tgaagcgccg attcatcatc tccagagaca attccaggaa ctccctgtat 240
ctgcaaaaga acagacggag agccgaggac atggctgtgt attactgtgt gagaaa 296

<210> 327
<211> 296
<212> DNA
<213> Artificial Sequence

<220>
<223> Synthetic DNA: IGHV3-16*02

<400> 327

```
gaggtgcagc tggtggagtc tgggggaggc ttggtacagc ctggggggtc cctgagactc      60
tcctgtgcag cctctggatt caccttcagt aacagtgaca tgaactgggc ccgcaaggct     120
ccaggaaagg ggctggagtg ggtatcgggt gttagttgga atggcagtag gacgcactat     180
gtggactccg tgaagcgccg attcatcatc tccagagaca attccaggaa ctccctgtat     240
ctgcaaaaga acagacggag agccgaggac atggctgtgt attactgtgt gagaaa         296
```

<210> 328
<211> 294
<212> DNA
<213> Artificial Sequence

<220>
<223> Synthetic DNA: IGHV3/OR16-15*02

<400> 328

```
gaggtgcagc tggtggagtc tgggggaggc ttggtccagc ctggggggtc cctgagacac      60
tcctgtgcag cctctggatt caccttcagt aacagtgaca tgaactgggt cctctaggct     120
ccaggaaagg ggctggagtg ggtctcgggt attagttgga atggcggtaa gacgcactat     180
gtggactccg tgaagggcca atttaccatc tccagagaca attccagcaa gtccctgtat     240
ctgcaaaaga acagacagag agccaaagac atggccgtgt attactgtgt gaga           294
```

<210> 329
<211> 294
<212> DNA
<213> Artificial Sequence

<220>
<223> Synthetic DNA: IGHV3/OR16-16*01

<400> 329

```
gaggtgcagc tggtggagtc tgggggaggc ttggtccagc ctggggggtc cctgagacac      60
tcctgtgcag cctctggatt caccttcagt aacagtgaca tgaactgggt cctctaggct     120
ccaggaaagg ggctggagtg ggtctcggat attagttgga atggcggtaa gacgcactat     180
```

gtggactccg tgaagggcca atttaccatc tccagagaca attccagcaa gtccctgtat 240

ctgcaaaaga acagacagag agccaaggac atggccgtgt attactgtgt gaga 294

<210> 330
<211> 296
<212> DNA
<213> Artificial Sequence

<220>
<223> Synthetic DNA: IGHV3/OR16-15*01

<400> 330

gaagtgcagc tggtggagtc tgggggaggc ttggtccagc ctggggggtc cctgagactc 60

tcctgtgcag cctctgtatt caccttcagt aacagtgaca taaactgggt cctctaggct 120

ccaggaaagg ggctggagtg ggtctcgggt attagttgga atggcggtaa gacgcactat 180

gtggactccg tgaagggcca attttccatc tccagagaca attccagcaa gtccctgtat 240

ctgcaaaaga acagacagag agccaaggac atggccgtgt attactgtgt gagaaa 296

<210> 331
<211> 296
<212> DNA
<213> Artificial Sequence

<220>
<223> Synthetic DNA: IGHV3-19*01

<400> 331

acagtgcagc tggtggagtc tgggggaggc ttggtagagc ctggggggtc cctgagactc 60

tcctgtgcag cctctggatt caccttcagt aacagtgaca tgaactgggt ccgccaggct 120

ccaggaaagg ggctggagtg ggtatcgggt gttagttgga atggcagtag gacgcactat 180

gcagactctg tgaagggccg attcatcatc tccagagaca attccaggaa cttcctgtat 240

cagcaaatga acagcctgag gcccgaggac atggctgtgt attactgtgt gagaaa 296

<210> 332
<211> 296
<212> DNA
<213> Artificial Sequence

<220>
<223> Synthetic DNA: IGHV3-35*01

<400> 332

gaggtgcagc tggtggagtc tgggggaggc ttggtacagc ctggggggatc cctgagactc 60

tcctgtgcag cctctggatt caccttcagt aacagtgaca tgaactgggt ccatcaggct 120

ccaggaaagg ggctggagtg ggtatcgggt gttagttgga atggcagtag gacgcactat 180

gcagactctg tgaagggccg attcatcatc tccagagaca attccaggaa caccctgtat 240

ctgcaaacga atagcctgag ggccgaggac acggctgtgt attactgtgt gagaaa 296

<210> 333
<211> 298
<212> DNA
<213> Artificial Sequence

<220>
<223> Synthetic DNA: IGHV3-43*01

<400> 333

gaagtgcagc tggtggagtc tgggggagtc gtggtacagc ctgggggggtc cctgagactc 60

tcctgtgcag cctctggatt cacctttgat gattatacca tgcactgggt ccgtcaagct 120

ccggggaagg gtctggagtg ggtctctctt attagttggg atggtggtag cacatactat 180

gcagactctg tgaagggccg attcaccatc tccagagaca acagcaaaaa ctccctgtat 240

ctgcaaatga acagtctgag aactgaggac accgccttgt attactgtgc aaaagata 298

<210> 334
<211> 294
<212> DNA
<213> Artificial Sequence

<220>
<223> Synthetic DNA: IGHV3-43*02

<400> 334

gaagtgcagc tggtggagtc tgggggaggc gtggtacagc ctgggggggtc cctgagactc 60

tcctgtgcag cctctggatt cacctttgat gattatgcca tgcactgggt ccgtcaagct 120

ccagggaagg gtctggagtg ggtctctctt attagtgggg atggtggtag cacatactat 180

gcagactctg tgaagggccg attcaccatc tccagagaca acagcaaaaa ctccctgtat 240

ctgcaaatga acagtctgag aactgaggac accgccttgt attactgtgc aaaa 294

<210> 335
<211> 298
<212> DNA
<213> Artificial Sequence

<220>
<223> Synthetic DNA: IGHV3-9*01

<400> 335

gaagtgcagc tggtggagtc tgggggaggc ttggtacagc ctggcaggtc cctgagactc 60

tcctgtgcag cctctggatt cacctttgat gattatgcca tgcactgggt ccggcaagct 120

ccagggaagg gcctggagtg ggtctcaggt attagttgga atagtggtag cataggctat 180

gcggactctg tgaagggccg attcaccatc tccagagaca acgccaagaa ctccctgtat 240

ctgcaaatga acagtctgag agctgaggac acggccttgt attactgtgc aaaagata 298

<210> 336
<211> 296
<212> DNA
<213> Artificial Sequence

<220>
<223> Synthetic DNA: IGHV3-20*01

<400> 336

gaggtgcagc tggtggagtc tgggggaggt gtggtacggc ctggggggtc cctgagactc 60

tcctgtgcag cctctggatt cacctttgat gattatggca tgagctgggt ccgccaagct 120

ccagggaagg ggctggagtg ggtctctggt attaattgga atggtggtag cacaggttat 180

gcagactctg tgaagggccg attcaccatc tccagagaca acgccaagaa ctccctgtat 240

ctgcaaatga acagtctgag agccgaggac acggccttgt atcactgtgc gagaga 296

<210> 337
<211> 296
<212> DNA
<213> Artificial Sequence

<220>
<223> Synthetic DNA: IGHV3-74*01

<400> 337

gaggtgcagc tggtggagtc cgggggaggc ttagttcagc ctggggggtc cctgagactc 60

tcctgtgcag cctctggatt caccttcagt agctactgga tgcactgggt ccgccaagct 120

ccagggaagg ggctggtgtg ggtctcacgt attaatagtg atgggagtag cacaagctac 180

gcggactccg tgaagggccg attcaccatc tccagagaca acgccaagaa cacgctgtat 240

ctgcaaatga acagtctgag agccgaggac acggctgtgt attactgtgc aagaga 296

<210> 338
<211> 294
<212> DNA
<213> Artificial Sequence

<220>

<223> Synthetic DNA: IGHV3-74*02

<400> 338

```
gaggtgcagc tggtggagtc tgggggaggc ttagttcagc ctggggggtc cctgagactc       60
tcctgtgcag cctctggatt caccttcagt agctactgga tgcactgggt ccgccaagct      120
ccagggaagg ggctggtgtg ggtctcacgt attaatagtg atgggagtag cacaagctac      180
gcggactccg tgaagggccg attcaccatc tccagagaca acgccaagaa cacgctgtat      240
ctgcaaatga acagtctgag agccgaggac acggctgtgt attactgtgc aaga            294
```

<210> 339
<211> 296
<212> DNA
<213> Artificial Sequence

<220>
<223> Synthetic DNA: IGHV3-74*03

<400> 339

```
gaggtgcagc tggtggagtc cgggggaggc ttagttcagc ctggggggtc cctgagactc       60
tcctgtgcag cctctggatt caccttcagt agctactgga tgcactgggt ccgccaagct      120
ccagggaagg ggctggtgtg ggtctcacgt attaatagtg atgggagtag cacaacgtac      180
gcggactccg tgaagggccg attcaccatc tccagagaca acgccaagaa cacgctgtat      240
ctgcaaatga acagtctgag agccgaggac acggctgtgt attactgtgc aagaga          296
```

<210> 340
<211> 294
<212> DNA
<213> Artificial Sequence

<220>
<223> Synthetic DNA: IGHV3/OR16-13*01

<400> 340

```
gaggtgcagc tggtggagtc tgggggaggc ttagtacagc ctggagggtc cctgagactc       60
tcctgtgcag cctctggatt caccttcagt agctactgga tgcactgggt ccgccaagct      120
ccagggaagg ggctggtgtg ggtctcacgt attaatagtg atgggagtag cacaagctac      180
gcagactcca tgaagggcca attcaccatc tccagagaca atgctaagaa cacgctgtat      240
ctgcaaatga acagtctgag agctgaggac atggctgtgt attactgtac taga            294
```

<210> 341
<211> 294
<212> DNA

<213> Artificial Sequence

<220>
<223> Synthetic DNA: IGHV3/OR16-14*01

<400> 341

```
gaggtgcagc tggaggagtc tgggggaggc ttagtacagc ctggagggtc cctgagactc     60
tcctgtgcag cctctggatt caccttcagt agctactgga tgcactgggt ccgccaatct    120
ccagggaagg ggctggtgtg agtctcacgt attaatagtg atgggagtag cacaagctac    180
gcagactcct tgaagggcca attcaccatc tccagagaca atgctaagaa cacgctgtat    240
ctgcaaatga acagtctgag agctgaggac atggctgtgt attactgtac taga          294
```

<210> 342
<211> 296
<212> DNA
<213> Artificial Sequence

<220>
<223> Synthetic DNA: IGHV3-30*01

<400> 342

```
caggtgcagc tggtggagtc tgggggaggc gtggtccagc ctgggaggtc cctgagactc     60
tcctgtgcag cctctggatt caccttcagt agctatgcta tgcactgggt ccgccaggct    120
ccaggcaagg ggctagagtg ggtggcagtt atatcatatg atggaagtaa taaatactac    180

gcagactccg tgaagggccg attcaccatc tccagagaca attccaagaa cacgctgtat    240
ctgcaaatga acagcctgag agctgaggac acggctgtgt attactgtgc gagaga        296
```

<210> 343
<211> 294
<212> DNA
<213> Artificial Sequence

<220>
<223> Synthetic DNA: IGHV3-30*08

<400> 343

```
caggtgcagc tggtggactc tgggggaggc gtggtccagc ctgggaggtc cctgagactc     60
tcctgtgcag cctctgcatt caccttcagt agctatgcta tgcactgggt ccgccaggct    120
ccaggcaagg ggctagagtg ggtggcagtt atatcatatg atggaagtaa taaatactac    180
gcagactccg tgaagggccg attcaccatc tccagagaca attccaagaa cacgctgtat    240
ctgcaaatga acagcctgag agctgaggac acggctgtgt attactgtgc gaga          294
```

<210> 344
<211> 296
<212> DNA
<213> Artificial Sequence

<220>
<223> Synthetic DNA: IGHV3-30*17

<400> 344

caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccgggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgaggac acggctgtgt attactgtgc gagaga 296

<210> 345
<211> 296
<212> DNA
<213> Artificial Sequence

<220>
<223> Synthetic DNA: IGHV3-30*11

<400> 345

caggtgcagc tggtggagtc tgggggaggc gtggtccagc ctgggaggtc cctgagactc 60

tcctgtgcag cgtctggatt caccttcagt agctatgcta tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgaggac acggctgtgt attactgtgc gagaga 296

<210> 346
<211> 296
<212> DNA
<213> Artificial Sequence

<220>
<223> Synthetic DNA: IGHV3-30*10

<400> 346

caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctagagtg ggtggcagtt atatcatatg atggaagtaa taaatactac 180

acagactccg tgaagggccg attcaccatc tccagagaca attccaagaa cacgctgtat 240

ctgcaaatga acagcctgag agctgaggac acggctgtgt attactgtgc gagaga 296

<210> 347
<211> 296
<212> DNA
<213> Artificial Sequence

<220>
<223> Synthetic DNA: IGHV3-30*16

<400> 347

caggtgcagc tggtggagtc tgggggaggc gtggtccagc ctgggaggtc cctgagactc 60

tcctgtgcag cctctggatt caccttcagt agctatgcta tgcactgggt ccgccaggcc 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgaggac acggctgtgt attactgtgc gagaga 296

<210> 348
<211> 296
<212> DNA
<213> Artificial Sequence

<220>
<223> Synthetic DNA: IGHV3-30*15

<400> 348

caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga gcagcctgag agctgaggac acggctgtgt attactgtgc gagaga 296

<210> 349
<211> 296
<212> DNA
<213> Artificial Sequence

<220>
<223> Synthetic DNA: IGHV3-30*07

<400> 349

caggtgcagc tggtggagtc tgggggaggc gtggtccagc ctgggaggtc cctgagactc 60
tcctgtgcag cctctggatt caccttcagt agctatgcta tgcactgggt ccgccaggct 120
ccaggcaagg ggctagagtg ggtggcagtt atatcatatg atggaagtaa taaatactac 180
gcagactccg tgaagggccg attcaccatc tccagagaca attccaagaa cacgctgtat 240
ctgcaaatga acagcctgag agccgaggac acggctgtgt attactgtgc gagaga 296

<210> 350
<211> 296
<212> DNA
<213> Artificial Sequence

<220>
<223> Synthetic DNA: IGHV3-30*04

<400> 350

caggtgcagc tggtggagtc tgggggaggc gtggtccagc ctgggaggtc cctgagactc 60
tcctgtgcag cctctggatt caccttcagt agctatgcta tgcactgggt ccgccaggct 120
ccaggcaagg ggctggagtg ggtggcagtt atatcatatg atggaagtaa taaatactac 180
gcagactccg tgaagggccg attcaccatc tccagagaca attccaagaa cacgctgtat 240
ctgcaaatga acagcctgag agctgaggac acggctgtgt attactgtgc gagaga 296

<210> 351
<211> 296
<212> DNA
<213> Artificial Sequence

<220>
<223> Synthetic DNA: IGHV3-30*09

<400> 351

caggtgcagc tggtggagtc tgggggaggc gtggtccagc ctgggaggtc cctgagactc 60
tcctgtgcag cctctggatt caccttcagt agctatgcta tgcactgggt ccgccaggct 120
ccaggcaagg ggctggagtg ggtggcagtt atatcatatg atggaagtaa taaatactac 180
gcagactccg tgaagggccg attcgccatc tccagagaca attccaagaa cacgctgtat 240
ctgcaaatga acagcctgag agctgaggac acggctgtgt attactgtgc gagaga 296

<210> 352
<211> 296
<212> DNA
<213> Artificial Sequence

<220>

<223> Synthetic DNA: IGHV3-30*14

<400> 352

caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctggagtg ggtggcagtt atatcatatg atggaagtaa taaatactac 180

gcagactccg tgaagggccg attcaccatc tccagagaca attccaagaa cacgctgtat 240

cttcaaatga acagcctgag agctgaggac acggctgtgt attactgtgc gagaga 296

<210> 353
<211> 294
<212> DNA
<213> Artificial Sequence

<220>
<223> Synthetic DNA: IGHV3-30-3*01

<400> 353

caggtgcagc tggtggagtc tgggggaggc gtggtccagc ctgggaggtc cctgagactc 60

tcctgtgcag cctctggatt caccttcagt agctatgcta tgcactgggt ccgccaggct 120

ccaggcaagg ggctggagtg ggtggcagtt atatcatatg atggaagcaa taaatactac 180

gcagactccg tgaagggccg attcaccatc tccagagaca attccaagaa cacgctgtat 240

ctgcaaatga acagcctgag agctgaggac acggctgtgt attactgtgc gaga 294

<210> 354
<211> 296
<212> DNA
<213> Artificial Sequence

<220>
<223> Synthetic DNA: IGHV3-30-3*02

<400> 354

caggtgcagc tggtggagtc tgggggaggc gtggtccagc ctgggaggtc cctgagactc 60

tcctgtgcag cgtctggatt caccttcagt agctatgcta tgcactgggt ccgccaggct 120

ccaggcaagg ggctggagtg ggtggcagtt atatcatatg atggaagcaa taaatactac 180

gcagactccg tgaagggccg attcaccatc tccagagaca attccaagaa cacgctgtat 240

ctgcaaatga acagcctgag agctgaggac acggctgtgt attactgtgc gaaaga 296

<210> 355
<211> 296
<212> DNA

<213> Artificial Sequence

<220>
<223> Synthetic DNA: IGHV3-30*03

<400> 355

```
caggtgcagc tggtggagtc tgggggaggc gtggtccagc ctgggaggtc cctgagactc        60
tcctgtgcag cctctggatt caccttcagt agctatggca tgcactgggt ccgccaggct       120
ccaggcaagg ggctggagtg ggtggcagtt atatcatatg atggaagtaa taaatactat       180
gcagactccg tgaagggccg attcaccatc tccagagaca attccaagaa cacgctgtat       240
ctgcaaatga acagcctgag agctgaggac acggctgtgt attactgtgc gagaga           296
```

<210> 356
<211> 296
<212> DNA
<213> Artificial Sequence

<220>
<223> Synthetic DNA: IGHV3-30*18

<400> 356

```
caggtgcagc tggtggagtc tgggggaggc gtggtccagc ctgggaggtc cctgagactc        60
tcctgtgcag cctctggatt caccttcagt agctatggca tgcactgggt ccgccaggct       120
ccaggcaagg ggctggagtg ggtggcagtt atatcatatg atggaagtaa taaatactat       180
gcagactccg tgaagggccg attcaccatc tccagagaca attccaagaa cacgctgtat       240
ctgcaaatga acagcctgag agctgaggac acggctgtgt attactgtgc gaaaga           296
```

<210> 357
<211> 296
<212> DNA
<213> Artificial Sequence

<220>
<223> Synthetic DNA: IGHV3-30*06

<400> 357

```
caggtgcagc tggtggagtc tgggggaggc gtggtccagc ctgggaggtc cctgagactc        60
tcctgtgcag cgtctggatt caccttcagt agctatggca tgcactgggt ccgccaggct       120
ccaggcaagg ggctagagtg ggtggcagtt atatcatatg atggaagtaa taaatactac       180
gcagactccg tgaagggccg attcaccatc tccagagaca attccaagaa cacgctgtat       240
ctgcaaatga acagcctgag agctgaggac acggctgtgt attactgtgc gagaga           296
```

<210> 358
<211> 296
<212> DNA
<213> Artificial Sequence

<220>
<223> Synthetic DNA: IGHV3-30*12

<400> 358

caggtgcagc tggtggagtc tgggggggc gtggtccagc ctgggaggtc cctgagactc 60

tcctgtgcag cgtctggatt caccttcagt agctatggca tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agccgaggac acggctgtgt attactgtgc gagaga 296

<210> 359
<211> 296
<212> DNA
<213> Artificial Sequence

<220>
<223> Synthetic DNA: IGHV3-30*19

<400> 359

caggtgcagc tggtggagtc tggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgaggac acggctgtgt attactgtgc gagaga 296

<210> 360
<211> 296
<212> DNA
<213> Artificial Sequence

<220>
<223> Synthetic DNA: IGHV3-33*05

<400> 360

caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatcatatg atggaagtaa taaatactat 180

gcagactccg tgaagggccg attcaccatc tccagagaca attccaagaa cacgctgtat 240

ctgcaaatga acagcctgag agccgaggac acggctgtgt attactgtgc gagaga 296

<210> 361
<211> 296
<212> DNA
<213> Artificial Sequence

<220>
<223> Synthetic DNA: IGHV3-30*05

<400> 361

caggtgcagc tggtggagtc tgggggaggc gtggtccagc ctgggaggtc cctgagactc 60

tcctgtgcag cctctggatt caccttcagt agctatggca tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa cacgctgtat 240

ctgcaaatga acagcctgag agctgagggc acggctgtgt attactgtgc gagaga 296

<210> 362
<211> 296
<212> DNA
<213> Artificial Sequence

<220>
<223> Synthetic DNA: IGHV3-30*13

<400> 362

caggtgcagc tggtggagtc tgggggaggc gtggtccagc ctgggaggtc cctgagactc 60

tcctgtgcag cctctggatt caccttcagt agctatggca tgcactgggt ccgccaggct 120

ccaggcaagg ggctagagtg ggtggcagtt atatcatatg atggaagtaa taaatactac 180

gcagactccg tgaagggccg attcaccatc tccagagaca attccaagaa caggctgtat 240

ctgcaaatga acagcctgag agctgaggac acggctgtgt attactgtgc gagaga 296

<210> 363
<211> 296
<212> DNA
<213> Artificial Sequence

<220>
<223> Synthetic DNA: IGHV3-33*01

<400> 363

caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatggtatg atggaagtaa taaatactat 180

gcagactccg tgaagggccg attcaccatc tccagagaca attccaagaa cacgctgtat 240

ctgcaaatga acagcctgag agccgaggac acggctgtgt attactgtgc gagaga 296

<210> 364
<211> 296
<212> DNA
<213> Artificial Sequence

<220>
<223> Synthetic DNA: IGHV3-33*04

<400> 364

caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctagagtg ggtggcagtt atatggtatg acggaagtaa taaatactat 180

gcagactccg tgaagggccg attcaccatc tccagagaca attccaagaa cacgctgtat 240

ctgcaaatga acagcctgag agccgaggac acggctgtgt attactgtgc gagaga 296

<210> 365
<211> 296
<212> DNA
<213> Artificial Sequence

<220>
<223> Synthetic DNA: IGHV3-33*02

<400> 365

caggtacagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatggtatg atggaagtaa taaatactat 180

gcagactccg cgaagggccg attcaccatc tccagagaca attccacgaa cacgctgttt 240

ctgcaaatga acagcctgag agccgaggac acggctgtgt attactgtgc gagaga 296

<210> 366
<211> 296
<212> DNA
<213> Artificial Sequence

<220>
<223> Synthetic DNA: IGHV3-33*03

<400> 366

caggtgcagc tggtggagtc tgggggaggc gtggtccagc ctgggaggtc cctgagactc 60

tcctgtgcag cgtctggatt caccttcagt agctatggca tgcactgggt ccgccaggct 120

ccaggcaagg ggctggagtg ggtggcagtt atatggtatg atggaagtaa taaatactat 180

gcagactccg tgaagggccg attcaccatc tccagagaca actccaagaa cacgctgtat 240

ctgcaaatga acagcctgag agccgaggac acggctgtgt attactgtgc gaaaga 296

<210> 367
<211> 296
<212> DNA
<213> Artificial Sequence

<220>
<223> Synthetic DNA: IGHV3-30*02

<400> 367

caggtgcagc tggtggagtc tgggggaggc gtggtccagc ctgggggGtc cctgagactc 60

tcctgtgcag cgtctggatt caccttcagt agctatggca tgcactgggt ccgccaggct 120

ccaggcaagg ggctggagtg ggtggcattt atacggtatg atggaagtaa taaatactat 180

gcagactccg tgaagggccg attcaccatc tccagagaca attccaagaa cacgctgtat 240

ctgcaaatga acagcctgag agctgaggac acggctgtgt attactgtgc gaaaga 296

<210> 368
<211> 296
<212> DNA
<213> Artificial Sequence

<220>
<223> Synthetic DNA: IGHV3-52*01

<400> 368

gaggtgcagc tggtggagtc tgggtgaggc ttggtacagc ctggagggtc cctgagactc 60

tcctgtgcag cctctggatt caccttcagt agctcctgga tgcactgggt ctgccaggct 120

ccggagaagg ggctggagtg ggtggccgac ataaagtgtg acggaagtga gaaatactat 180

gtagactctg tgaagggccg attgaccatc tccagagaca atgccaagaa ctccctctat 240

ctgcaagtga acagcctgag agctgaggac atgaccgtgt attactgtgt gagagg 296

<210> 369
<211> 294

<212> DNA
<213> Artificial Sequence

<220>
<223> Synthetic DNA: IGHV3-52*02

<400> 369

```
gaggtgcagc tggtggagtc tgggtgaggc ttggtacagc ctggagggtc cctgagactc      60
tcctgtgcag cctctggatt caccttcagt agctcctgga tgcactgggt ctgccaggct     120
ccggagaagg ggcaggagtg ggtggccgac ataaagtgtg acggaagtga gaaatactat     180
gtagactctg tgaagggccg attgaccatc tccagagaca atgccaagaa ctccctctat     240
ctgcaagtga acagcctgag agctgaggac atgaccgtgt attactgtgt gaga           294
```

<210> 370
<211> 294
<212> DNA
<213> Artificial Sequence

<220>
<223> Synthetic DNA: IGHV3-52*03

<400> 370

```
gaggtgcagc tggtcgagtc tgggtgaggc ttggtacagc ctggagggtc cctgagactc      60
tcctgtgcag cctctggatt caccttcagt agctcctgga tgcactgggt ctgccaggct     120
ccggagaagg ggctggagtg ggtggccgac ataaagtgtg acggaagtga gaaatactat     180
gtagactctg tgaagggccg attgaccatc tccagagaca atgccaagaa ctccctctat     240
ctgcaagtga acagcctgag agctgaggac atgaccgtgt attactgtgt gaga           294
```

<210> 371
<211> 296
<212> DNA
<213> Artificial Sequence

<220>
<223> Synthetic DNA: IGHV3-7*01

<400> 371

```
gaggtgcagc tggtggagtc tgggggaggc ttggtccagc ctggggggtc cctgagactc      60
tcctgtgcag cctctggatt cacctttagt agctattgga tgagctgggt ccgccaggct     120
ccagggaagg ggctggagtg ggtggccaac ataaagcaag atggaagtga gaaatactat     180
gtggactctg tgaagggccg attcaccatc tccagagaca acgccaagaa ctcactgtat     240
ctgcaaatga acagcctgag agccgaggac acggctgtgt attactgtgc gagaga         296
```

<210> 372
<211> 294
<212> DNA
<213> Artificial Sequence

<220>
<223> Synthetic DNA: IGHV3-7*02

<400> 372

gaggtgcagc tggtggagtc tgggggaggc ttggtccagc ctggggggtc cctgagactc 60

tcctgtgcag cctctggatt cacctttagt agctattgga tgagctgggt ccgccaggct 120

ccagggaaag ggctggagtg ggtggccaac ataaagcaag atggaagtga gaaatactat 180

gtggactctg tgaagggccg attcaccatc tccagagaca acgccaagaa ctcactgtat 240

ctgcaaatga acagcctgag agccgaggac acggctgtgt attactgtgc gaga 294

<210> 373
<211> 296
<212> DNA
<213> Artificial Sequence

<220>
<223> Synthetic DNA: IGHV3-23*01

<400> 373

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtat attactgtgc gaaaga 296

<210> 374
<211> 296
<212> DNA
<213> Artificial Sequence

<220>
<223> Synthetic DNA: IGHV3-23*04

<400> 374

gaggtgcagc tggtggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtat attactgtgc gaaaga 296

<210> 375
<211> 296
<212> DNA
<213> Artificial Sequence

<220>
<223> Synthetic DNA: IGHV3-23*02

<400> 375

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

ggagactccg tgaagggccg gttcaccatc tcaagagaca attccaagaa cacgctgtat 240

ctgcaaatga acagcctgag agccgaggac acggccgtat attactgtgc gaaaga 296

<210> 376
<211> 294
<212> DNA
<213> Artificial Sequence

<220>
<223> Synthetic DNA: IGHV3-23*03

<400> 376

gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagtt atttatagcg gtggtagtag cacatactat 180

gcagactccg tgaagggccg gttcaccatc tccagagata attccaagaa cacgctgtat 240

ctgcaaatga acagcctgag agccgaggac acggccgtat attactgtgc gaaa 294

<210> 377
<211> 294
<212> DNA
<213> Artificial Sequence

<220>
<223> Synthetic DNA: IGHV3-23*05

<400> 377

gaggtgcagc tgttggagtc tgggggaggc ttggtacagc ctggggggtc cctgagactc 60
tcctgtgcag cctctggatt cacctttagc agctatgcca tgagctgggt ccgccaggct 120
ccagggaagg ggctggagtg ggtctcagct atttatagca gtggtagtag cacatactat 180
gcagactccg tgaagggccg gttcaccatc tccagagaca attccaagaa cacgctgtat 240
ctgcaaatga acagcctgag agccgaggac acggccgtat attactgtgc gaaa 294

<210> 378
<211> 293
<212> DNA
<213> Artificial Sequence

<220>
<223> Synthetic DNA: IGHV3-53*01

<400> 378

gaggtgcagc tggtggagtc tggaggaggc ttgatccagc ctggggggtc cctgagactc 60
tcctgtgcag cctctgggtt caccgtcagt agcaactaca tgagctgggt ccgccaggct 120
ccagggaagg ggctggagtg ggtctcagtt atttatagcg gtggtagcac atactacgca 180
gactccgtga agggccgatt caccatctcc agagacaatt ccaagaacac gctgtatctt 240
caaatgaaca gcctgagagc cgaggacacg gccgtgtatt actgtgcgag aga 293

<210> 379
<211> 291
<212> DNA
<213> Artificial Sequence

<220>
<223> Synthetic DNA: IGHV3-53*02

<400> 379

gaggtgcagc tggtggagac tggaggaggc ttgatccagc ctggggggtc cctgagactc 60
tcctgtgcag cctctgggtt caccgtcagt agcaactaca tgagctgggt ccgccaggct 120
ccagggaagg ggctggagtg ggtctcagtt atttatagcg gtggtagcac atactacgca 180
gactccgtga agggccgatt caccatctcc agagacaatt ccaagaacac gctgtatctt 240
caaatgaaca gcctgagagc cgaggacacg gccgtgtatt actgtgcgag a 291

<210> 380
<211> 293
<212> DNA
<213> Artificial Sequence

<220>
<223> Synthetic DNA: IGHV3-66*03

<400> 380

gaggtgcagc tggtggagtc tggaggaggc ttgatccagc ctggggggtc cctgagactc 60

tcctgtgcag cctctgggtt caccgtcagt agcaactaca tgagctgggt ccgccaggct 120

ccagggaagg ggctggagtg ggtctcagtt atttatagct gtggtagcac atactacgca 180

gactccgtga agggccgatt caccatctcc agagacaatt ccaagaacac gctgtatctt 240

caaatgaaca gcctgagagc tgaggacacg gctgtgtatt actgtgcgag aga 293

<210> 381
<211> 293
<212> DNA
<213> Artificial Sequence

<220>
<223> Synthetic DNA: IGHV3-53*03

<400> 381

gaggtgcagc tggtggagtc tggaggaggc ttgatccagc ctggggggtc cctgagactc 60

tcctgtgcag cctctgggtt caccgtcagt agcaactaca tgagctgggt ccgccagcct 120

ccagggaagg ggctggagtg ggtctcagtt atttatagcg gtggtagcac atactacgca 180

gactctgtga agggccgatt caccatctcc agagacaatt ccaagaacac gctgtatctt 240

caaatgaaca gcctgagagc cgaggacacg gccgtgtatt actgtgctag gga 293

<210> 382
<211> 293
<212> DNA
<213> Artificial Sequence

<220>
<223> Synthetic DNA: IGHV3-66*01

<400> 382

gaggtgcagc tggtggagtc tgggggaggc ttggtccagc ctggggggtc cctgagactc 60

tcctgtgcag cctctggatt caccgtcagt agcaactaca tgagctgggt ccgccaggct 120

ccagggaagg ggctggagtg ggtctcagtt atttatagcg gtggtagcac atactacgca 180

gactccgtga agggcagatt caccatctcc agagacaatt ccaagaacac gctgtatctt 240

caaatgaaca gcctgagagc cgaggacacg gctgtgtatt actgtgcgag aga 293

<210> 383
<211> 293

<212> DNA
<213> Artificial Sequence

<220>
<223> Synthetic DNA: IGHV3-66*04

<400> 383

gaggtgcagc tggtggagtc tgggggaggc ttggtccagc ctggggggtc cctgagactc 60

tcctgtgcag cctctggatt caccgtcagt agcaactaca tgagctgggt ccgccaggct 120

ccagggaagg ggctggagtg ggtctcagtt atttatagcg gtggtagcac atactacgca 180

gactccgtga agggcagatt caccatctcc agagacaatt ccaagaacac gctgtatctt 240

caaatgaaca gcctgagagc cgaggacacg gctgtgtatt actgtgcgag aca 293

<210> 384
<211> 291
<212> DNA
<213> Artificial Sequence

<220>
<223> Synthetic DNA: IGHV3-66*02

<400> 384

gaggtgcagc tggtggagtc tgggggaggc ttggtccagc ctggggggtc cctgagactc 60

tcctgtgcag cctctggatt caccgtcagt agcaactaca tgagctgggt ccgccaggct 120

ccagggaagg ggctggagtg ggtctcagtt atttatagcg gtggtagcac atactacgca 180

gactccgtga agggccgatt caccatctcc agagacaatt ccaagaacac gctgtatctt 240

caaatgaaca gcctgagagc tgaggacacg gctgtgtatt actgtgcgag a 291

<210> 385
<211> 292
<212> DNA
<213> Artificial Sequence

<220>
<223> Synthetic DNA: IGHV3-38*01

<400> 385

gaggtgcagc tggtggagtc tgggggaggc ttggtacagc ctagggggtc cctgagactc 60

tcctgtgcag cctctggatt caccgtcagt agcaatgaga tgagctggat ccgccaggct 120

ccagggaagg ggctggagtg ggtctcatcc attagtggtg gtagcacata ctacgcagac 180

tccaggaagg gcagattcac catctccaga gacaattcca agaacacgct gtatcttcaa 240

atgaacaacc tgagagctga gggcacggcc gcgtattact gtgccagata ta 292

<210> 386
<211> 292
<212> DNA
<213> Artificial Sequence

<220>
<223> Synthetic DNA: IGHV3-38*02

<400> 386

gaggtgcagc tggtggagtc tgggggaggc ttggtacagc ctagggggtc cctgagactc 60

tcctgtgcag cctctggatt caccgtcagt agcaatgaga tgagctggat ccgccaggct 120

ccagggaagg ggctggagtg ggtctcatcc attagtggtg gtagcacata ctacgcagac 180

tccaggaagg gcagattcac catctccaga gacaattcca agaacacgct gtatcttcaa 240

atgaacaacc tgagagctga gggcacggcc gtgtattact gtgccagata ta 292

<210> 387
<211> 288
<212> DNA
<213> Artificial Sequence

<220>
<223> Synthetic DNA: IGHV3-d*01

<400> 387

gaggtgcagc tggtggagtc tcggggagtc ttggtacagc ctgggggtc cctgagactc 60

tcctgtgcag cctctggatt caccgtcagt agcaatgaga tgagctgggt ccgccaggct 120

ccagggaagg gtctggagtg ggtctcatcc attagtggtg gtagcacata ctacgcagac 180

tccaggaagg gcagattcac catctccaga gacaattcca agaacacgct gcatcttcaa 240

atgaacagcc tgagagctga ggacacggct gtgtattact gtaagaaa 288

<210> 388
<211> 294
<212> DNA
<213> Artificial Sequence

<220>
<223> Synthetic DNA: IGHV3/OR16-12*01

<400> 388

```
gaggtgcagc tggtagagtc tgggagaggc ttggcccagc ctggggggta cctaaaactc        60
tccggtgcag cctctggatt caccgtcggt agctggtaca tgagctggat ccaccaggct       120
ccagggaagg gtctggagtg ggtctcatac attagtagta gtggttgtag cacaaactac       180
gcagactctg tgaagggcag attcaccatc tccacagaca actcaaagaa cacgctctac       240
ctgcaaatga acagcctgag agtggaggac acggccgtgt attactgtgc aaga             294
```

<210> 389
<211> 302
<212> DNA
<213> Artificial Sequence

<220>
<223> Synthetic DNA: IGHV3-15*01

<400> 389

```
gaggtgcagc tggtggagtc tgggggaggc ttggtaaagc ctggggggtc ccttagactc        60
tcctgtgcag cctctggatt cactttcagt aacgcctgga tgagctgggt ccgccaggct       120
ccagggaagg ggctggagtg ggttggccgt attaaaagca aaactgatgg tgggacaaca       180
gactacgctg cacccgtgaa aggcagattc accatctcaa gagatgattc aaaaaacacg       240
ctgtatctgc aaatgaacag cctgaaaacc gaggacacag ccgtgtatta ctgtaccaca       300
ga                                                                      302
```

<210> 390
<211> 302
<212> DNA
<213> Artificial Sequence

<220>
<223> Synthetic DNA: IGHV3-15*02

<400> 390

```
gaggtgcagc tggtggagtc tgggggagcc ttggtaaagc ctggggggtc ccttagactc        60
tcctgtgcag cctctggatt cactttcagt aacgcctgga tgagctgggt ccgccaggct       120
ccagggaagg ggctggagtg ggttggccgt attaaaagca aaactgatgg tgggacaaca       180
gactacgctg cacccgtgaa aggcagattc accatctcaa gagatgattc aaaaaacacg       240
ctgtatctgc aaatgaacag cctgaaaacc gaggacacag ccgtgtatta ctgtaccaca       300
ga                                                                      302
```

<210> 391
<211> 302
<212> DNA
<213> Artificial Sequence

<220>
<223> Synthetic DNA: IGHV3-15*04

<400> 391

```
gaggtgcagc tggtggagtc tgggggaggc ttggtaaagc ctggggggtc ccttagactc      60
tcctgtgcag cctctggatt cactttcagt aacgcctgga tgagctgggt ccgccaggct     120
ccagggaagg ggctggagtg ggttggccgt attgaaagca aaactgatgg tgggacaaca     180
gactacgctg cacccgtgaa aggcagattc accatctcaa gagatgattc aaaaaacacg     240
ctgtatctgc aaatgaacag cctgaaaacc gaggacacag ccgtgtatta ctgtaccaca     300
ga                                                                    302
```

<210> 392
<211> 302
<212> DNA
<213> Artificial Sequence

<220>
<223> Synthetic DNA: IGHV3-15*05

<400> 392

```
gaggtgcagc tggtggagtc tgggggaggc ttggtaaagc ctggggggtc ccttagactc      60
tcctgtgcag cctctggatt cactttcagt aacgcctgga tgagctgggt ccgccaggct     120
ccagggaagg ggctggagtg ggttggccgt attaaaagca aaactgatgg tgggacaaca     180
gactacgctg cacccgtgaa aggcagattc accatctcaa gagatgattc aaaaaacacg     240
ctgtatctgc aaatgaacag tctgaaaacc gaggacacag ccgtgtatta ctgtaccaca     300
ga                                                                    302
```

<210> 393
<211> 302
<212> DNA
<213> Artificial Sequence

<220>
<223> Synthetic DNA: IGHV3-15*06

<400> 393

gaggtgcagc tggtggagtc tgggggaggc ttggtaaagc ctggggggtc ccttagactc 60

tcctgtgcag cctctggatt cactttcagt aacgcctgga tgagctgggt ccgccaggct 120

ccagggaagg ggctggagtg ggtcggccgt attaaaagca aaactgatgg tgggacaaca 180

aactacgctg cacccgtgaa aggcagattc accatctcaa gagatgattc aaaaaacacg 240

ctgtatctgc aaatgaacag cctgaaaacc gaggacacag ccgtgtatta ctgtaccaca 300

ga 302

<210> 394
<211> 302
<212> DNA
<213> Artificial Sequence

<220>
<223> Synthetic DNA: IGHV3-15*07

<400> 394

gaggtgcagc tggtggagtc tgggggaggc ttggtaaagc ctggggggtc ccttagactc 60

tcctgtgcag cctctggttt cactttcagt aacgcctgga tgaactgggt ccgccaggct 120

ccagggaagg ggctggagtg ggtcggccgt attaaaagca aaactgatgg tgggacaaca 180

gactacgctg cacccgtgaa aggcagattc accatctcaa gagatgattc aaaaaacacg 240

ctgtatctgc aaatgaacag cctgaaaacc gaggacacag ccgtgtatta ctgtaccaca 300

ga 302

<210> 395
<211> 302
<212> DNA
<213> Artificial Sequence

<220>
<223> Synthetic DNA: IGHV3-15*03

<400> 395

gaggtgcagc tggtggagtc tgccggagcc ttggtacagc ctggggggtc ccttagactc 60

tcctgtgcag cctctggatt cacttgcagt aacgcctgga tgagctgggt ccgccaggct 120

ccagggaagg ggctggagtg ggttggccgt attaaaagca aagctaatgg tgggacaaca 180

gactacgctg cacctgtgaa aggcagattc accatctcaa gagttgattc aaaaaacacg 240

ctgtatctgc aaatgaacag cctgaaaacc gaggacacag ccgtgtatta ctgtaccaca 300

ga 302

<210> 396
<211> 302

<212> DNA
<213> Artificial Sequence

<220>
<223> Synthetic DNA: IGHV3-15*08

<400> 396

gaggtgcagc tggtggagtc tgcgggaggc ttggtacagc ctggggggtc ccttagactc 60

tcctgtgcag cctctggatt cacttgcagt aacgcctgga tgagctgggt ccgccaggct 120

ccagggaagg ggctggagtg ggttggctgt attaaaagca aagctaatgg tgggacaaca 180

gactacgctg cacctgtgaa aggcagattc accatctcaa gagatgattc aaaaaacacg 240

ctgtatctgc aaatgatcag cctgaaaacc gaggacacgg ccgtgtatta ctgtaccaca 300

gg 302

<210> 397
<211> 302
<212> DNA
<213> Artificial Sequence

<220>
<223> Synthetic DNA: IGHV3-72*01

<400> 397

gaggtgcagc tggtggagtc tgggggaggc ttggtccagc ctggagggtc cctgagactc 60

tcctgtgcag cctctggatt caccttcagt gaccactaca tggactgggt ccgccaggct 120

ccagggaagg ggctggagtg ggttggccgt actagaaaca aagctaacag ttacaccaca 180

gaatacgccg cgtctgtgaa aggcagattc accatctcaa gagatgattc aaagaactca 240

ctgtatctgc aaatgaacag cctgaaaacc gaggacacgg ccgtgtatta ctgtgctaga 300

ga 302

<210> 398
<211> 165
<212> DNA
<213> Artificial Sequence

<220>
<223> Synthetic DNA: IGHV3-72*02

<400> 398

accttcagtg accactacat ggactgggtc cgccaggctc cagggaaggg gctggagtgg 60

gttggccgta ctagaaacaa agctaacagc tacaccacag aatacgccgc gtctgtgaaa 120

ggcagattca ccatctcaag agatgattca aagaactcac tgtat 165

<210> 399
<211> 300
<212> DNA
<213> Artificial Sequence

<220>
<223> Synthetic DNA: IGHV3/OR15-7*01

<400> 399

gaggtgcagc tggtggagtc tgggggaggc ttggtccagc ctggggggttc tctgagactc 60

tcatgtgcag cctctggatt caccttcagt gaccactaca tgagctgggt ccgccaggct 120

caagggaaag ggctagagtt ggtaggttta ataagaaaca aagctaacag ttacacgaca 180

gaatatgctg cgtctgtgaa aggcagactt accatctcaa gagaggattc aaagaacacg 240

atgtatctgc aaatgagcaa cctgaaaacc gaggacttgg ccgtgtatta ctgtgctaga 300

<210> 400
<211> 300
<212> DNA
<213> Artificial Sequence

<220>
<223> Synthetic DNA: IGHV3/OR15-7*03

<400> 400

gaggtgcagc tggtggagtc tgggggaggc ttggtccagc ctggggggttc tctgagactc 60

tcatgtgcag cctctggatt caccttcagt gaccactaca tgagctgggt ccgccaggct 120

caagggaaag ggctagagtt ggtaggttta ataagaaaca aagctaacag ttacacgaca 180

gaatatgctg cgtctgtgaa aggcagactt accatctcaa gagaggattc aaagaacacg 240

ctgtatctgc aaatgagcag cctgaaaacc gaggacttgg ccgtgtatta ctgtgctaga 300

<210> 401
<211> 300
<212> DNA
<213> Artificial Sequence

<220>
<223> Synthetic DNA: IGHV3/OR15-7*02

<400> 401

gaggtgcagc tgttggagtc tgggggaggc ttggtccagc ctggggggttc tctgagactc 60

tcatgtgctg cctctggatt caccttcagt gaccactaca tgagctgggt ccgccaggct 120

caagggaaag ggctagagtt ggtaggttta ataagaaaca aagctaacag ttacacgaca 180

gaatatgctg cgtctgtgaa aggcagactt accatctcaa gagaggattc aaagaacacg 240

ctgtatctgc aaatgagcag cctgaaaacc gaggacttgg ccgtgtatta ctgtgctaga 300

<210> 402
<211> 302
<212> DNA
<213> Artificial Sequence

<220>
<223> Synthetic DNA: IGHV3-73*01

<400> 402

gaggtgcagc tggtggagtc tgggggaggc ttggtccagc ctggggggtc cctgaaactc 60

tcctgtgcag cctctgggtt caccttcagt ggctctgcta tgcactgggt ccgccaggct 120

tccgggaaag ggctggagtg ggttggccgt attagaagca aagctaacag ttacgcgaca 180

gcatatgctg cgtcggtgaa aggcaggttc accatctcca gagatgattc aaagaacacg 240

gcgtatctgc aaatgaacag cctgaaaacc gaggacacgg ccgtgtatta ctgtactaga 300

ca 302

<210> 403
<211> 302
<212> DNA
<213> Artificial Sequence

<220>
<223> Synthetic DNA: IGHV3-73*02

<400> 403

gaggtgcagc tggtggagtc cgggggaggc ttggtccagc ctggggggtc cctgaaactc 60

tcctgtgcag cctctgggtt caccttcagt ggctctgcta tgcactgggt ccgccaggct 120

tccgggaaag ggctggagtg ggttggccgt attagaagca aagctaacag ttacgcgaca 180

gcatatgctg cgtcggtgaa aggcaggttc accatctcca gagatgattc aaagaacacg 240

gcgtatctgc aaatgaacag cctgaaaacc gaggacacgg ccgtgtatta ctgtactaga 300

ca 302

<210> 404
<211> 302
<212> DNA
<213> Artificial Sequence

<220>
<223> Synthetic DNA: IGHV3-22*01

<400> 404

gaggtgcatc tggtggagtc tgggggagcc ttggtacagc ctggggggtc cctgagactc 60
tcctgtgcag cctctggatt caccttcagt tactactaca tgagcggggt ccgccaggct 120
cccgggaagg ggctggaatg ggtaggtttc attagaaaca aagctaatgg tgggacaaca 180
gaatagacca cgtctgtgaa aggcagattc acaatctcaa gagatgattc caaaagcatc 240
acctatctgc aaatgaagag cctgaaaacc gaggacacgg ccgtgtatta ctgttccaga 300
ga 302

<210> 405
<211> 302
<212> DNA
<213> Artificial Sequence

<220>
<223> Synthetic DNA: IGHV3-22*02

<400> 405

gaggtgcagc tggtggagtc tgggggaggc ttggtacagc ctggggggtc cctgagactc 60
tcctgtgcag cctctggatt caccttcagt tactactaca tgagcggggt ccgccaggct 120
cccgggaagg ggctggaatg ggtaggtttc attagaaaca aagctaatgg tgggacaaca 180
gaatagacca cgtctgtgaa aggcagattc acaatctcaa gagatgattc caaaagcatc 240
acctatctgc aaatgaagag cctgaaaacc gaggacacgg ccgtgtatta ctgttccaga 300
ga 302

<210> 406
<211> 302
<212> DNA
<213> Artificial Sequence

<220>
<223> Synthetic DNA: IGHV3-71*01

<400> 406

gaggtgcagc tggtggagtc cgggggaggc ttggtccagc ctggggggtc cctgagactc 60
tcctgtgcag cctctggatt caccttcagt gactactaca tgagctgggt ccgccaggct 120

cccgggaagg ggctggagtg ggtaggtttc attagaaaca aagctaatgg tgggacaaca 180

gaatagacca cgtctgtgaa aggcagattc acaatctcaa gagatgattc caaaagcatc 240

acctatctgc aaatgaacag cctgagagcc gaggacacgg ccgtgtatta ctgtgcgaga 300

ga 302

<210> 407
<211> 302
<212> DNA
<213> Artificial Sequence

<220>
<223> Synthetic DNA: IGHV3-49*03

<400> 407

gaggtgcagc tggtggagtc tgggggaggc ttggtacagc cagggcggtc cctgagactc 60

tcctgtacag cttctggatt cacctttggt gattatgcta tgagctggtt ccgccaggct 120

ccagggaagg ggctggagtg ggtaggtttc attagaagca aagcttatgg tgggacaaca 180

gaatacgccg cgtctgtgaa aggcagattc accatctcaa gagatgattc caaaagcatc 240

gcctatctgc aaatgaacag cctgaaaacc gaggacacag ccgtgtatta ctgtactaga 300

ga 302

<210> 408
<211> 302
<212> DNA
<213> Artificial Sequence

<220>
<223> Synthetic DNA: IGHV3-49*05

<400> 408

gaggtgcagc tggtggagtc tgggggaggc ttggtaaagc cagggcggtc cctgagactc 60

tcctgtacag cttctggatt cacctttggt gattatgcta tgagctggtt ccgccaggct 120

ccagggaagg ggctggagtg ggtaggtttc attagaagca aagcttatgg tgggacaaca 180

gaatacgccg cgtctgtgaa aggcagattc accatctcaa gagatgattc caaaagcatc 240

gcctatctgc aaatgaacag cctgaaaacc gaggacacag ccgtgtatta ctgtactaga 300

ga 302

<210> 409
<211> 302
<212> DNA
<213> Artificial Sequence

<220>

<223> Synthetic DNA: |IGHV3-49*01

<400> 409

gaggtgcagc tggtggagtc tgggggaggc ttggtacagc cagggcggtc cctgagactc 60

tcctgtacag cttctggatt cacctttggt gattatgcta tgagctggtt ccgccaggct 120

ccagggaagg ggctggagtg ggtaggtttc attagaagca aagcttatgg tgggacaaca 180

gaatacaccg cgtctgtgaa aggcagattc accatctcaa gagatggttc caaaagcatc 240

gcctatctgc aaatgaacag cctgaaaacc gaggacacag ccgtgtatta ctgtactaga 300

ga 302

<210> 410
<211> 302
<212> DNA
<213> Artificial Sequence

<220>
<223> Synthetic DNA: IGHV3-49*04

<400> 410

gaggtgcagc tggtggagtc tgggggaggc ttggtacagc cagggcggtc cctgagactc 60

tcctgtacag cttctggatt cacctttggt gattatgcta tgagctgggt ccgccaggct 120

ccagggaagg ggctggagtg ggtaggtttc attagaagca aagcttatgg tgggacaaca 180

gaatacgccg cgtctgtgaa aggcagattc accatctcaa gagatgattc caaaagcatc 240

gcctatctgc aaatgaacag cctgaaaacc gaggacacag ccgtgtatta ctgtactaga 300

ga 302

<210> 411
<211> 302
<212> **DNA**
<213> Artificial Sequence

<220>
<223> Synthetic DNA: IGHV3-49*02

<400> 411

gaggtgcagc tggtggagtc tgggggaggc ttggtacagc cagggccgtc cctgagactc 60
tcctgtacag cttctggatt cacctttggg tattatccta tgagctgggt ccgccaggct 120
ccagggaagg ggctggagtg ggtaggtttc attagaagca aagcttatgg tgggacaaca 180
gaatacgccg cgtctgtgaa aggcagattc accatctcaa gagatgattc caaaagcatc 240
gcctatctgc aaatgaacag cctgaaaacc gaggacacag ccgtgtatta ctgtactaga 300
ga 302

<210> 412
<211> 296
<212> DNA
<213> Artificial Sequence

<220>
<223> Synthetic DNA: IGHV3-25*01

<400> 412

gagatgcagc tggtggagtc tgggggaggc ttgcaaaagc ctgcgtggtc cccgagactc 60
tcctgtgcag cctctcaatt caccttcagt agctactaca tgaactgtgt ccgccaggct 120
ccagggaatg ggctggagtt ggtttgacaa gttaatccta atgggggtag cacatacctc 180
atagactccg gtaaggaccg attcaatacc tccagagata acgccaagaa cacacttcat 240
ctgcaaatga acagcctgaa aaccgaggac acggccctct attagtgtac cagaga 296

<210> 413
<211> 296
<212> DNA
<213> Artificial Sequence

<220>
<223> Synthetic DNA: IGHV3-25*02

<400> 413

gagatgcagc tggtggagtc tgggggaggc ttggcaaagc ctgcgtggtc cccgagactc 60
tcctgtgcag cctctcaatt caccttcagt agctactaca tgaactgtgt ccgccaggct 120
ccagggaatg ggctggagtt ggtttgacaa gttaatccta atgggggtag cacatacctc 180
atagactccg gtaaggaccg attcaatacc tccagagata acgccaagaa cacacttcat 240
ctgcaaatga acagcctgaa aaccgaggac acggccctct attagtgtac cagaga 296

<210> 414
<211> 294
<212> DNA
<213> Artificial Sequence

<220>
<223> Synthetic DNA: IGHV3-25*03

<400> 414

gagatgcagc tggtggagtc tgggggaggc ttggcaaagc ctgcgtggtc cccgagactc 60
tcctgtgcag cctctcaatt caccttcagt agctactaca tgaactgtgt ccgccaggct 120
ccagggaatg ggctggagtt ggttggacaa gttaatccta atgggggtag cacatacctc 180
atagactccg gtaaggaccg attcaatacc tccagagata acgccaagaa cacacttcat 240
ctgcaaatga acagcctgaa aaccgaggac acggccctgt attagtgtac caga 294

<210> 415
<211> 298
<212> DNA
<213> Artificial Sequence

<220>
<223> Synthetic DNA: IGHV3-63*01

<400> 415

gaggtggagc tgatagagtc catagagggc ctgagacaac ttgggaagtt cctgagactc 60
tcctgtgtag cctctggatt caccttcagt agctactgaa tgagctgggt caatgagact 120
ctagggaagg ggctggaggg agtaatagat gtaaaatatg atggaagtca gatataccat 180
gcagactctg tgaagggcag attcaccatc tccaaagaca atgctaagaa ctcaccgtat 240
ctccaaacga acagtctgag agctgaggac atgaccatgc atggctgtac ataaggtt 298

<210> 416
<211> 294
<212> DNA
<213> Artificial Sequence

<220>
<223> Synthetic DNA: IGHV3-63*02

<400> 416

gaggtggagc tgatagagtc catagagggc ctgagacaac ttgggaagtt cctgagactc 60
tcctgtgtag cctctggatt caccttcagt agctactgaa tgagctgggt caatgagact 120
ctagggaagg ggctggaggg agtaatagat gtaaaatatg atggaagtca gatataccat 180
gcagactctg tgaagggcag attcaccatc tccaaagaca atgctaagaa ctcaccgtat 240
ctgcaaacga acagtctgag agctgaggac atgaccatgc atggctgtac ataa 294

<210> 417
<211> 298

<212> DNA
<213> Artificial Sequence

<220>
<223> Synthetic DNA: IGHV3-32*01

<400> 417

```
gaggtggagc tgatagagtc catagaggac ctgagacaac ctgggaagtt cctgagactc      60
tcctgtgtag cctctagatt cgccttcagt agcttctgaa tgagccgagt tcaccagtct     120
ccaggcaagg ggctggagtg agtaatagat ataaaagatg atggaagtca gatacaccat     180
gcagactctg tgaagggcag attctccatc tccaaagaca atgctaagaa ctctctgtat     240
ctgcaaatga acactcagag agctgaggac gtggccgtgt atggctatac ataaggtc       298
```

<210> 418
<211> 296
<212> DNA
<213> Artificial Sequence

<220>
<223> Synthetic DNA: IGHV3-54*01

<400> 418

```
gaggtacagc tggtggagtc tgaagaaaac caaagacaac ttgggggatc cctgagactc      60
tcctgtgcag actctggatt aaccttcagt agctactgaa tgagctcaga ttcccaagct     120
ccagggaagg ggctggagtg agtagtagat atatagtagg atagaagtca gctatgttat     180
gcacaatctg tgaagagcag attcaccatc tccaaagaaa atgccaagaa ctcactctgt     240
ttgcaaatga acagtctgag agcagagggc acggccgtgt attactgtat gtgagy         296
```

<210> 419
<211> 296
<212> DNA
<213> Artificial Sequence

<220>
<223> Synthetic DNA: IGHV3-54*04

<400> 419

```
gaggtacagc tggtggagtc tgaagaaaac caaagacaac ttgggggatc cctgagactc      60
tcctgtgcag actctggatt aaccttcagt agctactgaa tgagctcaga ttcccaggct     120
ccagggaagg ggctggagtg agtagtagat atatagtagg atagaagtca gctatgttat     180
gcacaatctg tgaagagcag attcaccatc tccaaagaaa atgccaagaa ctcactctgt     240
ttgcaaatga acagtctgag agcagagggc acggccgtgt attactgtat gtgagt         296
```

<210> 420
<211> 207
<212> DNA
<213> Artificial Sequence

<220>
<223> Synthetic DNA: IGHV3-54*02

<400> 420

tagctactga atgagctcag attcccaggc tccagggaag ggctggagt gagtagtaga 60

tatatagtac gatagaagtc agatatgtta tgcacaatct gtgaagagca gattcaccat 120

ctccaaagaa aatgccaaga actcactccg tttgcaaatg aacagtctga gagcagaggg 180

cacggccgtg tattactgta tgtgagg 207

<210> 421
<211> 31
<212> DNA
<213> Artificial Sequence

<220>
<223> Synthetic DNA: >IGHJ4_1

<400> 421
tgaggagacg gtgaccaggg ttccttggcc c 31

<210> 422
<211> 31
<212> DNA
<213> Artificial Sequence

<220>
<223> Synthetic DNA: >IGHJ4_3

<400> 422
tgaggagacg gtgaccaggg tcccttggcc c 31

<210> 423
<211> 31
<212> DNA
<213> Artificial Sequence

<220>
<223> Synthetic DNA: >IGHJ4_2

<400> 423
tgaggagacg gtgaccaggg ttccctggcc c 31

<210> 424
<211> 32
<212> DNA
<213> Artificial Sequence

<220>
<223> Synthetic DNA: >IGHJ3_12

<400> 424
ctgaagagac ggtgaccatt gtcccttggc cc 32

<210> 425
<211> 32
<212> DNA
<213> Artificial Sequence

<220>
<223> Synthetic DNA: >IGHJ6_1

<400> 425
ctgaggagac ggtgaccgtg gtcccttgcc cc 32

<210> 426
<211> 31
<212> DNA
<213> Artificial Sequence

<220>
<223> Synthetic DNA: >IGHJ6_2

<400> 426
tgaggagacg gtgaccgtgg tcccttggcc c 31

<210> 427
<211> 32
<212> DNA
<213> Artificial Sequence

<220>
<223> Synthetic DNA: >IGHJ6_34

<400> 427
ctgaggagac ggtgaccgtg gtccctttgc cc 32

<210> 428
<211> 32
<212> DNA
<213> Artificial Sequence

<220>
<223> Synthetic DNA: >IGHJ2_1

<400> 428
ctgaggagac agtgaccagg gtgccacggc cc 32

<210> 429
<211> 32
<212> DNA
<213> Artificial Sequence

<220>
<223> Synthetic DNA: >IGHJ5_1

<400> 429
ctgaggagac ggtgaccagg gttccttggc cc 32

<210> 430
<211> 32
<212> DNA
<213> Artificial Sequence

<220>
<223> Synthetic DNA: >IGHJ5_2

<400> 430
ctgaggagac ggtgaccagg gttccctggc cc 32

<210> 431
<211> 32
<212> DNA
<213> Artificial Sequence

<220>
<223> Synthetic DNA: >IGHJ1_1

<400> 431
ctgaggagac ggtgaccagg gtgccctggc cc 32

<210> 432
<211> 34
<212> DNA
<213> Artificial Sequence

<220>
<223> Synthetic DNA: >IGHJSEQ4_1

<400> 432
tgaggagacg gtgaccaggg ttccttggcc ccag 34

<210> 433
<211> 34
<212> DNA
<213> Artificial Sequence

<220>
<223> Synthetic DNA: >IGHJSEQ4_3

<400> 433
tgaggagacg gtgaccaggg tcccttggcc ccag 34

<210> 434
<211> 34
<212> DNA
<213> Artificial Sequence

<220>
<223> Synthetic DNA: >IGHJSEQ4_2

<400> 434
tgaggagacg gtgaccaggg ttccctggcc ccag 34

<210> 435
<211> 35
<212> DNA

<213> Artificial Sequence

<220>
<223> Synthetic DNA: >IGHJSEQ3_12

<400> 435
ctgaagagac ggtgaccatt gtcccttggc cccag 35

<210> 436
<211> 35
<212> DNA
<213> Artificial Sequence

<220>
<223> Synthetic DNA: >IGHJSEQ6_1

<400> 436
ctgaggagac ggtgaccgtg gtcccttgcc cccag 35

<210> 437
<211> 34
<212> DNA
<213> Artificial Sequence

<220>
<223> Synthetic DNA: >IGHJSEQ6_2

<400> 437
tgaggagacg gtgaccgtgg tcccttggcc ccag 34

<210> 438
<211> 35
<212> DNA
<213> Artificial Sequence

<220>
<223> Synthetic DNA: >IGHJSEQ6_34

<400> 438
ctgaggagac ggtgaccgtg gtccctttgc cccag 35

<210> 439
<211> 35
<212> DNA
<213> Artificial Sequence

<220>
<223> Synthetic DNA: >IGHJSEQ2_1

<400> 439
ctgaggagac agtgaccagg gtgccacggc cccag 35

<210> 440
<211> 35
<212> DNA
<213> Artificial Sequence

<220>

<223> Synthetic DNA: >IGHJSEQ5_1

<400> 440
ctgaggagac ggtgaccagg gttccttggc cccag 35

<210> 441
<211> 35
<212> DNA
<213> Artificial Sequence

<220>
<223> Synthetic DNA: >IGHJSEQ5_2

<400> 441
ctgaggagac ggtgaccagg gttccctggc cccag 35

<210> 442
<211> 35
<212> DNA
<213> Artificial Sequence

<220>
<223> Synthetic DNA: >IGHJSEQ1_1

<400> 442
ctgaggagac ggtgaccagg gtgccctggc cccag 35

<210> 443
<211> 36
<212> DNA
<213> Artificial Sequence

<220>
<223> Synthetic DNA: >IGHV1

<220>
<221> misc_feature
<222> (18)..(18)
<223> n is a, c, g, or t

<220>
<221> misc_feature
<222> (20)..(20)
<223> n is a, c, g, or t

<400> 443
tgggtgcacc aggtccangn acaagggctt gagtgg 36

<210> 444
<211> 36
<212> DNA
<213> Artificial Sequence

<220>
<223> Synthetic DNA: >IGHV2

<220>
<221> misc_feature

<222> (18)..(18)
<223> n is a, c, g, or t

<220>
<221> misc_feature
<222> (20)..(20)
<223> n is a, c, g, or t

<400> 444
tgggtgcgac aggctcgngn acaacgcctt gagtgg 36

<210> 445
<211> 36
<212> DNA
<213> Artificial Sequence

<220>
<223> Synthetic DNA: >IGHV3

<220>
<221> misc_feature
<222> (18)..(18)
<223> n is a, c, g, or t

<220>
<221> misc_feature
<222> (20)..(20)
<223> n is a, c, g, or t

<400> 445
tgggtgcgcc agatgccngn gaaaggcctg gagtgg 36

<210> 446
<211> 36
<212> DNA
<213> Artificial Sequence

<220>
<223> Synthetic DNA: >IGHV4

<220>
<221> misc_feature
<222> (18)..(18)
<223> n is a, c, g, or t

<220>
<221> misc_feature
<222> (20)..(20)
<223> n is a, c, g, or t

<400> 446
tgggtccgcc agscyccngn gaaggggctg gagtgg 36

<210> 447
<211> 36
<212> DNA
<213> Artificial Sequence

<220>
<223> Synthetic DNA: >IGHV5

<220>
<221> misc_feature
<222> (18)..(18)
<223> n is a, c, g, or t

<220>
<221> misc_feature
<222> (20)..(20)
<223> n is a, c, g, or t

<400> 447
tgggtccgcc aggctccngn aaaggggctg gagtgg 36

<210> 448
<211> 36
<212> DNA
<213> Artificial Sequence

<220>
<223> Synthetic DNA: >IGHV6

<220>
<221> misc_feature
<222> (18)..(18)
<223> n is a, c, g, or t

<220>
<221> misc_feature
<222> (20)..(20)
<223> n is a, c, g, or t

<400> 448
tgggtctgcc aggctccngn gaaggggcag gagtgg 36

<210> 449
<211> 37
<212> DNA
<213> Artificial Sequence

<220>
<223> Synthetic DNA: >IGH7_3.25p

<400> 449
tgtgtccgcc aggctccagg gaatgggctg gagttgg 37

<210> 450
<211> 37
<212> DNA
<213> Artificial Sequence

<220>
<223> Synthetic DNA: >IGH8_3.54p

<400> 450
tcagattccc aagctccagg gaaggggctg gagtgag 37

<210> 451
<211> 37
<212> DNA
<213> Artificial Sequence

<220>
<223> Synthetic DNA: >IGH9_3.63p

<400> 451
tgggtcaatg agactctagg gaaggggctg gagggag 37

<210> 452
<211> 48
<212> DNA
<213> Artificial Sequence

<220>
<223> Synthetic DNA: >IGHJ4*01/1-48

<400> 452
actactttga ctactggggc caaggaaccc tggtcaccgt ctcctcag 48

<210> 453
<211> 48
<212> DNA
<213> Artificial Sequence

<220>
<223> Synthetic DNA: >IGHJ4*03/1-48

<400> 453
gctactttga ctactggggc caagggaccc tggtcaccgt ctcctcag 48

<210> 454
<211> 48
<212> DNA
<213> Artificial Sequence

<220>
<223> Synthetic DNA: >IGHJ4*02/1-48

<400> 454
actactttga ctactggggc cagggaaccc tggtcaccgt ctcctcag 48

<210> 455
<211> 50
<212> DNA
<213> Artificial Sequence

<220>
<223> Synthetic DNA: >IGHJ3*01/1-50

<400> 455
tgatgctttt gatgtctggg gccaagggac aatggtcacc gtctcttcag 50

<210> 456
<211> 50
<212> DNA

<213> Artificial Sequence

<220>
<223> Synthetic DNA: >IGHJ3*02/1-50

<400> 456
tgatgctttt gatatctggg gccaagggac aatggtcacc gtctcttcag 50

<210> 457
<211> 63
<212> DNA
<213> Artificial Sequence

<220>
<223> Synthetic DNA: >IGHJ6*01/1-63

<400> 457

attactacta ctactacggt atggacgtct gggggcaagg gaccacggtc accgtctcct 60

cag 63

<210> 458
<211> 63
<212> DNA
<213> Artificial Sequence

<220>
<223> Synthetic DNA: >IGHJ6*02/1-62

<400> 458

attactacta ctactacggt atggacgtct ggggccaagg gaccacggtc accgtctcct 60

cag 63

<210> 459
<211> 63
<212> DNA
<213> Artificial Sequence

<220>
<223> Synthetic DNA: >IGHJ6*04/1-63

<400> 459

attactacta ctactacggt atggacgtct ggggcaaagg gaccacggtc accgtctcct 60

cag 63

<210> 460
<211> 63
<212> DNA
<213> Artificial Sequence

<220>
<223> Synthetic DNA: >IGHJ6*03/1-62

<400> 460

**attactacta ctactactac atggacgtct ggggcaaagg gaccacggtc accgtctcct 60**

**cag 63**

<210> 461
<211> 53
<212> DNA
<213> Artificial Sequence

<220>
<223> Synthetic DNA: >IGHJ2*01/1-53

<400> 461
ctactggtac ttcgatctct ggggccgtgg caccctggtc actgtctcct cag 53

<210> 462
<211> 51
<212> DNA
<213> Artificial Sequence

<220>
<223> Synthetic DNA: >IGHJ5*01/1-51

<400> 462
acaactggtt cgactcctgg ggccaaggaa ccctggtcac cgtctcctca g 51

<210> 463
<211> 51
<212> DNA
<213> Artificial Sequence

<220>
<223> Synthetic DNA: >IGHJ5*02/1-51

<400> 463
acaactggtt cgacccctgg ggccagggaa ccctggtcac cgtctcctca g 51

<210> 464
<211> 52
<212> DNA
<213> Artificial Sequence

<220>
<223> Synthetic DNA: >IGHJ1*01/1-52

<400> 464
gctgaatact tccagcactg gggccagggc accctggtca ccgtctcctc ag 52

<210> 465
<211> 61
<212> DNA
<213> Artificial Sequence

<220>
<223> Synthetic DNA: >IGHJ2P*01/1-61

<400> 465

ctacaagtgc ttggagcact ggggcagggc agcccggaca ccgtctccct gggaacgtca 60

g 61

<210> 466
<211> 54
<212> DNA
<213> Artificial Sequence

<220>
<223> Synthetic DNA: >IGHJ1P*01/1-54

<400> 466
aaaggtgctg ggggtcccct gaacccgacc cgccctgaga ccgcagccac atca 54

<210> 467
<211> 52
<212> DNA
<213> Artificial Sequence

<220>
<223> Synthetic DNA: >IGHJ3P*01/1-52

<400> 467
cttgcggttg gacttcccag ccgacagtgg tggtctggct tctgaggggt ca 52

<210> 468
<211> 58
<212> DNA
<213> Artificial Sequence

<220>
<223> Synthetic DNA: TRBJ1-2

<400> 468
aatgatacgg cgaccaccga gatctaccta caacggttaa cctggtcccc gaaccgaa 58

<210> 469
<211> 284
<212> DNA
<213> Artificial Sequence

<220>
<223> Synthetic DNA: TRBV2*02

<400> 469

```
gaacctgaag tcacccagac tcccagccat caggtcacac agatgggaca ggaagtgatc        60
ttgcactgtg tccccatctc taatcactta tacttctatt ggtacagaca aatcttgggg       120
cagaaagtcg agtttctggt ttccttttat aataatgaaa tctcagagaa gtctgaaata       180
ttcgatgatc aattctcagt tgaaaggcct gatggatcaa atttcactct gaagatccgg       240
tccacaaagc tggaggactc agccatgtac ttctgtgcca gcag                        284
```

<210> 470
<211> 33
<212> DNA
<213> Artificial Sequence

<220>
<223> Synthetic DNA: Jseq 1-1

<400> 470
acaactgtga gtctggtgcc ttgtccaaag aaa 33

<210> 471
<211> 33
<212> DNA
<213> Artificial Sequence

<220>
<223> Synthetic DNA: Jseq 1-2

<400> 471
acaacggtta acctggtccc cgaaccgaag gtg 33

<210> 472
<211> 33
<212> DNA
<213> Artificial Sequence

<220>
<223> Synthetic DNA: Jseq 1-3

<400> 472
acaacagtga gccaacttcc ctctccaaaa tat 33

<210> 473
<211> 33
<212> DNA
<213> Artificial Sequence

<220>
<223> Synthetic DNA: Jseq 1-4

<400> 473
aagacagaga gctgggttcc actgccaaaa aac 33

<210> 474
<211> 33
<212> DNA
<213> Artificial Sequence

<220>
<223> Synthetic DNA: Jseq 1-5

<400> 474
aggatggaga gtcgagtccc atcaccaaaa tgc 33

<210> 475
<211> 33
<212> DNA
<213> Artificial Sequence

<220>
<223> Synthetic DNA: Jseq 1-6

<400> 475
gtcacagtga gcctggtccc gttcccaaag tgg 33

<210> 476
<211> 33
<212> DNA
<213> Artificial Sequence

<220>
<223> Synthetic DNA: Jseq 2-1

<400> 476
agcacggtga gccgtgtccc tggcccgaag aac 33

<210> 477
<211> 33
<212> DNA
<213> Artificial Sequence

<220>
<223> Synthetic DNA: Jseq 2-2

<400> 477
agtacggtca gcctagagcc ttctccaaaa aac 33

<210> 478
<211> 33
<212> DNA
<213> Artificial Sequence

<220>
<223> Synthetic DNA: Jseq 2-3

<400> 478
agcactgtca gccgggtgcc tgggccaaaa tac 33

<210> 479
<211> 33
<212> DNA
<213> Artificial Sequence

<220>
<223> Synthetic DNA: Jseq 2-4

<400> 479
agcactgaga gccgggtccc ggcgccgaag tac 33

<210> 480
<211> 33
<212> DNA
<213> Artificial Sequence

<220>
<223> Synthetic DNA: Jseq 2-5

<400> 480
agcaccagga gccgcgtgcc tggcccgaag tac 33

<210> 481
<211> 33
<212> DNA
<213> Artificial Sequence

<220>
<223> Synthetic DNA: Jseq 2-6

<400> 481
agcacggtca gcctgctgcc ggccccgaaa gtc 33

<210> 482
<211> 33
<212> DNA
<213> Artificial Sequence

<220>
<223> Synthetic DNA: Jseq 2-7

<400> 482
gtgaccgtga gcctggtgcc cggcccgaag tac 33

<210> 483
<211> 34
<212> DNA
<213> Artificial Sequence

<220>
<223> Synthetic DNA: TRBJ1-5

<220>
<221> misc_feature
<222> (1)..(1)
<223> n is a, c, g, or t; 5 end modified with universal reverse primer

<400> 483
nacctaggat ggagagtcga gtcccatcac caaa 34

<210> 484
<211> 58
<212> DNA
<213> Artificial Sequence

<220>

<223> Synthetic DNA: TRBJ1-5

<400> 484
aatgatacgg cgaccaccga gatctaccta ggatggagag tcgagtccca tcaccaaa 58

## Description

TECHNICAL FIELD

What is described is a method to measure the adaptive immunity of a patient by analyzing the diversity of T cell receptor genes or antibody genes using large scale sequencing of nucleic acid extracted from adaptive immune system cells.

BACKGROUND

Immunocompetence is the ability of the body to produce a normal immune response (i.e., antibody production and/or cell-mediated immunity) following exposure to a pathogen, which might be a live organism (such as a bacterium or fungus), a virus, or specific antigenic components isolated from a pathogen and introduced in a vaccine. Immunocompetence is the opposite of immunodeficiency or immuno-incompetent or immunocompromised. Several examples would be a newborn that does not yet have a fully functioning immune system but may have maternally transmitted antibody (immunodeficient); a late stage AIDS patient with a failed or failing immune system (immuno-incompetent); a transplant recipient taking medication so their body will not reject the donated organ (immunocompromised); age-related attenuation of T cell function in the elderly; or individuals exposed to radiation or chemotherapeutic drugs. There may be cases of overlap but these terms are all indicators of a dysfunctional immune system. In reference to lymphocytes, immunocompetence means that a B cell or T cell is mature and can recognize antigens and allow a person to mount an immune response.

Immunocompetence depends on the ability of the adaptive immune system to mount an immune response specific for any potential foreign antigens, using the highly polymorphic receptors encoded by B cells (immunoglobulins, Igs) and T cells (T cell receptors, TCRs).

Igs expressed by B cells are proteins consisting of four polypeptide chains, two heavy chains (H chains) and two light chains (L chains), forming an $H_2L_2$ structure. Each pair of H and L chains contains a hypervariable domain, consisting of a $V_L$ and a $V_H$ region, and a constant domain. The H chains of Igs are of several types, $\mu$, $\delta$, $\gamma$, $\alpha$, and $\beta$. The diversity of Igs within an individual is mainly determined by the hypervariable domain. The V domain of H chains is created by the combinatorial joining of three types of germline gene segments, the $V_H$, $D_H$, and $J_H$ segments. Hypervariable domain sequence diversity is further increased by independent addition and deletion of nucleotides at the $V_H$-$D_H$, $D_H$-$J_H$, and $V_H$- $J_H$ junctions during the process of Ig gene rearrangement. In this respect, immunocompetence is reflected in the diversity of Igs.

TCRs expressed by $\alpha\beta$ T cells are proteins consisting of two transmembrane polypeptide chains ($\alpha$ and $\beta$), expressed from the TCRA and TCRB genes, respectively. Similar TCR proteins are expressed in gamma-delta T cells, from the TCRD and TCRG loci. Each TCR peptide contains variable complementarity determining regions (CDRs), as well as framework regions (FRs) and a constant region. The sequence diversity of $\alpha\beta$ T cells is largely determined by the amino acid sequence of the third complementarity-determining region (CDR3) loops of the $\alpha$ and $\beta$ chain variable domains, which diversity is a result of recombination between variable ($V_\beta$), diversity ($D_\beta$), and joining ($J_\beta$) gene segments in the $\beta$ chain locus, and between analogous $V_\alpha$ and $J_\alpha$ gene segments in the $\alpha$ chain locus, respectively. The existence of multiple such gene segments in the TCR $\alpha$ and $\beta$ chain loci allows for a large number of distinct CDR3 sequences to be encoded. CDR3 sequence diversity is further increased by independent addition and deletion of nucleotides at the $V_\beta$-$D_\beta$, $D_\beta$-$J_\beta$, and $V_\alpha$-$J_\alpha$ junctions during the process of TCR gene rearrangement. In this respect, immunocompetence is reflected in the diversity of TCRs.

There exists a long-felt need for methods of assessing or measuring the adaptive immune system of patients in a variety of settings, whether immunocompetence in the immunocompromised, or dysregulated adaptive immunity in autoimmune disease. A demand exists for methods of diagnosing a disease state or the effects of aging by assessing the immunocompetence of a patient. In the same way results of therapies that modify the immune system need to be monitored by assessing the immunocompetence of the patient while undergoing the treatment. Conversely, a demand exists for methods to monitor the adaptive immune system in the context of autoimmune disease flares and remissions, in order to monitor response to therapy, or the need to initiate prophylactic therapy pre-symptomatically.

Methods have been described for estimating TCR or Ig diversity without providing sequence information. These methods use primer sets amenable to determining CDR3 length polymorphism (Mariani et al., 2009 Exp Hematol. 37(6):728-738), qPCR of amplification products (EP 2062982), heteroduplex analysis or GeneScanning (Van Dongen et al., 2003 Leukemia. 17(12):2257-2317), and spectratyping (Maslanka et al., 1995 Hum Immunol. 44(1):28-34; Kiianitsa et al,. Blood. 2007 110(11):293B).

SUMMARY

The invention provides a composition comprising:

(a) V-segment primers comprising at least 45 forward primers consisting of the nucleotide sequences of SEQ ID Nos: 1-45, wherein each of the 45 forward primers is modified at the 5' end with a universal forward primer sequence compatible with a DNA sequencer; and
(b) J-segment primers comprising at least 13 reverse primers consisting of the nucleotide sequences of SEQ ID Nos: 46-57 and 483, wherein each of the 13 reverse primers is modified at the 5' end with a universal reverse primer sequence compatible with a DNA sequencer.

Described herein is a composition comprising:

- a multiplicity of V-segment primers, wherein each primer comprises a sequence that is complementary to a single functional V segment or a small family of V segments; and
- a multiplicity of J-segment primers, wherein each primer comprises a sequence that is complementary to a J segment;

wherein the V segment and J-segment primers permit amplification of a TCR CDR3 region by a multiplex polymerase chain reaction (PCR) to produce a multiplicity of amplified DNA molecules sufficient to quantify the diversity of the TCR genes. One instance of the disclosure is the composition, wherein each V-segment primer comprises a sequence that is complementary to a single Vβ segment, and each J segment primer comprises a sequence that is complementary to a Jβ segment, and wherein V segment and J-segment primers permit amplification of a TCRβ CDR3 region. Another instance is the composition, wherein each V-segment primer comprises a sequence that is complementary to a single functional Vα segment, and each J segment primer comprises a sequence that is complementary to a Jα segment, and wherein V segment and J-segment primers permit amplification of a TCRα CDR3 region.

Described herein is the composition, wherein the V segment primers hybridize with a conserved segment, and have similar annealing strength. Another instance is wherein the V segment primer is anchored at position -43 in the Vβ segment relative to the recombination signal sequence (RSS). Another instance is wherein the multiplicity of V segment primers consist of at least 45 primers specific to 45 different Vβ genes. Another embodiment is wherein the V segment primers have sequences that are selected from the group consisting of SEQ ID NOS:58-102. Another instance is wherein there is a V segment primer for each Vβ segment.

Described herein is the composition, wherein the J segment primers hybridize with a conserved framework region element of the Jβ segment, and have similar annealing strength. Another instance is wherein the multiplicity of J segment primers consist of at least thirteen primers specific to thirteen different Jβ genes. The J segment primers have sequences that are selected from the group consisting of SEQ ID NOS:46-57 and 483. Another embodiment is wherein the J segment primers have sequences that consist of SEQ ID NOS:103-113, 468 and 484. Another instance is wherein there is a J segment primer for each Jβ segment. Another instance is wherein all J segment primers anneal to the same conserved motif.

Another instance is the composition, wherein the amplified DNA molecule starts from said conserved motif and amplifies adequate sequence to diagnostically identify the J segment and includes the CDR3 junction and extends into the V segment. Another instance is wherein the amplified Jβ gene segments each have a unique four base tag at positions +11 through +14 downstream of the RSS site.

Another instance is the composition further comprising a set of sequencing oligonucleotides, wherein the sequencing oligonucleotides hybridize to regions within the amplified DNA molecules. An instance is wherein the sequencing oligonucleotides hybridize adjacent to a four base tag within the amplified Jβ gene segments at positions +11 through +14 downstream of the RSS site. Another embodiment is wherein the sequencing oligonucleotides are selected from the group consisting of SEQ ID NOS:58-70. Another instance is wherein the V-segment or J-segment are selected to contain a sequence error-correction by merger of closely related sequences. Another instance is the composition, further comprising a universal C segment primer for generating cDNA from mRNA.

Another instance is a composition comprising:

- a multiplicity of V segment primers, wherein each V segment primer comprises a sequence that is complementary to a single functional V segment or a small family of V segments; and
- a multiplicity of J segment primers, wherein each J segment primer comprises a sequence that is complementary to a J segment;

wherein the V segment and J segment primers permit amplification of the TCRG CDR3 region by a multiplex polymerase chain reaction (PCR) to produce a multiplicity of amplified DNA molecules sufficient to quantify the diversity of antibody heavy chain genes.

Another instance is a composition comprising:

- a multiplicity of V segment primers, wherein each V segment primer comprises a sequence that is complementary to a single functional V segment or a small family of V segments; and
- a multiplicity of J segment primers, wherein each J segment primer comprises a sequence that is complementary to a J segment;

wherein the V segment and J segment primers permit amplification of antibody heavy chain (IGH) CDR3 region by a multiplex polymerase chain reaction (PCR) to produce a multiplicity of amplified DNA molecules sufficient to quantify the diversity of antibody heavy chain genes.

Another instance is a composition comprising:

- a multiplicity of V segment primers, wherein each V segment primer comprises a sequence that is complementary to a single functional V segment or a small family of V segments; and
- a multiplicity of J segment primers, wherein each J segment primer comprises a sequence that is complementary to a J segment;

wherein the V segment and J segment primers permit amplification of antibody light chain (IGL) $V_L$ region by a multiplex polymerase chain reaction (PCR) to produce a multiplicity of amplified DNA molecules sufficient to quantify the diversity of antibody light chain genes.

The invention provides a method for quantifying TCRβ gene diversity in a sample, comprising:

(a) combining the composition as claimed herein with a sample of genomic DNA to permit amplification of rearranged TCRβ CDR3 regions from the sample, in a multiplex polymerase chain reaction (PCR), to produce a multiplicity of amplified DNA molecules that are sufficient to quantify TCRβ gene diversity in the sample by single molecule DNA sequencing, wherein each amplified DNA molecule includes adequate sequence to identify each V segment and each J segment by single molecule DNA sequencing; and optionally
(b) sequencing the amplified DNA molecules.

Described herein is a method comprising:

- selecting a multiplicity of V segment primers, wherein each V segment primer comprises a sequence that is complementary to a single functional V segment or a small family of V segments; and
- selecting a multiplicity of J segment primers, wherein each J segment primer comprises a sequence that is complementary to a J segment;
- combining the V segment and J segment primers with a sample of genomic DNA to permit amplification of a CDR3 region by a multiplex polymerase chain reaction (PCR) to produce a multiplicity of amplified DNA molecules sufficient to quantify the diversity of the TCR genes.

Described herein is the method wherein each V segment primer comprises a sequence that is complementary to a single functional Yβ segment, and each J segment primer comprises a sequence that is complementary to a Jβ segment; and wherein combining the V segment and J segment primers with a sample of genomic DNA permits amplification of a TCR CDR3 region by a multiplex polymerase chain reaction (PCR) and produces a multiplicity of amplified DNA molecules. Another instance is wherein each V segment primer comprises a sequence that is complementary to a single functional Vα segment, and each J segment primer comprises a sequence that is complementary to a Jα segment; and wherein combining the V segment and J segment primers with a sample of genomic DNA permits amplification of a TCR CDR3 region by a multiplex polymerase chain reaction (PCR) and produces a multiplicity of amplified DNA molecules.

The method comprises a step of sequencing the amplified DNA molecules. Another embodiment is wherein the sequencing step utilizes a set of sequencing oligonucleotides that hybridize to regions within the amplified DNA molecules. Another embodiment is the method, further comprising a step of calculating the total diversity of TCRjβ CDR3 sequences among the amplified DNA molecules. Another embodiment is wherein the method shows that the total diversity of a normal human subject is greater than $1*10^6$ sequences, greater than $2*10^6$ sequences, or greater than $3*10^6$ sequences.

Another aspect of the invention is a method of diagnosing immunodeficiency in a human patient, comprising measuring the diversity of TCR CDR3 sequences of the patient, and comparing the diversity of the subject to the diversity obtained from a normal subject. In the method, measuring the diversity of TCR sequences may comprise the steps of:

- selecting a multiplicity of V segment primers, wherein each V segment primer comprises a sequence that is complementary to a single functional V segment or a small family of V segments; and
- selecting a multiplicity of J segment primers, wherein each J segment primer comprises a sequence that is complementary to a J segment;
- combining the V segment and J segment primers with a sample of genomic DNA to permit amplification of a TCR CDR3 region by a multiplex polymerase chain reaction (PCR) to produce a multiplicity of amplified DNA molecules;
- sequencing the amplified DNA molecules;
- calculating the total diversity of TCR CDR3 sequences among the amplified DNA molecules.

An embodiment of the invention is the method, wherein comparing the diversity is determined by calculating using the following equation:

$$\Delta(t) = \sum_x E(n_x)\bigg|_{measurement\,1+2} - \sum_x E(n_x)\bigg|_{measurement\,2} = S\int_0^{\infty} e^{-\lambda}\left(1 - e^{-\lambda t}\right) dG(\lambda)$$

wherein G(λ) is the empirical distribution function of the parameters $\lambda_l, ..., \lambda_s$, $n_x$ is the number of clonotypes sequenced exactly x times, and

$$E(n_x) = S\int_0^{\infty}\left(\frac{e^{-\lambda}\lambda^x}{x!}\right) dG(\lambda)$$

Another embodiment of the invention is the method, wherein the diversity of at least two samples of genomic DNA are compared. Another embodiment is wherein one sample of genomic DNA is from a patient and the other sample is from a normal subject. Another embodiment is wherein one sample of genomic DNA is from a patient before a therapeutic treatment and the other sample is from the patient after treatment. Another embodiment is wherein the two samples of genomic DNA are from the same patient at different times during treatment. Another embodiment is wherein a disease is diagnosed based on the comparison of diversity among the samples of genomic DNA. Another embodiment is wherein the immunocompetence of a human patient is assessed by the comparison.

## DETAILED DESCRIPTION OF ILLUSTRATIVE EMBODIMENTS

The TCR and Ig genes can generate millions of distinct proteins via somatic mutation. Because of this diversity-generating mechanism, the hypervariable complementarity determining regions of these genes can encode sequences that can interact with millions of ligands, and these regions are linked to a constant region that can transmit a signal to the cell indicating binding of the protein's cognate ligand.

The adaptive immune system employs several strategies to generate a repertoire of T- and B-cell antigen receptors with sufficient diversity to recognize the universe of potential pathogens. In $\alpha\beta$ and γδ T cells, which primarily recognize peptide antigens presented by MHC molecules, most of this receptor diversity is contained within the third complementarity-determining region (CDR3) of the T cell receptor (TCR) $\alpha$ and $\beta$ chains (or $\gamma$ and δ chains). Although it has been estimated that the adaptive immune system can generate up to $10^{18}$ distinct TCR $\alpha\beta$ pairs, direct experimental assessment of TCR CDR3 diversity has not been possible.

What is described herein is a novel method of measuring TCR CDR3 diversity that is based on single molecule DNA sequencing, and use this approach to sequence the CDR3 regions in millions of rearranged TCR$\beta$ genes isolated from peripheral blood T cells of two healthy adults.

The ability of the adaptive immune system to mount an immune response specific for any of the vast number of potential foreign antigens to which an individual might be exposed relies on the highly polymorphic receptors encoded by B cells (immunoglobulins) and T cells (T cell receptors; TCRs). The TCRs expressed by $\alpha\beta$ T cells, which primarily recognize peptide antigens presented by major histocompatibility complex (MHC) class I and II molecules, are heterodimeric proteins consisting of two transmembrane polypeptide chains ($\alpha$ and $\beta$), each containing one variable and one constant domain. The peptide specificity of $\alpha\beta$ T cells is in large part determined by the amino acid sequence encoded in the third complementarity-determining region (CDR3) loops of the $\alpha$ and $\beta$ chain variable domains. The CDR3 regions of the $\beta$ and $\alpha$ chains are formed by recombination between noncontiguous variable ($V_\beta$), diversity ($D_\beta$), and joining ($J_\beta$) gene segments in the $\beta$ chain locus, and between analogous $V_\alpha$ and $J_\alpha$ gene segments in the $\alpha$ chain locus, respectively. The existence of multiple such gene segments in the TCR $\alpha$ and $\beta$ chain loci allows for a large number of distinct CDR3 sequences to be encoded. CDR3 sequence diversity is further increased by template-independent addition and deletion of nucleotides at the $V_\beta$-$D_\beta$, $D_\beta$-$J_\beta$, and $V_\alpha$-$J_\alpha$ junctions during the process of TCR gene rearrangement.

Previous attempts to assess the diversity of receptors in the adult human $\alpha\beta$ T cell repertoire relied on examining rearranged TCR $\alpha$ and $\beta$ chain genes expressed in small, well-defined subsets of the repertoire, followed by extrapolation of the diversity present in these subsets to the entire repertoire, to estimate approximately $10^6$ unique TCR$\beta$ chain CDR3 sequences per individual, with 10-20% of these unique TCR$\beta$ CDR3 sequences expressed by cells in the antigen-experienced CD45RO$^+$ compartment. The accuracy and precision of this estimate is severely limited by the need to extrapolate the diversity observed in hundreds of sequences to the entire repertoire, and it is possible that the actual number of unique TCR$\beta$ chain CDR3 sequences in the $\alpha\beta$ T cell repertoire is significantly larger than 1 x $10^6$.

Recent advances in high-throughput DNA sequencing technology have made possible significantly deeper sequencing than capillary-based technologies. A complex library of template molecules carrying universal PCR adapter sequences at each end is hybridized to a lawn of complementary oligonucleotides immobilized on a solid surface. Solid phase PCR is utilized to amplify the hybridized library, resulting in millions of template clusters on the surface, each comprising multiple (~1,000) identical copies of a single DNA molecule from the original library. A 30-54 bp interval in the molecules in each cluster is sequenced using reversible dye-termination chemistry, to permit simultaneous sequencing from genomic DNA of the rearranged TCR$\beta$ chain CDR3 regions carried in millions of T cells. This approach enables direct sequencing of a significant fraction of the uniquely rearranged TCR$\beta$ CDR3 regions in populations of $\alpha\beta$ T cells, which thereby permits estimation of the relative frequency of each CDR3 sequence in the population.

Accurate estimation of the diversity of TCR$\beta$ CDR3 sequences in the entire $\alpha\beta$ T cell repertoire from the diversity measured in a finite sample of T cells requires an estimate of the number of CDR3 sequences present in the repertoire that were not observed in the sample. TCR$\beta$ chain CDR3 diversity in the entire $\alpha\beta$ T cell repertoire were estimated using direct measurements of the number of unique TCR$\beta$ CDR3 sequences observed in blood samples containing millions of $\alpha\beta$ T cells. The results herein identify a lower bound for TCR$\beta$ CDR3 diversity in the CD4$^+$ and CD8$^+$ T cell compartments that is several fold higher than previous estimates. In addition, the results herein demonstrate that there are at least 1.5 x $10^6$ unique TCR$\beta$ CDR3 sequences in the CD45RO$^+$ compartment of antigen-experienced T-cells, a large proportion of which are present at low relative frequency. The existence of such a diverse population of TCR$\beta$ CDR3 sequences in antigen-experienced cells has not been previously demonstrated.

The diverse pool of TCR$\beta$ chains in each healthy individual is a sample from an estimated theoretical space of greater than $10^{11}$ possible sequences. However, the realized set of rearranged of TCRs is not evenly sampled from this theoretical space. Different V$\beta$'s and J$\beta$'s are found with over a thousand-fold frequency difference. Additionally, the insertion rates of nucleotides are strongly biased. This reduced space of realized TCR$\beta$ sequences leads to the possibility of shared $\beta$ chains between people. With the sequence data generated by the methods described herein, the in vivo J usage, V usage, mono- and di- nucleotide biases, and position dependent amino acid usage can be computed. These biases significantly narrow the size of the sequence space from which TCR$\beta$ are selected, suggesting that different individuals share TCR$\beta$ chains with identical amino acid sequences. Results herein show that many thousands of such identical sequences are shared pairwise between individual human genomes.

The assay technology uses two pools of primers to provide for a highly multiplexed PCR reaction. The "forward" pool has a primer specific to each V segment in the gene (several primers targeting a highly conserved region are used, to simultaneously capture many V segments). The "reverse" pool primers anneal to a conserved sequence in the joining ("J") segment. The amplified segment pool includes adequate sequence to identify each J segment and also to allow for a J-segment-specific primer to anneal for resequencing. This enables direct observation of a large fraction of the somatic rearrangements present in an individual. This in turn enables rapid comparison of the TCR repertoire in individuals with an autoimmune disorder (or other target disease indication) against the TCR repertoire of controls.

The adaptive immune system can in theory generate an enormous diversity of T cell receptor CDR3 sequences - far more than are likely to be expressed in any one individual at any one time. Previous attempts to measure what fraction of this theoretical diversity is actually utilized in the adult $\alpha\beta$ T cell repertoire, however, have not permitted accurate assessment of the diversity. What is described herein is the development of a novel approach to this question that is based on single molecule DNA sequencing and an analytic computational approach to estimation of repertoire diversity using diversity measurements in finite samples. The analysis demonstrated that the number of unique TCR$\beta$

CDR3 sequences in the adult repertoire significantly exceeds previous estimates based on exhaustive capillary sequencing of small segments of the repertoire. The TCRβ chain diversity in the $CD45RO^-$ population (enriched for naive T cells) observed using the methods described herein is five-fold larger than previously reported. A major discovery is the number of unique TCRβ CDR3 sequences expressed in antigen-experienced $CD45RO^+$ T cells - the results herein show that this number is between 10 and 20 times larger than expected based on previous results of others. The frequency distribution of CDR3 sequences in $CD45RO^+$ cells suggests that the T cell repertoire contains a large number of clones with a small clone size.

The results herein show that the realized set of TCR$\beta$ chains are sampled non-uniformly from the huge potential space of sequences. In particular, the $\beta$ chains sequences closer to germ line (few insertions and deletions at the V-D and D-J boundaries) appear to be created at a relatively high frequency. TCR sequences close to germ line are shared between different people because the germ line sequence for the V's, D's, and J's are shared, modulo a small number of polymorphisms, among the human population.

The T cell receptors expressed by mature αβ T cells are heterodimers whose two constituent chains are generated by independent rearrangement events of the TCR α and β chain variable loci. The $\alpha$ chain has less diversity than the $\beta$ chain, so a higher fraction of $\alpha$'s are shared between individuals, and hundreds of exact TCR $\alpha\beta$ receptors are shared between any pair of individuals.

**Cells**

B cells and T cells can be obtained from a variety of tissue samples including marrow, thymus, lymph glands, peripheral tissues and blood, but peripheral blood is most easily accessed. Peripheral blood samples are obtained by phlebotomy from subjects. Peripheral blood mononuclear cells (PBMC) are isolated by techniques known to those of skill in the art, e.g., by Ficoll-Hypaque® density gradient separation. Preferably, whole PBMCs are used for analysis. The B and/or T lymphocytes, instead, may be flow sorted into multiple compartments for each subject: e.g. $CD8^+CD45RO^{+/-}$ and $CD4^+CD45RO^{+/-}$ using fluorescently labeled anti-human antibodies, e.g, CD4 FITC (clone M-T466, Miltenyi Biotec), CD8 PE (clone RPA-T8, BD Biosciences), CD45RO ECD (clone UCHL-1, Beckman Coulter), and CD45RO APC (clone UCHL-1, BD Biosciences). Staining of total PBMCs may be done with the appropriate combination of antibodies, followed by washing cells before analysis. Lymphocyte subsets can be isolated by FACS sorting, e.g., by a BD FACSAria™ cell-sorting system (BD Biosciences) and by analyzing results with FlowJo software (Treestar Inc.), and also by conceptually similar methods involving specific antibodies immobilized to surfaces or beads.

**Nucleic Acid Extraction**

Total genomic DNA is extracted from cells, e.g., by using the QIAamp® DNA blood Mini Kit (QLAGEN®). The approximate mass of a single haploid genome is 3 pg. Preferably, at least 100,000 to 200,000 cells are used for analysis of diversity, i.e., about 0.6 to 1.2 μg DNA from diploid T cells. Using PBMCs as a source, the number of T cells can be estimated to be about 30% of total cells.

Alternatively, total nucleic acid can be isolated from cells, including both genomic DNA and mRNA. If diversity is to be measured from mRNA in the nucleic acid extract, the mRNA must be converted to cDNA prior to measurement. This can readily be done by methods of one of ordinary skill.

**DNA Amplification**

A multiplex PCR system is used to amplify rearranged TCR loci from genomic DNA, preferably from a CDR3 region, more preferably from a TCRα, TCRγ or TCRδ CDR3 region, most preferably from a TCR$\beta$ CDR3 region.

In general, a multiplex PCR system may use at least 14, 15, 16, 17, 18, 19, 20, 21, 22, 23, 24, or 25, preferably 26, 27, 28, 29, 30, 31, 32, 33, 34, 35, 36, 37, 38, or 39, most preferably 40, 41, 42, 43, 44, or 45 forward primers, in which each forward primer is specific to a sequence corresponding to one or more TRB V region segments shown in SEQ ID NOS:114-248; and at least 3, 4, 5, 6, or 7, preferably 8, 9, 10, 11, 12 or 13 reverse primers, in which each reverse primer is specific to a sequence corresponding to one or more TRB J region segments shown in SEQ ID NOS:249-261. Most preferably, there is a J segment primer for every J segment.

Preferably, the primers are designed not to cross an intron/exon boundary. The forward primers must preferably anneal to the V segments in a region of relatively strong sequence conservation between V segments so as to maximize the conservation of sequence among these primers. Accordingly, this minimizes the potential for differential annealing properties of each primer, and so that the amplified region between V and J primers contains sufficient TCR V sequence information to identify the specific V gene segment used.

Preferably, the J segment primers hybridize with a conserved element of the J segment, and have similar annealing strength. Most preferably, all J segment primers anneal to the same conserved framework region motif. The forward and reverse primers are both preferably modified at the 5' end with the universal forward primer sequence compatible with a DNA sequencer.

For example, a multiplex PCR system may use 45 forward primers (Table 1), each specific to a functional TCR V$\beta$ segment, and thirteen reverse primers (Table 2), each specific to a TCR J$\beta$ segment. Xn and Yn correspond to polynucleotides of lengths n and m, respectively, which would be specific to the single molecule sequencing technology being used to read out the assay.

**Table 1:** TCR-V$\beta$ Forward primer sequences

| TRBV gene segment(s) | SEQ ID NO: | Primer sequence* |
|---|---|---|
| TRBV2 | 1 | **Xn**TCAAATTTCACTCTGAAGATCCGGTCCACAA |
| TRBV3-1 | 2 | **Xn**GCTCACTTAAATCTTCACATCAATTCCCTGG |
| TRBV4-1 | 3 | **Xn**CTTAAACCTTCACCTACACGCCCTGC |
| TRBV(4-2, 4-3) | 4 | **Xn**CTTATTCCTTCACCTACACACCCTGC |
| TRBV5-1 | 5 | **Xn**GCTCTGAGATGAATGTGAGCACCTTG |
| TRBV5-3 | 6 | **Xn**GCTCTGAGATGAATGTGAGTGCCTTG |
| TRBV(5-4, 5-5, 5-6,5-7, 5-8) | 7 | **Xn**GCTCTGAGCTGAATGTGAACGCCTTG |
| TRBV6-1 | 8 | **Xn**TCGCTCAGGCTGGAGTCGGCTG |
| TRBV(6-2, 6-3) | 9 | **Xn**GCTGGGGTTGGAGTCGGCTG |
| TRBV6-4 | 10 | **Xn**CCCTCACGTTGGCGTCTGCTG |
| TRBV6-5 | 11 | **Xn**GCTCAGGCTGCTGTCGGCTG |
| TRBV6-6 | 12 | **Xn**CGCTCAGGCTGGAGTTGGCTG |
| TRBV6-7 | 13 | **Xn**CCCCTCAAGCTGGAGTCAGCTG |
| TRBV6-8 | 14 | **Xn**CACTCAGGCTGGTGTCGGCTG |
| TRBV6-9 | 15 | **Xn**CGCTCAGGCTGGAGTCAGCTG |
| TRBV7-1 | 16 | **Xn**CCACTCTGAAGTTCCAGCGCACAC |
| TRBV7-2 | 17 | **Xn**CACTCTGACGATCCAGCGCACAC |
| TRBV7-3 | 18 | **Xn**CTCTACTCTGAAGATCCAGCGCACAG |
| TRBV7-4 | 19 | **Xn**CCACTCTGAAGATCCAGCGCACAG |
| TRBV7-6 | 20 | **Xn**CACTCTGACGATCCAGCGCACAG |
| TRBV7-7 | 21 | **Xn**CCACTCTGACCATTCAGCGCACAG |
| TRBV7-8 | 22 | **Xn**CCACTCTGAAGATCCAGCGCACAC |
| TRBV7-9 | 23 | **Xn**CACCTTGGAGATCCAGCGCACAG |
| TRBV9 | 24 | **Xn**GCACTCTGAACTAAACCTGAGCTCTCTG |
| TRBV10-1 | 25 | **Xn**CCCCTCACTCTGGAGTCTGCTG |
| TRBV10-2 | 26 | **Xn**CCCCCTCACTCTGGAGTCAGCTA |
| TRBV10-3 | 27 | **Xn**CCTCCTCACTCTGGAGTCCGCTA |
| TRBV(11-1, 11-3) | 28 | **Xn**CCACTCTCAAGATCCAGCCTGCAG |
| TRBV11-2 | 29 | **Xn**CTCCACTCTCAAGATCCAGCCTGCAA |
| TRBV(12-3, 12-4, 12-5) | 30 | **Xn**CCACTCTGAAGATCCAGCCCTCAG |
| TRBV13 | 31 | **Xn**CATTCTGAACTGAACATGAGCTCCTTGG |
| TRBV14 | 32 | **Xn**CTACTCTGAAGGTGCAGCCTGCAG |
| TRBV15 | 33 | **Xn**GATAACTTCCAATCCAGGAGGCCGAACA |

(continued)

| TRBV gene segment(s) | SEQ ID NO: | Primer sequence* |
|---|---|---|
| TRBV16 | 34 | **Xn**CTGTAGCCTTGAGATCCAGGCTACGA |
| TRBV17 | 35 | **Xn**CTTCCACGCTGAAGATCCATCCCG |
| TRBV18 | 36 | **Xn**GCATCCTGAGGATCCAGCAGGTAG |
| TRBV19 | 37 | **Xn**CCTCTCACTGTGACATCGGCCC |
| TRBV20-1 | 38 | **Xn**CTTGTCCACTCTGACAGTGACCAGTG |
| TRBV23-1 | 39 | **Xn**CAGCCTGGCAATCCTGTCCTCAG |
| TRBV24-1 | 40 | **Xn**CTCCCTGTCCCTAGAGTCTGCCAT |
| TRBV25-1 | 41 | **Xn**CCCTGACCCTGGAGTCTGCCA |
| TRBV27 | 42 | **Xn**CCCTGATCCTGGAGTCGCCCA |
| TRBV28 | 43 | **Xn**CTCCCTGATTCTGGAGTCCGCCA |
| TRBV29-1 | 44 | **Xn**CTAACATTCTCAACTCTGACTGTGAGCAACA |
| TRBV30 | 45 | **Xn**CGGCAGTTCATCCTGAGTTCTAAGAAGC |

**Table 2:** TCR-J$\beta$ Reverse Primer Sequences

| TRBJ gene segment | SEQ ID NO: | Primer sequence* |
|---|---|---|
| TRBJ1-1 | 46 | **Ym**TTACCTACAACTGTGAGTCTGGTGCCTTGTCCAAA |
| TRBJ1-2 | 47 | **Ym**ACCTACAACGGTTAACCTGGTCCCCGAACCGAA |
| TRBJ1-3 | 48 | **Ym**ACCTACAACAGTGAGCCAACTTCCCTCTCCAAA |
| TRBJ1-4 | 49 | **Ym**CCAAGACAGAGAGCTGGGTTCCACTGCCAAA |
| TRBJ1-5 | 483 | **Ym**ACCTAGGATGGAGAGTCGAGTCCCATCACCAAA |
| TRBJ1-6 | 50 | **Ym**CTGTCACAGTGAGCCTGGTCCCGTTCCCAAA |
| TRBJ2-1 | 51 | **Ym**CGGTGAGCCGTGTCCCTGGCCCGAA |
| TRBJ2-2 | 52 | **Ym**CCAGTACGGTCAGCCTAGAGCCTTCTCCAAA |
| TRBJ2-3 | 53 | **Ym**ACTGTCAGCCGGGTGCCTGGGCCAAA |
| TRBJ2-4 | 54 | **Ym**AGAGCCGGGTCCCGGCGCCGAA |
| TRBJ2-5 | 55 | **Ym**GGAGCCGCGTGCCTGGCCCGAA |
| TRBJ2-6 | 56 | **Ym**GTCAGCCTGCTGCCGGCCCCGAA |
| TRBJ2-7 | 57 | **Ym**GTGAGCCTGGTGCCCGGCCCGAA |

The 45 forward PCR primers of Table 1 are complementary to each of the 48 functional Variable segments, and the thirteen reverse PCR primers of Table 2 are complementary to each of the functional joining (J) gene segments from the TRB locus (TRBJ). The TRB V region segments are identified in the Sequence Listing at SEQ ID NOS:114-248 and the TRB J region segments are at SEQ ID NOS:249-261. The primers have been designed such that adequate information is present within the amplified sequence to identify both the V and J genes uniquely (>40 base pairs of sequence upstream of the V gene recombination signal sequence (RSS), and >30 base pairs downstream of the J gene RSS). Alternative primers may be selected by one of ordinary skill from the V and J regions of the genes of each TCR subunit.

The forward primers are modified at the 5' end with the universal forward primer sequence compatible with the DNA sequencer (Xn of Table 1). Similarly, all of the reverse primers are modified with a universal reverse primer sequence (Ym of Table 2). One example of such universal primers is shown in Tables 3 and 4, for the Illumina GAII single-end read sequencing system. The 45 TCR V$\beta$ forward primers anneal to the V$\beta$ segments in a region of relatively strong sequence conservation between V$\beta$ segments so as to maximize the conservation of sequence among these primers.

**Table 3:** TCR-V$\beta$ Forward primer sequences

| TRBV gene segment(s) | SEQ ID NO: | Primer sequence* |
|---|---|---|
| TRBV2 | 58 | **CAAGCAGAAGACGGCATACGAGCTCTTCCGATCT**TCAAATTTCACTCTGAAGATCCGGTCCACAA |
| TRBV3-1 | 59 | **CAAGCAGAAGACGGCATACGAGCTCTTCCGATCT**GCTCACTTAAATCTTCACATCAATTCCCTGG |
| TRBV4-1 | 60 | **CAAGCAGAAGACGGCATACGAGCTCT**T**CCGATCT**CTTAAACCTTCACCTACACGCCCTGC |
| TRBV(4-2, 4-3) | 61 | **CAAGCAGAAGACGGCATACGAGCTCTTCCGATCT**CTTATTCCTTCACCTACACACCCTGC |
| TRBV5-1 | 62 | **CAAGCAGAAGACGGCATACGAGCTCTTCCGATCT**GCTCTGAGATGAATGAATGTGAGCACCTTG |
| TRBV5-3 | 63 | **CAAGCAGAAGACGGCATACGAGCTCTTCCGATCT**GCTCTGAGATGAATGTGAGTGCCTTG |
| TRBV(5-4, 5-5, 5-6, 5-7, 5-8) | 64 | **CAAGCAGAAGACGGCATACGAGCTCTTCCGATCT**GCTCTGAGCTGAATGTGAACGCCTTG |
| TRBV6-1 | 65 | **CAAGCAGAAGACGGCATACGAGCTCTTCCGATCT**TCGCTCAGGCTGGAGTCGGCTG |
| TRBV(6-2, 6-3) | 66 | **CAAGCAGAAGACGGCATACGAGCTCTTCCGATCT**GCTGGGGTTGGAGTCGGCTG |
| TRBV6-4 | 67 | **CAAGCAGAAGACGGCATACGAGCTCTTCCGATCT**CCCTCACGTTGGCGTCTGCTG |
| TRBV6-5 | 68 | **CAAGCAGAAGACGGCATACGAGCTCTTCCGATCT**GCTCAGGCTGCTGTCGGCTG |
| TRBV6-6 | 69 | **CAAGCAGAAGACGGCATACGAGCTCTTCCGATCT**CGCTCAGGCTGGAGTTGGCTG |
| TRBV6-7 | 70 | **CAAGCAGAAGACGGCATACGAGCTCTTCCGATCT**CCCCTCAAGCTGGAGTCAGCTG |
| TRBV6-8 | 71 | **CAAGCAGAAGACGGCATACGAGCTCTTCCGATCT**CACTCAGGCTGGTGTCGGCTG |
| TRBV6-9 | 72 | **CAAGCAGAAGACGGCATACGAGCTCTTCCGATCT**CGCTCAGGCTGGAGTCAGCTG |
| TRBV7-1 | 73 | **CAAGCAGAAGACGGCATACGAGCTCTTCCGATCT**CCACTCTGAAGTTCCAGCGCACAC |
| TRBV7-2 | 74 | **CAAGCAGAAGACGGCATACGAGCTCTTCCGATCT**CACTCTGACGATCCAGCGCACAC |
| TRBV7-3 | 75 | **CAAGCAGAAGACGGCATACGAGCTCTTCCGATCTCT**CTACTCTGAAGATCCAGCGCACAG |
| TRBV7-4 | 76 | **CAAGCAGAAGACGGCATACGAGCTCTTCCGATCT**CCACTCTGAAGATCCAGCGCACAG |
| TRBV7-6 | 77 | **CAAGCAGAAGACGGCATACGAGCTCTTCCGATCT**CACTCTGACGATCCAGCGCACAG |
| TRBV7-7 | 78 | **CAAGCAGAAGACGGCATACGAGCTCTTCCGATCT**CCACTCTGACGATTCAGCGCACAG |
| TRBV7-8 | 79 | **CAAGCAGAAGACGGCATACGAGCTCTTCCGATCTC**CACTCTGAAGATCCAGCGCACAC |
| TRBV7-9 | 80 | **CAAGCAGAAGACGGCATACGAGCTCTTCCGATCT**CACCTTGGAGATCCAGCGCACAG |
| TRBV9 | 81 | **CAAGCAGAAGACGGCATACGAGCTCTTCCGATCT**GCACTCTGAACTAAACCTGAGCTCTCTG |
| TRBV10-1 | 82 | **CAAGCAGAAGACGGCATACGAGCTCTTCCGATCT**CCCCTCACTCTGGAGTCTGCTG |
| TRBV10-2 | 83 | **CAAGCAGAAGACGGCATACGAGCTCTTCCGATCT**CCCCCTCACTCTGGAGTCAGCTA |
| TRBV10-3 | 84 | **CAAGCAGAAGACGGCATACGAGCTCTTCCGATCT**CCTCCTCACTCTGGAGTCCGCTA |
| TRBV(11-1, 11-3) | 85 | **CAAGCAGAAGACGGCATACGAGCTCTTCCGATCT**CCACTCTCAAGATCCAGCCTGCAG |
| TRBV11-2 | 86 | **CAAGCAGAAGACGGCATACGAGCTCTTCCGATCT**CTCCACTCTCAAGATCCAGCCTGCAA |
| TRBV(12-3, 12-4, 12-5) | 87 | **CAAGCAGAAGACGGCATACGAGCTCTTCCGATCT**CCACTCTGAAGATCCAGCCCTCAG |
| TRBV13 | 88 | **CAAGCAGAAGACGGCATACGAGCTCTTCCGATCT**CATTCTGAACTGAACATGAGCTCCTTGG |
| TRBV14 | 89 | **CAAGCAGAAGACGGCATACGAGCTCTTCCGATCT**CTACTCTGAAGGTGCAGCCTGCAG |
| TRBV15 | 90 | **CAAGCAGAAGACGGCATACGAGCTCTTCCGATCT**GATAACTTCCAATCCAGGAGGCCGAACA |

(continued)

| TRBV gene segment(s) | SEQ ID NO: | Primer sequence* |
|---|---|---|
| TRBV16 | 91 | **CAAGCAGAAGACGGCATACGAGCTCTTCCGATCT**CTGTAGCCTTGAGATCCAGGCTACGA |
| TRBV17 | 92 | **CAAGCAGAAGACGGCATACGAGCTCTTCCGATCT**CTTCCACGCTGAAGATCCATCCCG |
| TRBV18 | 93 | **CAAGCAGAAGACGGCATACGAGCTCTTCCGATCT**GCATCCTGAGGATCCAGCAGGTAG |
| TRBV19 | 94 | **CAAGCAGAAGACGGCATACGAGCTCTTCCGATCT**CCTCTCACTGTGACATCGGCCC |
| TRBV20-1 | 95 | **CAAGCAGAAGACGGCATACGAGCTCTTCCGATCT**CTTGTCCACTCTGACAGTGACCAGTG |
| TRBV23-1 | 96 | **CAAGCAGAAGACGGCATACGAGCTCTTCCGATCT**CAGCCTGGCAATCCTGTCCTCAG |
| TRBV24-1 | 97 | **CAAGCAGAAGACGGCATACGAGCTCTTCCGATCT**CTCCCTGTCCCTAGAGTCTGCCAT |
| TRBV25-1 | 98 | **CAAGCAGAAGACGGCATACGAGCTCTTCCGATCT**CCCTGACCCTGGAGTCTGCCA |
| TRBV27 | 99 | **CAAGCAGAAGACGGCATACGAGCTCTTCCGATCT**CCCTGATCCTGGAGTCGCCCA |
| TRBV28 | 100 | **CAAGCAGAAGACGGCATACGAGCTCTTCCGATCTCT**CCCTGATTCTGGAGTCCGCCA |
| TRBV29-1 | 101 | **CAAGCAGAAGACGGCATACGAGCTCTTCCGATCT**CTAACATTCAACTCTGACTGTGAGCAACA |
| TRBV30 | 102 | **CAAGCAGAAGACGGCATACGAGCTCTTCCGATCT**CGGCAGTTCATCCTGAGTTCTAAGAAGC |

**Table 4:** TCR-J$\beta$ Reverse Primer Sequences

| TRBJ gene segment | SEQ ID NO: | Primer sequence* |
|---|---|---|
| TRBJ1-1 | 103 | **AATGATACGGCGACCACCGAGATCTT**TACCTACAACTGTGAGTCTGGTGCCTTGTCCAAA |
| TRBJ1-2 | 468 | **AATGATACGGCGACCACCGAGATCT**ACCTACAACGGTTAACCTGGTCCCCGAACCGAA |
| TRBJ1-3 | 104 | **AATGATACGGCGACCACCGAGATCT**ACCTACAACAGTGAGCCAACTTCCCTCTCCAAA |
| TRBJ1-4 | 105 | **AATGATACGGCGACCACCGAGATCT**CCAAGACAGAGAGCTGGGTTCCACTGCCAAA |
| TRBJ1-5 | 484 | **AATGATACGGCGACCACCGAGATCT**ACCTAGGATGGAGAGTCGAGTCCCATCACCAAA |
| TRBJ1-6 | 106 | **AATGATACGGCGACCACCGAGATCT**CTGTCACAGTGAGCCTGGTCCCGTTCCCAAA |
| TRBJ2-1 | 107 | **AATGATACGGCGACCACCGAGATCT**CGGTGAGCCGTGTCCCTGGCCCGAA |
| TRBJ2-2 | 108 | **AATGATACGGCGACCACCGAGATCT**CCAGTACGGTCAGCCTAGAGCCTTCTCCAAA |
| TRBJ2-3 | 109 | **AATGATACGGCGACCACCGAGATCT**ACTGTCAGCCGGGTGCCTGGGCCAAA |
| TRBJ2-4 | 110 | **AATGATACGGCGACCACCGAGATCT**AGAGCCGGGTCCCGGCGCCGAA |
| TRBJ2-5 | 111 | **AATGATACGGCGACCACCGAGATCT**GGAGCCGCGTGCCTGGCCCGAA |
| TRBJ2-6 | 112 | **AATGATACGGCGACCACCGAGATCT**GTCAGCCTGCTGCCGGCCCCGAA |
| TRBJ2-7 | 113 | **AATGATACGGCGACCACCGAGATCT**GTGAGCCTGGTGCCCGGCCCGAA |

*bold sequence indicates universal R oligonucleotide for the sequence analysis

The total PCR product for a rearranged TCRβ CDR3 region using this system is expected to be approximately 200 bp long. Genomic templates are PCR amplified using a pool of the 45 TCR Vβ F primers (the "VF pool") and a pool of the twleve TCR Jβ R primers (the "JR pool"). For example, 50 μl PCR reactions may be used with 1.0 μM VF pool (22 nM for each unique TCR Vβ F primer), 1.0 μM JR pool (77 nM for each unique TCRBJR primer), 1X QIAGEN Multiple PCR master mix (QIAGEN part number 206145), 10% Q-solution (QIAGEN), and 16 ng/ul gDNA.

The IGH primer set was designed to try to accommodate the potential for somatic hypermutation within the rearranged IGH genes, as is observed after initial stimulation of naive B cells. Consequently all primers were designed to be slightly longer than normal, and to anchor the 3' ends of each primer into highly conserved sequences of three or more nucleotides that should be resistant to both functional and non-functional somatic mutations.

The IGHJ reverse primers were designed to anchor the 3' end of each PCR primer on a highly conserved GGGG sequence motif within the IGHJ segments. These sequences are shown in Table 5. Underlined sequence are ten base pairs in from RSS that may be deleted. These were excluded from barcode design. Bold sequence is the reverse complement of the IGH J reverse PCR primers. Italicized sequence is the barcode for J identity (eight barcodes reveal six genes, and two alleles within genes). Further sequence within underlined segment may reveal additional allelic identities.

**Table 5**

| IgH J segment | SEQ ID | Sequence |
|---|---|---|
| >IGHJ4*01/1-48 | 452 | ACTACTTTGA*CTA*CTGGGGCCAAGGAACCCTGGTCACCGTCTCCTCAG |
| >IGHJ4*03/1-48 | 453 | GCTACTTTGA*CTA*GTGGGGCCAAGGGACCCTGGTCACCGTCTCCTCAG |
| >IGHJ4*02/1-48 | 454 | ACTACTTTGA*CTA*CTGGGGCCAGGGAACCCTGGTCACCGTCTCCTCAG |
| >IGHJ3*01/1-50 | 455 | TGATGCTTTTGA*TGT*CTGGGGCCAAGGGACAATGGTCACCGTCTCTTCAG |
| >IGHJ3*02/1-50 | 456 | TGATGCTTTTGA*TATC*TGGGGCCAAGGOACAATGGTCACCGTCTCTTCAG |
| >IGHJ6*01/1-63 | 457 | ATTACTACTACTACTACGGTATGGA*CGT*CTGGGGGCAAGGGACCACGGTCACCGTCTCCTCAG |
| >IGHJ6*02/1-62 | 458 | ATTACTACTACTACTACGGTATGGA*CGT*CTGGGGCCAAGGGACCACGGTCACCGTCTCCTCAG |
| >IGHJ6*04/1-63 | 459 | ATTACTACTACTACTACGGTATGGA*CGT*CTGGGGCAAAGGGACCACGGTCACCGTCTCCTCAG |
| >IGHJ6*03/1-62 | 460 | ATTACTACTACTACTACTACATGGA*CGT*CTGGGGCAAAGGGACCACGGTCACCGTCTCCTCAG |
| >IGHJ2*01/1-53 | 461 | CTACTGGTACTTCGA*TCT*CTGGGGCCGTGGCACCCTGGTCACTGTCTCCTCAG |
| >IGHJ5*01/1-51 | 462 | ACAACTGGTTCGA*CTC*CTGGGGCCAAGGAACCCTGGTCACCGTCTCCTCAG |
| >IGHJ5*02/1-51 | 463 | ACAACTGGTTCGA*CCCC*TGGGGCCAGGGAACCCTGGTCACCGTCTCCTCAG |
| >IGHJ1*01/1-52 | 464 | GCTGAATACTTCCA*GCA*CTGGGGCCAGGGCACCCTGGTCACCGTCTCCTCAG |
| >IGHJ2P*01/1-61 | 465 | CTACAAGTGCTTGGAGCACTGGGGCAGGGCAGCCCGGACACCGTCTCCCTGGGAACGTCAG |
| >IGHJ1P*01/1-54 | 466 | AAAGGTGCTGGGGGTCCCCTGAACCCGACCCGCCCTGAGACCGCAGCCACATCA |
| >IGHJ3P*01/1-52 | 467 | CTTGCGGTTGGACTTCCCAGCCGACAGTGGTGGTCTGGCTTCTGAGGGGTCA |

Sequences of the IGHJ reverse PCR primers are shown in Table 6.

**Table 6**

| IgH J segment | SEQ ID NO: | sequence |
|---|---|---|
| >IGHJ4_1 | 421 | TGAGGAGACGGTGACCAGGGTTCCTTGGCCC |
| >IGHJ4_3 | 422 | TGAGGAGACGGTGACCAGGGTCCCTTGGCCC |
| >IGHJ4_2 | 423 | TGAGGAGACGGTGACCAGGGTTCCCTGGCCC |
| >IGHJ3_12 | 424 | CTGAAGAGACGGTGACCATTGTCCCTTGGCCC |
| >IGHJ6-1 | 425 | CTGAGGAGACGGTGACCGTGGTCCCTTGCCCC |
| >IGHJ6_2 | 426 | TGAGGAGACGGTGACCGTGGTCCCTTGGCCC |
| >IGHJ6_34 | 427 | CTGAGGAGACGGTGACCGTGGTCCCTTTGCCC |
| >IGHJ2_1 | 428 | CTGAGGAGACAGTGACCAGGGTGCCACGGCCC |
| >IGHJ5_1 | 429 | CTGAGGAGACGGTGACCAGGGTTCCTTGGCCC |
| >IGHJ5_2 | 430 | CTGAGGAGACGGTGACCAGGGTTCCCTGGCCC |
| >IGHJ1_1 | 431 | CTGAGGAGACGGTGACCAGGGTGCCCTGGCCC |

V primers were designed in a conserved in region of FR2 between the two conserved tryptophan (W) codons.

The primer sequences are anchored at the 3' end on a tryptophan codon for all IGHV families that conserve this codon. his allows for the last three nucleotides (tryptophan's TGG) to anchor on sequence that is expected to be resistant to somatic hypermutation, providing a 3' anchor of five out of six nucleotides for each primer. The upstream sequence is extended further than normal, and includes degenerate nucleotides to allow for mismatches induced by hypermutation (or between closely relate IGH V families) without dramatically changing the annealing characteristics of the primer, as shown in Table 7. The sequences of the V gene segments are SEQ ID NOS:262-420.

**Table 7**

| IgH V segment | SEQ ID NO: | sequence |
|---|---|---|
| >IGHV1 | 443 | TGGGTGCACCAGGTCCANGNACAAGGGCTTGAGTGG |
| >IGHV2 | 444 | TGGGTGCGACAGGCTCGNGNACAACGCCTTGAGTGG |
| >IGHV3 | 445 | TGGGTGCGCCAGATGCCNGNGAAAGGCCTGGAGTGG |
| >IGHV4 | 446 | TGGGTCCGCCAGSCYCCNGNGAAGGGGCTGGAGTGG |
| >IGHV5 | 447 | TGGGTCCGCCAGGCTCCNGNAAAGGGGCTGGAGTGG |
| >IGHV6 | 448 | TGGGTCTGCCAGGCTCCNGNGAAGGGGCAGGAGTGG |
| >IGH7_3.25p | 449 | TGTGTCCGCCAGGCTCCAGGGAATGGGCTGGAGTTGG |
| >IGH8_3.54p | 450 | TCAGATTCCCAAGCTCCAGGGAAGGGGCTGGAGTGAG |
| >IGH9_3.63p | 451 | TGGGTCAATGAGACTCTAGGGAAGGGGCTGGAGGGAG |

Thermal cycling conditions may follow methods of those skilled in the art. For example, using a PCR Express thermal cycler (Hybaid, Ashford, UK), the following cycling conditions may be used: 1 cycle at 95°C for 15 minutes, 25 to 40 cycles at 94°C for 30 seconds, 59°C for 30 seconds and 72°C for 1 minute, followed by one cycle at 72°C for 10 minutes.

**Sequencing**

Sequencing is achieved using a set of sequencing oligonucleotides that hybridize to a defined region within the amplified DNA molecules.

Preferably, the amplified J gene segments each have a unique four base tag at positions +11 through +14 downstream from the RSS site. Accordingly, the sequencing oligonucleotides hybridize adjacent to a four base tag within the amplified Jβ gene segments at positions +11 through +14 downstream of the RSS site.

For example, sequencing oligonucleotides for TCRB may be designed to anneal to a consensus nucleotide motif observed just downstream of this "tag", so that the first four bases of a sequence read will uniquely identify the J segment (Table 8).

**Table 8:** Sequencing oligonucleotides

| Sequencing oligonucleotide | SEQ ID NO: | Oligonucleotide sequence |
|---|---|---|
| Jseq 1-1 | 470 | ACAACTGTGAGTCTGGTGCCTTGTCCAAAGAAA |
| Jseq 1-2 | 471 | ACAACGGTTAACCTGGTCCCCGAACCGAAGGTG |
| Jseq 1-3 | 472 | ACAACAGTGAGCCAACTTCCCTCTCCAAAATAT |
| Jseq 1-4 | 473 | AAGACAGAGAGCTGGGTTCCACTGCCAAAAAAC |
| Jseq 1-5 | 474 | AGGATGGAGAGTCGAGTCCCATCACCAAAATGC |
| Jseq 1-6 | 475 | GTCACAGTGAGCCTGGTCCCGTTCCCAAAGTGG |
| Jseq 2-1 | 476 | AGCACGGTGAGCCGTGTCCCTGGCCCGAAGAAC |
| Jseq 2-2 | 477 | AGTACGGTCAGCCTAGAGCCTTCTCCAAAAAAC |
| Jseq 2-3 | 478 | AGCACTGTCAGCCGGGTGCCTGGGCCAAAATAC |
| Jseq 2-4 | 479 | AGCACTGAGAGCCGGGTCCCGGCGCCGAAGTAC |
| Jseq 2-5 | 480 | AGCACCAGGAGCCGCGTGCCTGGCCCGAAGTAC |
| Jseq 2-6 | 481 | AGCACGGTCAGCCTGCTGCCGGCCCCGAAAGTC |
| Jseq 2-7 | 482 | GτGACCGTGAGCCTGGTGCCCGGCCCGAAGTAC |

The information used to assign the J and V segment of a sequence read is entirely contained within the amplified sequence, and does not rely upon the identity of the PCR primers. These sequencing oligonucleotides were selected such that promiscuous priming of a sequencing reaction for one J segment by an oligonucleotide specific to another J segment would generate sequence data starting at exactly the same nucleotide as sequence data from the correct sequencing oligonucleotide. In this way, promiscuous annealing of the sequencing oligonucleotides did not impact the quality of the sequence data generated.

The average length of the CDR3 region, defined as the nucleotides between the second conserved cysteine of the V segment and the conserved phenylalanine of the J segment, is 35+/-3, so sequences starting from the Jβ segment tag will nearly always capture the complete V-D-J junction in a 50 base pair read.

TCR βJ gene segments are roughly 50 base pair in length. PCR primers that anneal and extend to mismatched sequences are referred to as promiscuous primers. The TCR Jβ Reverse PCR primers were designed to minimize overlap with the sequencing oligonucleotides to minimize promiscuous priming in the context of multiplex PCR. The 13 TCR Jβ reverse primers are anchored at the 3' end on the consensus splice site motif, with minimal overlap of the sequencing primers. The TCR Jβ primers provide consistent annealing temperature using the sequencer program under default parameters.

For the sequencing reaction, the IGHJ sequencing primers extend three nucleotides across the conserved CAG sequences as shown in Table 9.

**Table 9**

| IgH J segment | SEQ ID NO: | sequence |
|---|---|---|
| >IGHJSEQ4_1 | 432 | TGAGGAGACGGTGACCAGGGTTCCTTGGCCCCAG |
| >IGHJSEQ4_3 | 433 | TGAGGAGACGGTGACCAGGGTCCCTTGGCCCCAG |
| >IGHJSEQ4_2 | 434 | TGAGGAGACGGTGACCAGGGTTCCCTGGCCCCAG |
| >IGHJSEQ3_12 | 435 | CTGAAGAGACGGTGACCATTGTCCCTTGGCCCCAG |
| >IGHJSEQ6_1 | 436 | CTGAGGAGACGGTGACCGTGGTCCCTTGCCCCCAG |
| >IGHJSEQ6_2 | 437 | TGAGGAGACGGTGACCGTGGTCCCTTGGCCCCAG |

(continued)

| IgH J segment | SEQ ID NO: | sequence |
|---|---|---|
| >IGHJSEQ6_34 | 438 | CTGAGGAGACGGTGACCGTGGTCCCTTTGCCCCAG |
| >IGHJSEQ2_1 | 439 | CTGAGGAGACAGTGACCAGGGTGCCACGGCCCCAG |
| >IGHJSEQ5_1 | 440 | CTGAGGAGACGGTGACCAGGGTTCCTTGGCCCCAG |
| >IGHJSEQ5_2 | 441 | CTGAGGAGACGGTGACCAGGGTTCCCTGGCCCCAG |
| >IGHJSEQ1_1 | 442 | CTGAGGAGACGGTGACCAGGGTGCCCTGGCCCCAG |

**Processing sequence data**

For rapid analysis of sequencing results, an algorithm can be developed by one of ordinary skill. A preferred method is as follows.

The use of a PCR step to amplify the TCR$\beta$ CDR3 regions prior to sequencing could potentially introduce a systematic bias in the inferred relative abundance of the sequences, due to differences in the efficiency of PCR amplification of CDR3 regions utilizing different V$\beta$ and J$\beta$ gene segments. Each cycle of PCR amplification potentially introduces a bias of average magnitude $1.5^{1/15} = 1.027$. Thus, the 25 cycles of PCR introduces a total bias of average magnitude $1.027^{25} = 1.95$ in the inferred relative abundance of distinct CDR3 region sequences.

Sequenced reads were filtered for those including CDR3 sequences. Sequencer data processing involves a series of steps to remove errors in the primary sequence of each read, and to compress the data. A complexity filter removes approximately 20% of the sequences that are misreads from the sequencer. Then, sequences were required to have a minimum of a six base match to both one of the thirteen TCRB J-regions and one of 54 V-regions. Applying the filter to the control lane containing phage sequence, on average only one sequence in 7-8 million passed these steps. Finally, a nearest neighbor algorithm was used to collapse the data into unique sequences by merging closely related sequences, in order to remove both PCR error and sequencing error.

Analyzing the data, the ratio of sequences in the PCR product must be derived working backward from the sequence data before estimating the true distribution of clonotypes in the blood. For each sequence observed a given number of times in the data herein, the probability that that sequence was sampled from a particular size PCR pool is estimated. Because the CDR3 regions sequenced are sampled randomly from a massive pool of PCR products, the number of observations for each sequence are drawn from Poisson distributions. The Poisson parameters are quantized according to the number of T cell genomes that provided the template for PCR. A simple Poisson mixture model both estimates these parameters and places a pairwise probability for each sequence being drawn from each distribution. This is an expectation maximization method which reconstructs the abundances of each sequence that was drawn from the blood.

To estimate diversity, the "unseen species" formula is employed. To apply this formula, unique adaptive immune receptors (e.g. TCRB) clonotypes takes the place of species. The mathematical solution provides that for a total number of TCRβ "species" or clonotypes, *S*, a sequencing experiment observes $x_s$ copies of sequence s. For all of the unobserved clonotypes, $x_s$ equals *0*, and each TCR clonotype is "captured" in a blood draw according to a Poisson process with parameter $\lambda_s$. The number of T cell genomes sequenced in the first measurement *1*, and in the second measurement. Since there are a large number of unique sequences, an integral will represent the sum. If $G(\lambda)$ is the empirical distribution function of the parameters $\lambda_l, ..., \lambda_S$, and $n_x$ is the number of clonotypes sequenced exactly x times, then the total number of clonotypes, i.e., the measurement of diversity *E*, is given by the following formula:

$$E(n_x) = S\int_0^\infty \left( \frac{e^{-\lambda}\lambda^x}{x!} \right) dG(\lambda).$$

For a given experiment, where T cells are sampled from some arbitrary source (e.g. a blood draw), the formula is used to estimate the total diversity of species in the entire source. The idea is that the sampled number of clonotypes at each size contains sufficient information to estimate the underlying distribution of clonotypes in the whole source. To derive the formula, the number of new species expected if the exact measurement was repeated was estimated. The limit of the formula as if repeating the measurements an infinite number of times. The result is the expect number of species in the total underlying source population. The value for Δ(t), the number of *new* clonotypes observed in a second measurement, should be determined, preferably using the following equation:

$$\Delta(t) = \sum_x E(n_x)_{msmt1+msmt2} - \sum_x E(n_x)_{msmt1} = S\int_0^{\infty} e^{-\lambda}\left(1-e^{-\lambda t}\right)dG(\lambda)$$

in which *msmt1* and *msmt2* are the number of clonotypes from measurement 1 and 2, respectively. Taylor expansion of $1-e^{-\lambda t}$ gives $\Delta(t) = E(x_1)t-E(x_2)t^2+E(x_3)t^3-...$, which can be approximated by replacing the expectations $E(n_x)$ with the observed numbers in the first measurement. Using in the numbers observed in the first measurement, this formula predicts that $1.6*10^5$ new unique sequences should be observed in the second measurement. The actual value of the second measurement was $1.8*10^5$ new TCRβ sequences, which implies that the prediction provided a valid lower bound on total diversity. An Euler's transformation was used to regularize $\Delta(t)$ to produce a lower bound for $\Delta(\infty)$.

**Using a measurement of diversity to diagnose disease**

The measurement of diversity can be used to diagnose disease or the effects of a treatment, as follows. T cell and/or B cell receptor repertoires can be measured at various time points, e.g., after hematopoietic stem cell transplant (HSCT) treatment for leukemia. Both the change in diversity and the overall diversity of TCRB repertoire can be utilized to measure immunocompetence. A standard for the expected rate of immune reconstitution after transplant can be utilized. The rate of change in diversity between any two time points may be used to actively modify treatment. The overall diversity at a fixed time point is also an important measure, as this standard can be used to compare between different patients. In particular, the overall diversity is the measure that should correlate with the clinical definition of immune reconstitution. This information may be used to modify prophylactic drug regiments of antibiotics, antivirals, and antifungals, e.g., after HSCT.

The assessment of immune reconstitution after allogeneic hematopoietic cell transplantation can be determined by measuring changes in diversity. These techniques will also enhance the analysis of how lymphocyte diversity declines with age, as measured by analysis of T cell responses to vaccination. Further, the methods of the invention provide a means to evaluate investigational therapeutic agents (e.g., Interleukin-7 (IL-7)) that have a direct effect on the generation, growth, and development of αβ T cells. Moreover, application of these techniques to the study of thymic T cell populations will provide insight into the processes of both T cell receptor gene rearrangement as well as positive and negative selection of thymocytes.

A newborn that does not yet have a fully functioning immune system but may have maternally transmitted antibody is immunodeficient. A newborn is susceptible to a number of diseases until its immune system autonomously develops, and our measurement of the adaptive immune system may will likely prove useful with newborn patients.

Lymphocyte diversity can be assessed in other states of congenital or acquired immunodeficiency. An AIDS patient with a failed or failing immune system can be monitored to determine the stage of disease, and to measure a patient's response to therapies aimed to reconstitute immunocompetence.

Another application of the methods of the invention is to provide diagnostic measures for solid organ transplant recipients taking medication so their body will not reject the donated organ. Generally, these patients are under immunosuppressive therapies. Monitoring the immunocompetence of the host will assist before and after transplantation.

Individuals exposed to radiation or chemotherapeutic drugs are subject to bone marrow transplantations or otherwise require replenishment of T cell populations, along with associated immunocompetence. The methods of the invention provide a means for qualitatively and quantitatively assessing the bone marrow graft, or reconstitution of lymphocytes in the course of these treatments.

One manner of determining diversity is by comparing at least two samples of genomic DNA, preferably in which one sample of genomic DNA is from a patient and the other sample is from a normal subject, or alternatively, in which one sample of genomic DNA is from a patient before a therapeutic treatment and the other sample is from the patient after treatment, or in which the two samples of genomic DNA are from the same patient at different times during treatment. Another manner of diagnosis may be based on the comparison of diversity among the samples of genomic DNA, e.g., in which the immunocompetence of a human patient is assessed by the comparison.

**Biomarkers**

Shared TCR sequences between individuals represent a new class of potential biomarkers for a variety of diseases, including cancers, autoimmune diseases, and infectious diseases. These are the public T cells that have been reported for multiple human diseases. TCRs are useful as biomarkers because T cells are a result of clonal expansion, by which the immune system amplifies these biomarkers through rapid cell division. Following amplification, the TCRs are readily detected even if the target is small (e.g. an early stage tumor). TCRs are also useful as biomarkers because in many cases the T cells might additionally contribute to the disease causally and, therefore could constitute a drug target. T cells self interactions are thought to play a major role in several diseases associated with autoimmunity, e.g., multiple sclerosis, Type I diabetes, and rheumatoid arthritis.

## EXAMPLES

**Example 1:** Sample acquisition, PBMC isolation, FACS sorting and genomic DNA extraction

Peripheral blood samples from two healthy male donors aged 35 and 37 were obtained with written informed consent using forms approved by the Institutional Review Board of the Fred Hutchinson Cancer Research Center (FHCRC). Peripheral blood mononuclear cells (PBMC) were isolated by Ficoll-Hypaque® density gradient separation. The T-lymphocytes were flow sorted into four compartments for each subject: $CD8^+CD45RO^{+/-}$ and $CD4^+CD45RO^{+/-}$. For the characterization of lymphocytes the following conjugated anti-human antibodies were used: CD4 FITC (clone M-T466, Miltenyi Biotec), CD8 PE (clone RPA-T8, BD Biosciences), CD45RO ECD (clone UCHL-1, Beckman Coulter), and CD45RO APC (clone UCHL-1, BD Biosciences). Staining of total PBMCs was done with the appropriate combination of antibodies for 20 minutes at 4°C, and stained cells were washed once before analysis. Lymphocyte subsets were isolated by FACS sorting in the BD FACSAria™ cell-sorting system (BD Biosciences). Data were analyzed with FlowJo software (Treestar Inc.).

Total genomic DNA was extracted from sorted cells using the QIAamp® DNA blood Mini Kit (QIAGEN®). The approximate mass of a single haploid genome is 3 pg. In order to sample millions of rearranged TCRB in each T cell compartment, 6 to 27 micrograms of template DNA were obtained from each compartment (see Table 10).

**Table 10**

| | CD8+/CD45RO- | CD8+/CD45RO+ | CD4+/CD45RO- | CD4+/CD45RO+ | Donor |
|---|---|---|---|---|---|
| cells ($x10^6$) | 9.9 | 6.3 | 6.3 | 10 | 2 |
| DNA (μg) | 27 | 13 | 19 | 25 | |
| PCR cycles | 25 | 25 | 30 | 30 | |
| clusters (K/ tile) | 29.3 | 27 | 102.3* | 118,3* | |
| VJ sequences ($x10^6$) | 3.0 | 2.0 | 4.4 | 4.2 | |
| Cells | 4.9 | 4.8 | 3.3 | 9 | 1 |
| DNA | 12 | 13 | 6.6 | 19 | |
| PCR cycles | 30 | 30 | 30 | 30 | |
| Clusters | 116.3 | 121 | 119.5 | 124.6 | |
| VJ sequences | 3.2 | 3.7 | 4.0 | 3.8 | |
| Cells | NA | NA | NA | 0.03 | PCR Bias assessment |
| DNA | NA | NA | NA | 0.015 | |
| PCR cycles | NA | NA | NA | 25 + 15 | |
| clusters | NA | NA | NA | 1.4/23.8 | |
| VJ sequences | NA | NA | NA | 1.6 | |

**Example 2:** Virtual T cell receptor β chain spectratyping

Virtual TCR β chain spectratyping was performed as follows. Complementary DNA was synthesized from RNA extracted from sorted T cell populations and used as template for multiplex PCR amplification of the rearranged TCR β chain CDR3 region. Each multiplex reaction contained a 6-FAM-labeled antisense primer specific for the TCR β chain constant region, and two to five TCR β chain variable (TRBV) gene-specific sense primers. All 23 functional Vβ families were studied. PCR reactions were carried out on a Hybaid PCR Express thermal cycler (Hybaid, Ashford, UK) under the following cycling conditions: 1 cycle at 95°C for 6 minutes, 40 cycles at 94°C for 30 seconds, 58°C for 30 seconds, and 72°C for 40 seconds, followed by 1 cycle at 72°C for 10 minutes. Each reaction contained cDNA template, 500 μM dNTPs, 2mM $MgCl_2$ and 1 unit of AmpliTaq Gold DNA polymerase (Perkin Elmer) in AmpliTaq Gold buffer, in a final volume of 20 μl. After completion, an aliquot of the PCR product was diluted 1:50 and analyzed using a DNA analyzer. The output of the DNA analyzer was converted to a distribution of fluorescence intensity vs. length by comparison with the fluorescence intensity trace of a reference sample containing known size standards.

**Example 3:** Multiplex PCR amplification of TCRβ CDR3 regions

The CDR3 junction region was defined operationally, as follows. The junction begins with the second conserved cysteine of the V-region and ends with the conserved phenylalanine of the J-region. Taking the reverse complements of the observed sequences and translating the flanking regions, the amino acids defining the junction boundaries were identified. The number of nucleotides between these boundaries determines the length and therefore the frame of the CDR3 region. In order to generate the template library for sequencing, a multiplex PCR system was selected to amplify rearranged TCR*β* loci from genomic DNA. The multiplex PCR system uses 45 forward primers (Table 3), each specific to a functional TCR V*β* segment, and thirteen reverse primers (Table 4), each specific to a TCR J*β* segment. The primers were selected to provide that adequate information is present within the amplified sequence to identify both the V and J genes uniquely (>40 base pairs of sequence upstream of the V gene recombination signal sequence (RSS), and >30 base pairs downstream of the J gene RSS).

The forward primers are modified at the 5' end with the universal forward primer sequence compatible with the Illumina GA2 cluster station solid-phase PCR. Similarly, all of the reverse primers are modified with the GA2 universal reverse primer sequence. The 3' end of each forward primer is anchored at position -43 in the Vβ segment, relative to the recombination signal sequence (RSS), thereby providing a unique Vβ tag sequence within the amplified region. The thirteen reverse primers specific to each Jβ segment are anchored in the 3' intron, with the 3' end of each primer crossing the intron/exon junction. Thirteen sequencing primers complementary to the Jβ segments were designed that are complementary to the amplified portion of the Jβ segment, such that the first few bases of sequence generated will capture the unique Jβ tag sequence.

On average J deletions were 4 bp +/- 2.5 bp, which implies that J deletions greater than 10 nucleotides occur in less than 1% of sequences. The thirteen different TCR J*β* gene segments each had a unique four base tag at positions +11 through +14 downstream of the RSS site. Thus, sequencing oligonucleotides were designed to anneal to a consensus nucleotide motif observed just downstream of this "tag", so that the first four bases of a sequence read will uniquely identify the J segment (Table 5).

The information used to assign the J and V segment of a sequence read is entirely contained within the amplified sequence, and does not rely upon the identity of the PCR primers. These sequencing oligonucleotides were selected such that promiscuous priming of a sequencing reaction for one J segment by an oligonucleotide specific to another J segment would generate sequence data starting at exactly the same nucleotide as sequence data from the correct sequencing oligonucleotide. In this way, promiscuous annealing of the sequencing oligonucleotides did not impact the quality of the sequence data generated.

The average length of the CDR3 region, defined following convention as the nucleotides between the second conserved cysteine of the V segment and the conserved phenylalanine of the J segment, is 35+/-3, so sequences starting from the Jβ segment tag will nearly always capture the complete VNDNJ junction in a 50 bp read.

TCR βJ gene segments are roughly 50 bp in length. PCR primers that anneal and extend to mismatched sequences are referred to as promiscuous primers. Because of the risk of promiscuous priming in the context of multiplex PCR, especially in the context of a gene family, the TCR Jβ Reverse PCR primers were designed to minimize overlap with the sequencing oligonucleotides. Thus, the 13 TCR Jβ reverse primers are anchored at the 3' end on the consensus splice site motif, with minimal overlap of the sequencing primers. The TCR Jβ primers were designed for a consistent annealing temperature (58 degrees in 50 mM salt) using the OligoCalc program under default parameters (http://www.basic.northwestern.edu/biotools/oligocalc.html).

The 45 TCR Vβ forward primers were designed to anneal to the Vβ segments in a region of relatively strong sequence conservation between Vβ segments, for two express purposes. First, maximizing the conservation of sequence among these primers minimizes the potential for differential annealing properties of each primer. Second, the primers were chosen such that the amplified region between V and J primers will contain sufficient TCR V*β* sequence information to identify the specific Vβ gene segment used. This obviates the risk of erroneous TCR Vβ gene segment assignment, in the event of promiscuous priming by the TCR Vβ primers. TCR Vβ forward primers were designed for all known non-pseudogenes in the TCRβ locus.

The total PCR product for a successfully rearranged TCRβ CDR3 region using this system is expected to be approximately 200 bp long. Genomic templates were PCR amplified using an equimolar pool of the 45 TCR Vβ F primers (the "VF pool") and an equimolar pool of the thirteen TCR Jβ R primers (the "JR pool"). 50 μl PCR reactions were set up at 1.0 μM VF pool (22 nM for each unique TCR Vβ F primer), 1.0 μM JR pool (77 nM for each unique TCRBJR primer), 1X QIAGEN Multiple PCR master mix (QIAGEN part number 206145), 10% Q-solution (QIAGEN), and 16 ng/ul gDNA. The following thermal cycling conditions were used in a PCR Express thermal cycler (Hybaid, Ashford, UK) under the following cycling conditions: 1 cycle at 95°C for 15 minutes, 25 to 40 cycles at 94°C for 30 seconds, 59°C for 30 seconds and 72°C for 1 minute, followed by one cycle at 72°C for 10 minutes. 12-20 wells of PCR were performed for each library, in order to sample hundreds of thousands to millions of rearranged TCRβ CDR3 loci.

**Example 4:** Pre-processing of sequence data

Sequencer data processing involves a series of steps to remove errors in the primary sequence of each read, and to compress the data. First, a complexity filter removes approximately 20% of the sequences which are misreads from the sequencer. Then, sequences were required to have a minimum of a six base match to both one of the thirteen J-regions and one of 54 V-regions. Applying the filter to the control lane containing phage sequence, on average only one sequence in 7-8 million passed these steps without false positives. Finally, a nearest neighbor algorithm was used to collapse the data into unique sequences by merging closely related sequences, in order to remove both PCR error and sequencing error (see Table 10).

**Example 5:** Estimating relative CDR3 sequence abundance in PCR pools and blood samples

After collapsing the data, the underlying distribution of T-cell sequences in the blood reconstructing were derived from the sequence data. The procedure used three steps; 1) flow sorting T-cells drawn from peripheral blood, 2) PCR amplification, and 3) sequencing. Analyzing the data, the ratio of sequences in the PCR product must be derived working backward from the sequence data before estimating the true distribution of clonotypes in the blood.

For each sequence observed a given number of times in the data herein, the probability that that sequence was sampled from a particular size PCR pool is estimated. Because the CDR3 regions sequenced are sampled randomly from a massive pool of PCR products, the number of observations for each sequence are drawn from Poisson distributions. The Poisson parameters are quantized according to the number of T cell genomes that provided the template for PCR. A simple Poisson mixture model both estimates these parameters and places a pairwise probability for each sequence being drawn from each distribution. This is an expectation maximization method which reconstructs the abundances of each sequence that was drawn from the blood.

**Example 6:** Unseen species model for estimation of true diversity

A mixture model can reconstruct the frequency of each TCR$\beta$ CDR3 species drawn from the blood, but the larger question is how many unique CDR3 species were present in the donor? This is a fundamental question that needs to be answered as the available sample is limited in each donor, and will be more important in the future as these techniques are extrapolated to the smaller volumes of blood that can reasonably be drawn from patients undergoing treatment.

The mathematical solution provides that for a total number of TCRβ "species" or clonotypes, *S,* a sequencing experiment observes $x_s$ copies of sequence s. For all of the unobserved clonotypes, $x_s$ equals *0*, and each TCR clonotype is "captured" in a blood draw according to a Poisson process with parameter $\lambda_S$. The number of T cell genomes sequenced in the first measurement *1,* and in the second measurement. Since there are a large number of unique sequences, an integral will represent the sum. If $G(\lambda)$ is the empirical distribution function of the parameters $\lambda_l$, ..., $\lambda_S$, and $n_x$ is the number of clonotypes sequenced exactly x times, then

$$E(n_x) = S\int_0^\infty \left(\frac{e^{-\lambda}\lambda^x}{x!}\right) dG(\lambda).$$

The value Δ(t) is the number of *new* clonotypes observed in the second sequencing experiment.

$$\Delta(t) = \sum_x E(n_x)_{\text{exp1+exp2}} - \sum_x E(n_x)_{\text{exp1}} = S\int_0^\infty e^{-\lambda}\left(1-e^{-\lambda t}\right) dG(\lambda)$$

Taylor expansion of $1\text{-}e^{\lambda t}$ gives $\Delta(t) = E(x_1)t - E(x_2)t^2 + E(x_3)t^3 - ...$, which can be approximated by replacing the expectations ($E(n_x)$) with the observed numbers in the first measurement. Using in the numbers observed in the first measurement, this formula predicts that $1.6 \cdot 10^5$ new unique sequences should be observed in the second measurement. The actual value of the second measurement was $1.8 \cdot 10^5$ new TCRβ sequences, which implies that the prediction provided a valid lower bound on total diversity. An Euler's transformation was used to regularize $\Delta(t)$ to produce a lower bound for $\Delta(\infty)$.

**Example 7:** Error correction and bias assessment

Sequence error in the primary sequence data derives primarily from two sources: (1) nucleotide misincorporation that occurs during the amplification by PCR of TCR$\beta$ CDR3 template sequences, and (2) errors in base calls introduced during sequencing of the PCR-amplified library of CDR3 sequences. The large quantity of data allows us to implement a straightforward error correcting code to correct most of the errors in the primary sequence data that are attributable to these two sources. After error correction, the number of unique, in-frame CDR3 sequences and the number of observations of each unique sequence were tabulated for each of the four flow-sorted T cell populations from the two donors. The relative frequency distribution of CDR3 sequences in the four flow cytometrically-defined populations demonstrated that antigen-experienced CD45RO$^+$ populations contained significantly more unique CDR3 sequences with high relative frequency than the CD45RO$^-$ populations. Frequency histograms of TCRβ CDR3 sequences observed in four different T cell subsets distinguished by expression of CD4, CD8, and CD45RO and present in blood showed that ten unique sequences were each observed 200 times in the CD4$^+$CD45RO$^+$ (antigen-experienced) T cell sample, which was more than twice as frequent as that observed in the CD4$^+$CD45RO$^-$ populations.

The use of a PCR step to amplify the TCR$\beta$ CDR3 regions prior to sequencing could potentially introduce a systematic bias in the inferred relative abundance of the sequences, due to differences in the efficiency of PCR amplification of CDR3 regions utilizing different V$\beta$ and J$\beta$ gene segments. To estimate the magnitude of any such bias, the TCR$\beta$ CDR3 regions from a sample of approximately 30,000 unique CD4$^+$CD45RO$^+$ T lymphocyte genomes were amplified through 25 cycles of PCR, at which point the PCR product was split in half. Half was set aside, and the other half of the PCR product was amplified for an additional 15 cycles of PCR, for a total of 40 cycles of amplification. The PCR products amplified through 25 and 40 cycles were then sequenced and compared. Over 95% of the 25 cycle sequences were also found in the 40-cycle sample: a linear correlation is observed when comparing the frequency of sequences between these samples. For sequences observed a given number of times in the 25 cycle lane, a combination of PCR bias and sampling variance accounts for the variance around the mean of the number of observations at 40 cycles. Conservatively attributing the mean variation about the line (1.5-fold) entirely to PCR bias, each cycle of PCR amplification potentially introduces a bias of average magnitude $1.5^{1/15} = 1.027$. Thus, the 25 cycles of PCR introduces a total bias of average magnitude $1.027^{25} = 1.95$ in the inferred relative abundance of distinct CDR3 region sequences.

**Example 8:** J$\beta$ gene segment usage

The CDR3 region in each TCR $\beta$ chain includes sequence derived from one of the thirteen $J_\beta$ gene segments. Analysis of the CDR3 sequences in the four different T cell populations from the two donors demonstrated that the fraction of total sequences which incorporated sequences derived from the thirteen different $J_\beta$ gene segments varied more than 20-fold. J$\beta$ utilization among four different T flow cytometrically-defined T cells from a single donor is was relatively constant within a given donor. Moreover, the $J_\beta$ usage patterns observed in two donors, which were inferred from analysis of genomic DNA from T cells sequenced using the GA, are qualitatively similar to those observed in T cells from umbilical cord blood and from healthy adult donors, both of which were inferred from analysis of cDNA from T cells sequenced using exhaustive capillary-based techniques.

**Example 9:** Nucleotide insertion bias

Much of the diversity at the CDR3 junctions in TCR $\alpha$ and $\beta$ chains is created by non-templated nucleotide insertions by the enzyme Terminal Deoxynucleotidyl Transferase (TdT). However, in vivo, selection plays a significant role in shaping the TCR repertoire giving rise to unpredictability. The TdT nucleotide insertion frequencies, independent of selection, were calculated using out of frame TCR sequences. These sequences are non-functional rearrangements that are carried on one allele in T cells where the second allele has a functional rearrangement. The mono-nucleotide insertion bias of TdT favors C and G (Table 11).

**Table 11:** Mono-nucleotide bias in out of frame data

| | A | C | G | T |
|---|---|---|---|---|
| Lane 1 | 0.24 | 0.294 | 0.247 | 0.216 |
| Lane 2 | 0.247 | 0.284 | 0.256 | 0.211 |
| Lane 3 | 0.25 | 0.27 | 0.268 | 0.209 |
| Lane 4 | 0.255 | 0.293 | 0.24 | 0.21 |

Similar nucleotide frequencies are observed in the in frame sequences (Table 12).

**Table 12:** Mono-nucleotide bias in in-frame data

| | A | C | G | T |
|---|---|---|---|---|
| Lane 1 | 0.21 | 0.285 | 0.275 | 0.228 |
| Lane 2 | 0.216 | 0.281 | 0.266 | 0.235 |
| Lane 3 | 0.222 | 0.266 | 0.288 | 0.221 |
| Lane 4 | 0.206 | 0.294 | 0.228 | 0.27 |

The N regions from the out of frame TCR sequences were used to measure the di-nucleotide bias. To isolate the marginal contribution of a di-nucleotide bias, the di-nucleotide frequencies was divided by the mononucleotide frequencies of each of the two bases. The measure is

$$m = \frac{f(n_1 n_2)}{f(n_1)f(n_2)}.$$

The matrix for m is found in Table 13.

**Table 13:** Di-nucleotide odd ratios for out of frame data

| | A | C | G | T |
|---|---|---|---|---|
| A | 1.198 | 0.938 | 0.945 | 0.919 |
| C | 0.988 | 1.172 | 0.88 | 0.931 |
| G | 0.993 | 0.701 | 1.352 | 0.964 |
| T | 0.784 | 1.232 | 0.767 | 1.23 |

Many of the dinucleotides are under or over represented. As an example, the odds of finding a GG pair are very high. Since the codons GGN translate to glycine, many glycines are expected in the CDR3 regions.

**Example 10:** Amino Acid distributions in the CDR3 regions

The distribution of amino acids in the CDR3 regions of TCR$\beta$ chains are shaped by the germline sequences for V, D, and J regions, the insertion bias of TdT, and selection. The distribution of amino acids in this region for the four different T cell sub-compartments is very similar between different cell subtypes. Separating the sequences into $\beta$ chains of fixed length, a position dependent distribution among amino acids, which are grouped by the six chemical properties: small, special, and large hydrophobic, neutral polar, acidic and basic. The distributions are virtually identical except for the CD8+ antigen experienced T cells, which have a higher proportion of acidic bases, particularly at position 5.

Of particular interest is the comparison between $CD8^+$ and $CD4^+$ TCR sequences as they bind to peptides presented by class I and class II HLA molecules, respectively. The $CD8^+$ antigen experienced T cells have a few positions with a higher proportion of acidic amino acids. This could be do binding with a basic residue found on HLA Class I molecules, but not on Class II.

**Example 11:** TCR $\beta$ chains with identical amino acid sequences found in different people

The TCR $\beta$ chain sequences were translated to amino acids and then compared pairwise between the two donors. Many thousands of exact sequence matches were observed. For example, comparing the $CD4^+$ $CD45RO^-$ sub-compartments, approximately 8,000 of the 250,000 unique amino acid sequences from donor 1 were exact matches to donor 2. Many of these matching sequences at the amino acid level have multiple nucleotide differences at third codon positions. Following the example mentioned above, 1,500/8,000 identical amino acid matches had >5 nucleotide mismatches. Between any two T cell sub-types, 4-5% of the unique TCR$\beta$ sequences were found to have identical amino acid matches.

Two possibilities were examined: that 1) selection during TCR development is producing these common sequences and 2) the large bias in nucleotide insertion frequency by TdT creates similar nucleotide sequences. The in-frame pairwise matches were compared to the out-of-frame pairwise matches (see Examples 1-4, above). Changing frames preserved all of the features of the genetic code and so the same number of matches should be found if the sequence bias was responsible for the entire observation. However, almost twice as many in-frame matches as out-of-frame matches were found, suggesting that selection at the protein level is playing a significant role.

To confirm this finding of thousands of identical TCR $\beta$ chain amino acid sequences, two donors were compared with respect to the $CD8^+$ $CD62L^+$ $CD45RA^+$ (naive-like) TCRs from a third donor, a 44 year old $CMV^+$ Caucasian female. Identical pairwise matches of many thousands of sequences at the amino acid level between the third donor and each of the original two donors were found. In contrast, 460 sequences were shared between all three donors. The large variation in total number of unique sequences between the donors is a product of the starting material and variations in loading onto the sequencer, and is not representative of a variation in true diversity in the blood of the donors.

**Example 12:** Higher frequency clonotypes are closer to germline

The variation in copy number between different sequences within every T cell sub-compartment ranged by a factor of over 10,000-fold. The only property that correlated with copy number was (the number of insertions plus the number of deletions), which inversely correlated. Results of the analysis showed that deletions play a smaller role than insertions in the inverse correlation with copy number.

Sequences with less insertions and deletions have receptor sequences closer to germ line. One possibility for the increased number of sequences closer to germ line is that they are the created multiple times during T cell development. Since germ line sequences are shared between people, shared TCR$\beta$ chains are likely created by TCRs with a small number of insertions and deletions.

**Example 13:** "Spectratype" analysis of TCR$\beta$ CDR3 sequences by V gene segment utilization and CDR3 length

TCR diversity has commonly been assessed using the technique of TCR spectratyping, an RT-PCR-based technique that does not assess TCR CDR3 diversity at the sequence level, but rather evaluates the diversity of TCR$\alpha$ or TCR$\beta$ CDR3 lengths expressed as mRNA in subsets of $\alpha\beta$ T cells that use the same $V_\alpha$ or $V_\beta$ gene segment. The spectratypes of polyclonal T cell populations with diverse repertoires of TCR CDR3 sequences, such as are seen in umbilical cord blood or in peripheral blood of healthy young adults typically contain CDR3 sequences of 8-10 different lengths that are multiples of three nucleotides, reflecting the selection for in-frame transcripts. Spectratyping also provides roughly quantitative information about the relative frequency of CDR3 sequences with each specific length. To assess whether direct sequencing of TCR$\beta$ CDR3 regions from T cell genomic DNA using the sequencer could faithfully capture all of the CDR3 length diversity that is identified by spectratyping, "virtual" TCR$\beta$ spectratypes (see Examples above) were generated from the sequence data and compared with TCR$\beta$ spectratypes generated using conventional PCR techniques. The virtual spectratypes contained all of the CDR3 length and relative frequency information present in the conventional spectratypes. Direct TCRβ CDR3 sequencing captures all of the TCR diversity information present in a conventional spectratype. A comparison of standard TCRβ spectratype data and calculated TCRβ CDR3 length distributions for sequences utilizing representative TCR Vβ gene segments and present in $CD4^+CD45RO^+$ cells from donor 1. Reducing the information contained in the sequence data to a frequency histogram of the unique CDR3 sequences with different lengths within each Vβ family readily reproduces all of the information contained in the spectratype data. In addition, the virtual spectratypes revealed the presence within each $V_\beta$ family of rare CDR3 sequences with both very short and very long CDR3 lengths that were not detected by conventional PCR-based spectratyping.

**Example 14:** Estimation of total CDR3 sequence diversity

After error correction, the number of unique CDR3 sequences observed in each lane of the sequencer flow cell routinely exceeded 1 x $10^5$. Given that the PCR products sequenced in each lane were necessarily derived from a small fraction of the T cell genomes present in each of the two donors, the total number of unique TCR$\beta$ CDR3 sequences in the entire T cell repertoire of each individual is likely to be far higher. Estimating the number of unique sequences in the entire repertoire, therefore, requires an estimate of the number of additional unique CDR3 sequences that exist in the blood but were not observed in the sample. The estimation of total species diversity in a large, complex population using measurements of the species diversity present in a finite sample has historically been called the "unseen species problem" (see Examples above). The solution starts with determining the number of new species, or TCR$\beta$ CDR3 sequences, that are observed if the experiment is repeated, i.e., if the sequencing is repeated on an identical sample of peripheral blood T cells, e.g., an identically prepared library of TCR$\beta$ CDR3 PCR products in a different lane of the sequencer flow cell and counting the number of new CDR3 sequences. For $CD8^+CD45RO^-$ cells from donor 2, the predicted and observed number of new CDR3 sequences in a second lane are within 5% (see Examples above), suggesting that this analytic solution can, in fact, be used to estimate the total number of unique TCR$\beta$ CDR3 sequences in the entire repertoire.

The resulting estimates of the total number of unique TCR$\beta$ CDR3 sequences in the four flow cytometrically-defined T cell compartments are shown in Table 14.

**Table 14:** TCR repertoire diversity

| Donor | CD8 | CD4 | CD45RO | Diversity |
|---|---|---|---|---|
| 1 | + | - | + | $6.3*10^5$ |
| | + | - | - | $1.24*10^6$ |
| | - | + | + | $8.2*10^5$ |
| | - | + | - | $1.28*10^6$ |
| | Total T cell diversity | | | $3.97*10^6$ |
| 2 | + | - | + | $4.4*10^5$ |
| | + | - | - | $9.7*10^5$ |
| | - | + | + | $8.7*10^5$ |
| | - | + | - | $1.03*10^6$ |
| | Total T cell diversity | | | $3.31*10^6$ |

Of note, the total TCR$\beta$ diversity in these populations is between 3-4 million unique sequences in the peripheral blood. Surprisingly, the CD45RO$^+$, or antigen-experienced, compartment constitutes approximately 1.5 million of these sequences. This is at least an order of magnitude larger than expected. This discrepancy is likely attributable to the large number of these sequences observed at low relative frequency, which could only be detected through deep sequencing. The estimated TCR$\beta$ CDR3 repertoire sizes of each compartment in the two donors are within 20% of each other.

The results herein demonstrate that the realized TCR$\beta$ receptor diversity is at least fivefold higher than previous estimates (~$4*10^6$ distinct CDR3 sequences), and, in particular, suggest far greater TCR$\beta$ diversity among CD45RO$^+$ antigen-experienced $\alpha\beta$ T cells than has previously been reported (~$1.5*10^6$ distinct CDR3 sequences). However, bioinformatic analysis of the TCR sequence data shows strong biases in the mono- and di- nucleotide content, implying that the utilized TCR sequences are sampled from a distribution much smaller than the theoretical size. With the large diversity of TCR$\beta$ chains in each person sampled from a severely constrict space of sequences, overlap of the TCR sequence pools can be expected between each person. In fact, the results showed about 5% of CD8$^+$ naive TCR$\beta$ chains with exact amino acid matches are shared between each pair of three different individuals. As the TCR$\alpha$ pool has been previously measured to be substantially smaller than the theoretical TCR$\beta$ diversity, these results show that hundreds to thousands of truly public $\alpha\beta$ TCRs can be found.

## Claims

**1.** A composition comprising:

(a) V-segment primers comprising at least 45 forward primers consisting of the nucleotide sequences of SEQ ID NOs: 1-45, wherein each of the 45 forward primers is modified at the 5' end with a universal forward primer sequence compatible with a DNA sequencer; and
(b) J-segment primers comprising at least 13 reverse primers consisting of the nucleotide sequences of SEQ ID NOs: 46-57 and 483, wherein each of the 13 reverse primers is modified at the 5' end with a universal reverse primer sequence compatible with a DNA sequencer.

**2.** The composition of claim 1, wherein

(a) the 45 forward primers consist of the nucleotide sequences of SEQ ID NOs: 58-102; and
(b) the 13 reverse primers consist of the nucleotide sequences of SEQ ID NOs: 103-113, 468 and 484.

**3.** A method for quantifying TCRβ gene diversity in a sample, comprising:

(a) combining the composition of claim 1 or claim 2 with a sample of genomic DNA to permit amplification of rearranged TCRβ CDR3 regions from the sample, in a multiplex polymerase chain reaction (PCR), to produce a multiplicity of amplified DNA molecules that are sufficient to quantify TCRβ gene diversity in the sample by single molecule DNA sequencing, wherein each amplified DNA molecule includes adequate sequence to identify each V segment and each J segment by single molecule DNA sequencing; and optionally
(b) sequencing the amplified DNA molecules.

**4.** The method of claim 3, wherein the step of sequencing comprises hybridizing a set of sequencing oligonucleotides to one or more defined regions within the amplified DNA molecules.

**5.** The method of claim 4, further comprising a step of calculating the total diversity of TCRβ CDR3 sequences among the amplified DNA molecules, optionally wherein the method shows that the total diversity of a normal human subject is greater than $1*10^6$ sequences, greater than $2*10^6$ sequences, or greater than $3*10^6$ sequences.

**6.** A method of diagnosing immunodeficiency in a human patient comprising measuring the diversity of TCRβ CDR3 sequences in a sample from the patient according to the method of claim 3, and comparing the TCRβ CDR3 sequence diversity of the sample from the patient to the TCRβ CDR3 sequence diversity of a sample from a normal subject, wherein a difference in the TCRβ CDR3 sequence diversity of the sample from the patient compared to the TCRβ CDR3 sequence diversity of the sample from the normal subject is an indication of immunodeficiency

**7.** The method of claim 6, wherein the diversity of TCRβ CDR3 sequences of the sample from the normal subject is measured according to the method of claim 3.

**8.** The method of claim 6 or claim 7, wherein comparing the TCRβ CDR3 sequence diversity comprises calculating the total diversity of TCRβ CDR3 sequences among the amplified DNA molecules from the sample from the normal subject and from the patient.

**9.** The method of claim 8, wherein diversity is determined using the following equation:

$$\Delta(t) = \sum_x E(n_x)_{measurement 1+2} - \sum_x E(n_x)_{measurement 2} = S\int_0^\infty e^{-\lambda}\left(1-e^{-\lambda t}\right)dG(\lambda)$$

wherein $G(\lambda)$ is the empirical distribution function of the parameters $\lambda_I, \ldots, \lambda_S$, $n_x$ is the number of clonotypes sequenced exactly x times, and

$$E(n_x) = S\int_0^\infty \left( \frac{e^{-\lambda} \lambda^x}{x!} \right) dG(\lambda)$$

**10.** The method of claim 6 in which at least two samples of genomic DNA from the patient are compared, optionally wherein:

(i) one sample of genomic DNA is from the patient before the patient has received a therapeutic treatment and the other sample is from the patient after treatment; or
(ii) the two samples of genomic DNA are from the same patient at different times during treatment.

**11.** The method of claim 6 or claim 10 in which:

(i) a disease is diagnosed based on the comparison of TCRβ CDR3 sequence diversity among the at least two samples of genomic DNA; and/or
(ii) immunocompetence of the patient is assessed by comparing said at least two samples.

**Patentansprüche**

**1.** Zusammensetzung, umfassend

(a) V-Segment-Primer, umfassend wenigstens 45 Forward-Primer, bestehend aus den Nukleotid-Sequenzen der SEQ-ID-Nrn. 1 bis 45, wobei jeder der 45 Forward-Primer an dem 5'-Ende mit einer universellen Forward-Primer-Sequenz modifiziert ist, die mit einem DNS-Sequenzer kompatibel ist; und
(b) J-Segment-Primer, umfassend wenigstens 13 Reverse-Primer, die aus den Nukleotid-Sequenzen der SEQ-ID-Nrn. 46 bis 57 und 483 bestehen, wobei jeder der 13 Reverse-Primer an dem 5'-Ende mit einer universellen Reverse-Primer-Sequenz modifiziert ist, die mit einem DNS-Sequenzer kompatibel ist.

**2.** Zusammensetzung nach Anspruch 1, wobei

(a) die 45 Forward-Primer aus den Nukleotid-Sequenzen der SEQ-ID-Nrn. 58 bis 102 bestehen; und
(b) die 13 Reverse-Primer aus den Nukleotid-Sequenzen SEQ-ID-Nrn. 103 bis 113, 468 und 484 bestehen.

**3.** Verfahren zum Quantifizieren der TCBβ-Gen-Diversität einer Probe, umfassend

(a) ein Zusammengeben der Zusammensetzung nach Anspruch 1 oder Anspruch 2 mit einer Probe von Genom-DNS unter Erlauben einer Amplifikation umgestalteter TCRβ-CDR3-Bereiche von der Probe in einer Multiplex-Polymerase-Kettenreaktion (PCR) unter Herstellen einer Vielzahl amplifizierter DNS-Moleküle, die ausreichend sind zum Quantifizieren der TCRβ-Gen-Diversität in der Probe durch Einzelmolekül-DNS-Sequenzieren, wobei jedes amplifizierte DNS-Molekül eine adäquate Sequenz zum Identifizieren jedes V-Segments und jedes J-Segments durch Einzelmolekül-DNS-Sequenzierung einschließt; und gegebenenfalls
(b) ein Sequenzieren der amplifizierten DNS-Moleküle.

**4.** Verfahren nach Anspruch 3, wobei der Schritt des Sequenzierens umfasst ein Hybridisieren eines Satzes sequenzierender Oligonukleotide zu einem oder mehreren definierten Bereichen innerhalb der amplifizierenden DNS-Moleküle.

**5.** Verfahren nach Anspruch 4, weiter umfassend einen Schritt eines Berechnens der Gesamt-Diversität von TCRβ-CDR3-Sequenzen unter den amplifizierten DNS-Molekülen, gegebenenfalls wobei das Verfahren zeigt, dass die Gesamt-Diversität einer normalen menschlichen Person größer ist als $1 \cdot 10^6$ Sequenzen, größer als $2 \cdot 10^6$ Sequenzen oder größer als $3 \cdot 10^6$ Sequenzen.

**6.** Verfahren zum diagnostischen Ermitteln von Immunschwäche in einem menschlichen Patienten, umfassend ein Messen der Diversität von TCRβ-CDR3-Sequenzen in einer Probe von dem Patienten nach dem Verfahren von Anspruch 3 und ein Vergleichen der TCRβ-CDR3-Sequenzdiversität der Probe von dem Patienten mit der TCRβ-CDR3-Sequenzdiversität einer Probe von einer normalen Person, wobei eine Differenz der TCRβ-CDR3-Sequenzdiversität der Probe von dem Patienten im Vergleich zu der TCRβ-CDR3-Sequenzdiversität der Probe von der normalen Person ein Hinweis auf Immunschwäche ist.

**7.** Verfahren nach Anspruch 6, wobei die Diversität von TCRβ-CDR3-Sequenzen der Probe von dem normalen Menschen gemäß dem Verfahren nach Anspruch 3 gemessen wird.

**8.** Verfahren nach Anspruch 6 oder Anspruch 7, wobei ein Vergleichen der TCRβ-CDR3-Sequenzdiversität ein Berechnen der gesamten Diversität von TCRβ-CDR3-Sequenzen unter den amplifizierten DNS-Molekülen von der Probe von dem normalen Menschen und von dem Patienten umfasst.

**9.** Verfahren nach Anspruch 8, wobei die Diversität unter Verwendung der folgenden Gleichung bestimmt wird:

$$\Delta(t) = \sum_x E(n_x)_{\text{Messung } 1+2} - \sum_x E(n_x)_{\text{Messung } 2} = S\int_0^\infty e^{-\lambda}\left(1-e^{-\lambda t}\right)dG(\lambda)$$

wobei G(λ) die empirische Verteilungsfunktion der Parameter $\lambda_1, ...., \lambda_S$ ist, $n_x$ die Zahl von exakt x Mal sequenzierten Clonotypen ist, und gilt:

$$E(n_x) = S\int_0^\infty \left(\frac{e^{-\lambda}\lambda^x}{x!}\right)dG(\lambda)$$

**10.** Verfahren nach Anspruch 6, in dem wenigstens zwei Proben von Genom-DNS von dem Patienten verglichen werden; gegebenenfalls wobei

(i) eine Probe von Genom-DNS von dem Patienten ist, bevor der Patient eine therapeutische Behandlung erfahren hat, und die andere Probe von dem Patienten nach der Behandlung ist; oder
(ii) die beiden Proben von Genom-DNS von demselben Patienten zu verschiedenen Zeiten während der Behandlung sind.

**11.** Verfahren nach Anspruch 6 oder Anspruch 10, in dem

(i) die Diagnose einer Krankheit gestellt wird auf der Basis des Vergleichs von TCRβ-CDR3-Sequenzdiversität unter den wenigstens zwei Proben von Genom-DNS; und/oder
(ii) eine Immunkompetenz des Patienten bewertet wird durch Vergleichen der wenigstens zwei Proben.

## Revendications

**1.** Composition comprenant :

(a) des amorces de segment V comprenant au moins 45 amorces sens consistant en les séquences de nucléotides de SEQ ID NO: 1-45, où chacune des 45 amorces sens est modifiée à l'extrémité 5' avec une séquence d'amorce sens universelle compatible avec un séquenceur d'ADN ; et
(b) des amorces de segment J comprenant au moins 13 amorces antisens consistant en les séquences de nucléotides de SEQ ID NO: 46-57 et 483, où chacune des 13 amorces antisens est modifiée à l'extrémité 5' avec une séquence d'amorce antisens universelle compatible avec un séquenceur d'ADN.

**2.** Composition selon la revendication 1, dans laquelle

(a) les 45 amorces sens consistent en les séquences de nucléotides de SEQ ID NO: 58-102 ; et
(b) les 13 amorces antisens consistent en les séquences de nucléotides de SEQ ID NO: 103-113, 468 et 484.

**3.** Procédé pour quantifier la diversité du gène TCRβ dans un échantillon, comprenant :

(a) la combinaison de la composition selon la revendication 1 ou la revendication 2 avec un échantillon d'ADN génomique pour permettre l'amplification de régions CDR3 de TCRβ réarrangées à partir de l'échantillon, dans une réaction en chaîne par polymérase (PCR) multiplexe, afin de produire une multiplicité de molécules d'ADN amplifiées qui sont suffisantes pour quantifier la diversité du gène TCRβ dans l'échantillon par le séquençage de l'ADN d'une seule molécule, où chaque molécule d'ADN amplifiée comprend une séquence adéquate pour identifier chaque segment V et chaque segment J par le séquençage de l'ADN d'une seule molécule ; et facultativement

(b) le séquençage des molécules d'ADN amplifiées.

**4.** Procédé selon la revendication 3, dans lequel l'étape de séquençage comprend l'hybridation d'un ensemble d'oligonucléotides de séquençage à une ou plusieurs régions définies au sein des molécules d'ADN amplifiées.

**5.** Procédé selon la revendication 4, comprenant en outre une étape consistant à calculer la diversité totale des séquences CDR3 de TCRβ parmi les molécules d'ADN amplifiées, facultativement où le procédé montre que la diversité totale d'un sujet humain normal est supérieure à $1*10^6$ séquences, supérieure à $2*10^6$ séquences, ou supérieure à $3*10^6$ séquences.

**6.** Procédé de diagnostic d'une immunodéficience chez un patient humain comprenant la mesure de la diversité des séquences CDR3 de TCRβ dans un échantillon du patient selon le procédé de la revendication 3, et la comparaison de la diversité de séquences CDR3 de TCRβ de l'échantillon du patient avec la diversité de séquences CDR3 de TCRβ d'un échantillon d'un sujet normal, dans lequel une différence de diversité de séquences CDR3 de TCRβ de l'échantillon du patient par comparaison à la diversité de séquences CDR3 de TCRβ de l'échantillon du sujet normal est une indication d'une immunodéficience.

**7.** Procédé selon la revendication 6, dans lequel la diversité de séquences CDR3 de TCRβ de l'échantillon du sujet normal est mesurée selon le procédé de la revendication 3.

**8.** Procédé selon la revendication 6 ou la revendication 7, dans lequel la comparaison de la diversité de séquences CDR3 de TCRβ comprend le calcul de la diversité totale des séquences CDR3 de TCRβ parmi les molécules d'ADN amplifiées de l'échantillon du sujet normal et du patient.

**9.** Procédé selon la revendication 8, dans lequel la diversité est déterminée en utilisant l'équation suivante :

$$\Delta(t) = \sum_x E(n_x)_{mesure1+2} - \sum_x E(n_x)_{mesure2} = S \int_0^\infty e^{-\lambda}\left(1 - e^{-\lambda t}\right) dG(\lambda)$$

dans laquelle $G(\lambda)$ est la fonction de distribution empirique des paramètres $\lambda_1, ..., \lambda_S$, $n_x$ est le nombre de clonotypes séquencés exactement x fois, et

$$E(n_x) = S \int_0^\infty \left(\frac{e^{-\lambda}\lambda^x}{x!}\right) dG(\lambda)$$

**10.** Procédé selon la revendication 6 dans lequel au moins deux échantillons d'ADN génomique du patient sont comparés, facultativement dans lequel :

(i) un échantillon d'ADN génomique est prélevé chez le patient avant que le patient ne reçoive un traitement thérapeutique et l'autre échantillon est prélevé chez le patient après le traitement ; ou

(ii) les deux échantillons d'ADN génomique sont prélevés chez le même patient à différents moments pendant le traitement.

**11.** Procédé selon la revendication 6 ou la revendication 10 dans lequel :

(i) une maladie est diagnostiquée en se basant sur la comparaison de la diversité de séquences CDR3 de TCRβ parmi les au moins deux échantillons d'ADN génomique ; et/ou

(ii) l'immunocompétence du patient est évaluée en comparant lesdits au moins deux échantillons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2062982 A
- US 61220344 B


### Non-patent literature cited in the description


- **MARIANI et al.** *Exp Hematol.,* 2009, vol. 37 (6), 728-738
- **VAN DONGEN et al.** *Leukemia,* 2003, vol. 17 (12), 2257-2317
- **MAŚLANKA et al.** *Hum Immunol.,* 1995, vol. 44 (1), 28-34
- **KIIANITSA et al.** *Blood,* 2007, vol. 110 (11), 293B